# EUROPEAN PATENT SPECIFICATION

(11) **EP 2 645 987 B1**
(45) Date of publication and mention of the grant of the patent: **04.11.2015**
(21) Application number: 11791279.0
(22) Date of filing: 01.12.2011
(51) Int. Cl.: A61K 8/66, A61K 38/44, A61L 12/08, A61Q 19/08, C11D 3/386, C12N 9/08

(54) **NOVEL CATALASES**
NEUE KATALASEN
CATALASES NOUVELLES

(30) Priority: 01.12.2010 EP 10015193
(43) Date of publication of application: 09.10.2013
(73) Proprietor: B.R.A.I.N. Biotechnology Research And Information Network AG, 64673 Zwingenberg (DE)
(72) Inventor: SCHULZE, Renate, 64625 Bensheim (DE); ECK, Jürgen, 64625 Bensheim (DE)
(74) Representative: Vossius & Partner Patentanwälte Rechtsanwälte mbB
(86) International application number: PCT/EP2011/071576
(87) International publication number: WO 2012/072777

(56) References cited:
- EP-A2- 0 082 798
- WO-A1-2009/104622
- GB-A- 2 307 175
- JP-A- 2006 008 571
- US-A1- 2007 218 045

## Description

The invention relates to a nucleic acid molecule encoding a polypeptide having the activity of a catalase (EC 1.11.1.6), which nucleic acid molecule is (a) a nucleic acid molecule encoding a polypeptide comprising or consisting of the amino acid sequence of SEQ ID NO: 1; (b) a nucleic acid molecule comprising or consisting of the nucleotide sequence of SEQ ID NO: 2; (c) a nucleic acid molecule comprising or consisting of a nucleic acid molecule encoding a polypeptide having the activity of a catalase the amino acid sequence of which is at least 99% identical to the amino acid sequence of SEQ ID NO: 1, (d) a nucleic acid molecule encoding a polypeptide having the activity of a catalase and comprising or consisting of a nucleotide sequence which is at least 98% identical to the nucleotide sequence of SEQ ID NO: 2, (e) a fragment of the polypeptide of any of (a) to (d) having the activity of a catalase and comprising at least 675 amino acids; or (f) the nucleic acid sequence of any of (a) to (d) wherein T is U.

Catalases can be found in a wide variety of eukaryotic and prokaryotic organisms including, but not limited to *Agrobacterium tumefaciens, Aliivibrio salmonicida, Anopheles gambiae, Aspergillus nidulans, Aspergillus niger, Bacillus firmus, Bacillus megaterium, Bacillus subtilis, Bacteroides fragilis, Bordetella perfussis, Bos taurus, Brassica oleracea var. gongylodes, Burkholderia cenocepacia, Burkholderia pseudomallei, Candida tropicalis, Canis familiaris, Capra capra, Chrysosporium pannorum, Cladosporium herbarum, Comamonas compransoris, Equus caballus, Escherichia coli, Gallus gallus, Ginkgo biloba, Haloarcula marismortui, Halobacterium halobium, Helianthus annuus, Helicobacter pylori, Homo sapiens, Klebsiella pneumoniae, Leuciscus cephalus, Listeria seeligeri, Micrococcus luteus, Micrococcus lysodeikticus, Mus musculus, Mycobacterium tuberculosis, Neurospora crassa, Oryza sativa, Ovis aries, Paracoccidioides brasiliensis, Penicillium chrysogenum, Penicillium cyclopium, Penicillium islandicum, Penicillium piceum, Penicillium implicissimum, Petroselinum hortense, Phaseolus vulgaris, Phytophthora nicotianae, Pichia pastoris, Pinus strobus, Pisum sativum, Protaetia brevitarsis, Proteus mirabilis, Pseudomonas aeruginosa, Pseudomonas putida, Pseudomonas syringae, Pyrobaculum calidifontis, Rattus norvegicus, Rhodobacter sphaeroides, Rhodopseudomonas capsulata, Rhodospirillum rubrum, Saccharomyces cerevisiae, Scytalidium thermophilum, Serratia marcescens, Sinorhizobium meliloti, Streptomyces coelicolor, Thermus brockianus, Trigonopsis variabilis, Vibrio rumoiensis, Xanthomonas campestris, Zea mays.*

In filamentous fungi, such as *Penicillium,* catalases and/or catalase-peroxidases are thought to play a role in host-fungus interactions and pathogenicity. The molecular mechanism of catalases, amongst them a catalase from *Penicillium vitale* was investigated in detail (Alfonso-Prieto M, Biarnés X, Vidossich P, Rovira C., (2009), The molecular mechanism of the catalase reaction, J Am Chem Soc. 26;131(33):11751-61).

In general, catalases (EC 1.11.1.6, CAS 9001-05-02) catalyse the disproportionation of two molecules of hydrogen peroxide (H₂O₂) into two molecules water (H₂O) and one molecule oxygen (O₂). The operating temperature of catalase activity comprises the whole range from 0°C to 100 °C, depending on the exact nature of the enzyme. The pH of catalase activity can range between 3 and 13. Cells utilize catalases, together with other cellular enzyme systems, to protect themselves against the harmful effects of reactive oxygen species such as super-oxide anions, hydrogen peroxide, and hydroxyl radicals.

Three general classes of catalases have been described in the literature: the typical or monofunctional catalases; the catalase-peroxidases that have a peroxidative activity as well as catalase activity; and the Mn-catalases or pseudocatalases. Typical catalases, which have similar properties, have been isolated from numerous animals, plants, and microorganisms. These enzymes typically have four subunits of equal size with a combined molecular mass of 225,000-270,000 kDa and characteristically have four protoheme IX prosthetic groups per tetrameric molecule. These enzymes also typically display a broad pH activity range from 4 to 10, are specifically inhibited by 3-amino-1,2,4-triazole, and are resistant to reduction by dithionite.

Generally, in processes where hydrogen peroxide is a by-product, catalases can be used to destroy or detect hydrogen peroxide, e.g., in production of glyoxylic acid and in glucose sensors. Also, in processes where hydrogen peroxide is used as a bleaching or antibacterial agent, catalases can be used to destroy residual hydrogen peroxide, e.g. in contact lens cleaning, in bleaching steps in pulp and paper production, and in the pasteurization of dairy products.

There is growing interest in utilizing hydrogen peroxide in industrial sectors as a more environmentally friendly alternative to existing chemical treatments for bleaching and sterilization. Consequently, catalases find many applications in industry. Due to their efficiency in the degradation of hydrogen peroxide, catalases are often applied in processes that require the destruction of excess hydrogen peroxide from a previous process step, because hydrogen peroxide might disturb the subsequent step of that particular process. Thus, there is a need to remove hydrogen peroxide from the process stream so that the process water may be reused in subsequent steps. E.g. in the textile industry, catalase is applied to remove hydrogen peroxide from the bleach liquor and bleached fabric after the bleaching step. Hydrogen peroxide would have a detrimental effect during the subsequent dying step, because it could bleach the dyes.

Catalase can be utilized as oxygen producer e.g. for reducing pollution in an aqueous environment. EP 0946427 B1 describes the benefits of catalase as oxygen supplier for promoting growth of aerobic species and reducing biological pollution e.g. in silage, animal feed, plumbing systems and the like.

A number of publications describe the effects of catalases on the propagation of microorganisms, especially those of clinical or hygienic relevance. E.g. *Staphylococcus aureus* cells which are thermally stressed are much more sensitive to H₂O₂ and therefore addition of catalase to the culture medium lead to an increase of enumeration of S. *aureus* cells by a factor of 1.000 (Flowers, R.S., Martin, S.E., Brewer, D.G. and Ordal, Z.J., Catalase and enumeration of stressed Staphylococcus aureus cells, Appl. Env. Microbiol. May 1977, 1112-1117). A similar effect was described for *Clostridium perfringens* (Harmon, S.M. and Kautter, D.A., Beneficial effect of catalase treatment on growth of Clostridium perfringens, Appl. Env. Microbiol., Sept. 1976, 409-416) or a certain strain of *E.coli* responsible for EHEC, ah enterohemorrhagic disease (Mizunoe, Y, Wai, S.N., Takade, A, Yoshida, S-I, Restoration of culturability of starvation-stressed and low-temperature stressed Escherichia coli 0157 cells by using H2O2 degrading compounds, Arch. Microbiol. 1999, 172, 63-67). Furthermore, supplementation of media with catalase was suitable to elicit a greater occurrence of members of previously uncultured microbes in cultivation experiments with samples from natural habitats (Stevenson, B.S., Eichorst, S.A., Wertz, J.T., Schmidt, T.M. and Breznak, J.A. New strategies for cultivation and detection of previously uncultured microbes. Appl. Env. Microbiol., Aug. 2004, 4748-4755).

In the beverage industry catalases can be used, amongst other applications, to increase the sulfite content during fermentation of alcoholic products. WO 2007/105350 A2 discloses a gene encoding a catalase in brewery yeast which can be utilized in the production of beer. Furthermore, catalases are used in textile and pupl and paper processing. In these processes in general high temperatures occur. EP 2256192 A1 describes a thermostable catalase which is, for example, intended to be used to remove hydrogen peroxide remaining after bleaching of cotton or disinfection of food.

During processing of eggs to egg powder, a desugaring is performed with a combination of glucose oxidase and catalase. Glucose oxidase oxidises ß-D-glucose to δ-D-gluconolactone while reducing molecular oxygen to hydrogen peroxide. The disproportionation reaction of the catalase in the enzyme cocktail can be used to regenerate the consumed oxygen in order to let the reaction proceed and to consume excess hydrogen peroxide that might compromise the taste of the egg powder.

Furthermore, a potential role of catalases as therapeutic agent against oxidative stress in diseases like vitiligo as well as in a cosmetic application during senile hair greying is postulated (Schallreuter, K.U., Rübsam, K., Gibbons, N.CJ., Maitland, D.J., Chavan, B., Zothner, C., Rokos, H. and Wood, J.M., Methionine Sulfoxide Reductases A and B are Deactivated by Hydrogen Peroxide (H2O2) in the Epidermis of Patients with Vitiligo, J. Invest. Dermatol. (2008) 128, 808-815; Wood, J.M., Decker, H., Hartmann, H., Chavan, B., Rokos, H., Spencer, J.D., Hasse, S., Thornton, M.J., Shalbaf, M., Paus, R., and Schallreuter, K.U., Senile hair greying: H2O2-mediated oxidative stress affects human hair color by blunting methionine sulfoxide repair, FASEB J. (2009), 23, 2065-75).

The industrial use of hydrogen peroxide constantly increases, because it reduces environmental impact and hazard relative to an equivalent amount of chlorine. Nevertheless, the environmental exposure to hydrogen peroxide may occur through emissions in all major environmental compartments, air, surface water, and soil. Thus, there is a need to treat hydrogen peroxide to reduce environmental exposure. The catalase enzyme has been used in the textile industry as a environmentally friendly agent of removing or decreasing residual hydrogen peroxide in exhausted bleach baths. However, bleaching of textiles, pulp and paper typically occurs at high temperatures and pH. At these elevated temperatures and pH, commercially available catalases do not retain sufficient activity to provide an economically practical method of removing the hydrogen peroxide. Thus, the temperature and pH of the process water must be reduced prior to treatment with traditional catalases.

Many industrial processes utilizing hydrogen peroxide occur at elevated temperatures and pH (> 60 °C and pH 9). The currently available commercial enzymes, which rapidly lose their activity under these conditions, are unsuitable for use under such conditions. Thus, temperature plays a significant role in enzyme performance. In addition to temperature, pH also affects enzyme kinetics and stability of the enzyme. The pH may affect deactivation of the enzyme due to covalent changes, such as the deamination of asparagine residues and non-covalent changes such as the rearrangement of the protein chain. High pH, indicative of a basic or alkaline environment, may also result in random cleavage of the peptide. Beyond deamination and cleavage, pH has a substantial effect on the protonation state of the amino acid side chains and the function of the enzyme. Thus, enzymes display a range of pH within which they will function adequately. In particular, commercially available catalases are optimally active at a temperature range between 20-50 °C and at neutral pH.

WO 2002/103032 A2 describes methods for neutralizing hydrogen peroxide using catalase enzymes as well as methods of designing new catalases with increased activity and stability at increased pH and temperatures.

In view of the various industrial applications of catalases referred to herein above, there is an ongoing need of further catalases, ideally being improved as compared to the catalases known from the prior art.

This need is addressed by the provision of the embodiments characterized in the claims. Accordingly, the invention relates in first embodiment to a nucleic acid molecule encoding a polypeptide having the activity of a catalase (EC 1.11.1.6), which nucleic acid molecule is (a) a nucleic acid molecule encoding a polypeptide comprising or consisting of the amino acid sequence of SEQ ID NO: 1; (b) a nucleic acid molecule comprising or consisting of the nucleotide sequence of SEQ ID NO: 2; (c) a nucleic acid molecule comprising or consisting of a nucleic acid molecule encoding a polypeptide having the activity of a catalase the amino acid sequence of which is at least 99% identical to the amino acid sequence of SEQ ID NO: 1, (d) a nucleic acid molecule encoding a polypeptide having the activity of a catalase and comprising or consisting of a nucleotide sequence which is at least 98% identical to the nucleotide sequence of SEQ ID NO: 2, (e) a fragment of the polypeptide of any of (a) to (d) having the activity of a catalase and comprising with increasing preference at least 500 amino acids, at least 550 amino acids, at least 600 amino acids, at least 650 amino acids, or at least 675 amino acids, or (f) the nucleic acid sequence of any of (a) to (d) wherein T is U.

With regard to the fragment as defined in (e) it is preferred that this fragment comprises the core region of SEQ ID NO:1, which is the region extending from amino acid position 70 to amino acid position 460 of SEQ ID NO: 1.

In accordance with the present invention the term "nucleic acid molecule" defines a linear molecular chain consisting of at least 2025 nucleotides. The group of molecules designated herein as "nucleic acid molecules" also comprises complete genes. The term "nucleic acid molecule" is interchangeably used herein with the term "polynucleotide".

The term "nucleic acid molecule" in accordance with the present invention includes DNA, such as cDNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases. Included are also single- and double-stranded hybrid molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Polynucleotides may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphorarnidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule of the invention may additionally comprise promoters, enhancers, response elements, signal sequences, polyadenylation sequences, introns, 5'- and 3'- non-coding regions, and the like.

The term "polypeptide" as used herein interchangeably with the term "protein" describes linear molecular chains of amino acids, including single chain proteins or their fragments containing at least 675 amino acids. Polypeptides may further form oligomers consisting of at least two identical or different molecules. The corresponding higher order structures of such multimers are, correspondingly, termed homo- or heterodimers, homo- or heterotrimers etc.. The polypeptides of the invention may form heteromultimers or homomultimers, such as heterodimers or homodimers. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein" also refer to naturally modified polypeptides and proteins where the modification is effected e.g. by glycosylation, acetylation, phosphorylation, ubiqitinylation and similar modifications which are well known in the art.

The term "a polypeptide having the activity of a catalase (EC 1.11.1.6)" as used herein means a polypeptide which catalyzes the chemical reaction 2H₂O₂ → 2H₂O + O₂. The enzymatic activity of a catalase is thus the disproportionation of two molecules of hydrogen peroxide (H₂O₂) into one molecule water (H₂O) and one molecule oxygen (O₂).

In this regard it is noted that catalase activity can be assayed as described in the examples of the invention or by methods well known in the art. Free catalase activity may be for example monitored spectrophotometrically following the action of catalase on hydrogen peroxide, based upon the measurement of the ultraviolet absorption of peroxide (Beers and Sizer "A spectrophotometric method for measuring the breakdown of hydrogen peroxide by catalase" (1952), Journal of Biological Chemistry 195: 133-140).

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 98% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Amino acid sequence analysis and alignment in connection with the present invention was carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402) which is preferably employed in accordance with this invention. The skilled person is aware of additional suitable programs to align nucleic acid sequences.

As defined herein above, an amino acid sequence identity of at least 99% identity is envisaged by the invention. Furthermore, are envisaged with increasing preference amino acid sequence identities of at least 99,5 %, at least 99,8 %, and 100% identity by the invention.

As also defined herein above, a nucleotide sequence identity of at least 98% identity is envisaged by the invention. Furthermore, are envisaged with increasing preference nucleotide sequence identities of at least 99%, at least 99,5%, at least 99,8 %, and 100% identity by the invention.

In order to arrive at the desired sequence identity, the nucleic acid or polypeptide may be modified using methods known in the art, such as, mutations or introduction of truncations, substitutions, deletions and/or additions. For example, a nucleic acid derived from *Penicillium* spec. may be modified by altering the codons of the nucleic acid to reflect codon bias in an appropriate host cell and a catalase derived from *Penicillium* may be modified by substituting amino acids. Also encompassed are allelic variants, which denotes any of two or more alternative forms of a gene occupying the same chromosomal locus. Allelic variation arises naturally through mutation, and may result in polymorphism within populations. Gene mutations can be silent (no change in the encoded polypeptide) or may encode polypeptides having altered amino acid sequences. An allelic variant of a polypeptide is a polypeptide encoded by an allelic variant of a gene, wherein the allelic variant of a gene produces a change in the amino acid sequence of the polypeptide encoded therein.

Fragments according to the present invention are polypeptides having the activity of a catalase as defined herein above and comprise at least 675 amino acids. In this regard, it is preferred with increasing preference that the fragments according the present invention are polypeptides of at least, 680, at least 685 and at least 690 amino acids.

Fragments of the polypeptide of the invention, which substantially retain catalase activity, include N-terminal truncations, C-terminal truncations, amino acid substitutions, internal deletions and addition of amino acids (either internally or at either terminus of the protein). For example, conservative amino acid substitutions are known in the art and may be introduced into the catalase of the invention without substantially affecting catalase activity.

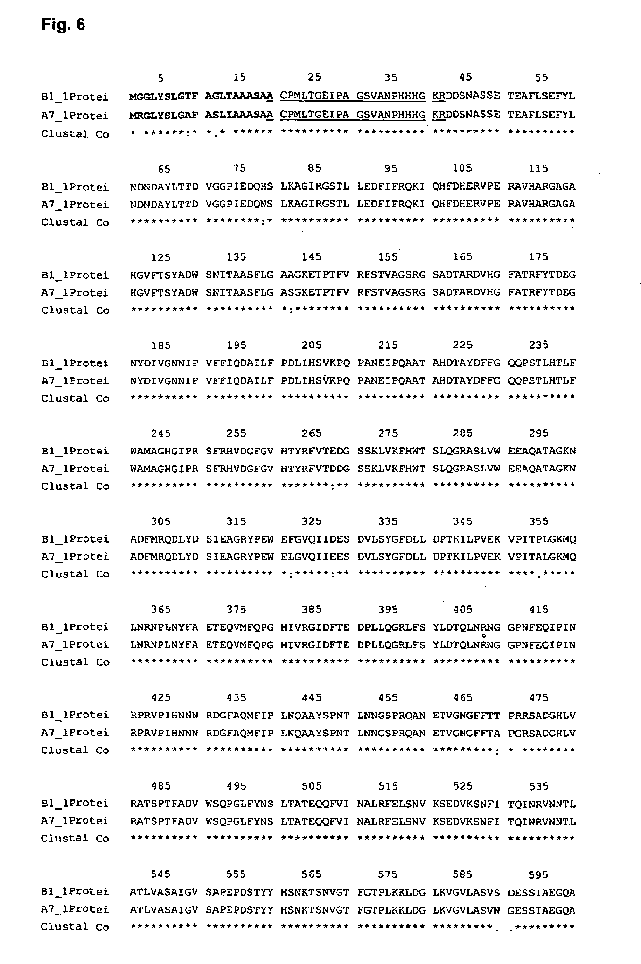

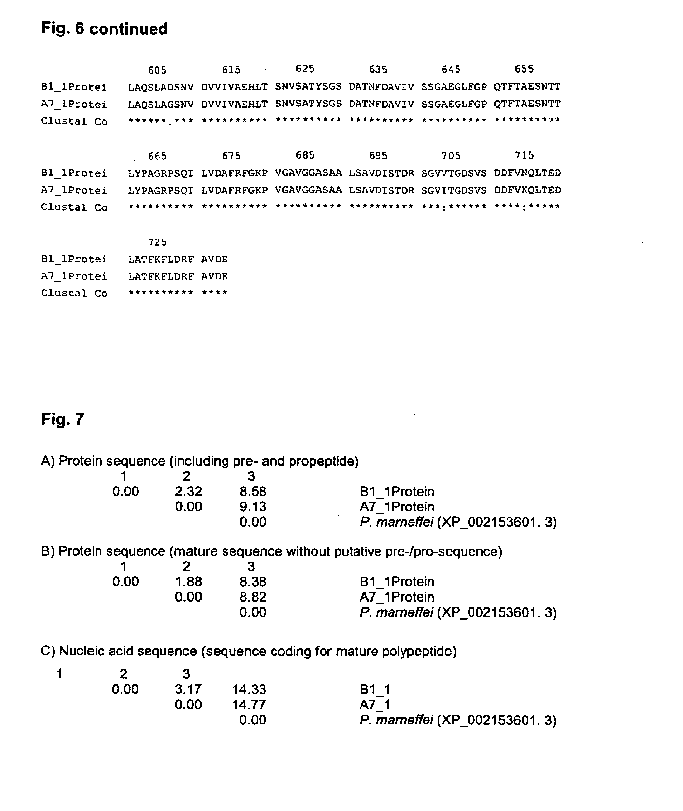

The catalases of the invention, is also termed B1_1 throughout this specification. As it can be seen in Figure 6 of the application the catalase of the invention B1_1 and the catalase A7_1 which is also described herein comprise an putative N-terminal signal peptide of 19 amino acids (sequence shown in bold in Figure 6) and directly C-terminal thereof a putative propeptide of 23 amino acids (underlined sequence in Figure 6). The signal peptide and propeptide together are also referred to as pre-propeptide herein. Accordingly, SEQ ID NOs: 1 and 3 show the amino acid sequence of the putative mature catalses B1_1 and A7_1 (corresponding amino acid positions 43 to 734 in Figure 6), respectively. SEQ ID NOs: 5 and 7 show the amino acid sequence of the catalses B1_1 and A7_1 including the putative propetide (corresponding to amino acid positions 20 to 734 in Figure 6), respectively. Furthermore, SEQ ID NOs: 9 and 11 show the amino acid sequence of the catalses B1_1 and A7_1 including the putative pre-propetide (corresponding to amino acid positions 1 to 734 in Figure 6), respectively. The amino acids sequences as set forth in SEQ ID NOs: 1, 3, 5, 7, 9 and 11 are encoded by the nucleotide sequences as set forth in SEQ ID NOs: 2, 4, 6, 8, 10 and 12, respectively. Without wishing to be bound by theory, the inventors believe that the polypeptide of SEQ ID NOs: 5, 7, 9 and 11, or encoded by SEQ ID NOs: 6, 8, 10 and 12 has the activity of a catalase after proteolytic cleavage, in particular if said cleavage results in a polypeptide of SEQ ID NOs: 1 or 3, or a polypeptide encoded by SEQ ID NOs: 2 or 4.

The nucleotide sequences as set forth in SEQ ID NOs: 6 and 10 and the nucleotide sequences encoding the amino acid sequences of SEQ ID NOs: 5 and 9 are particularly preferred examples of the nucleic acid molecule of the invention.

The term "propeptide" as used herein describes a linear molecular chain of amino acids, which characterizes a precursor of a protein and is cleaved during maturation or activation of the protein (e.g. the amino acid sequence of amino acids positions 19 to 42 in Figure 6 are cleaved off). The term "pre-propeptide" as used herein characterizes a precursor of a propeptide containing protein and further includes a signal peptide (e.g. the first 19 amino acids as shown in Figure 6 are cleaved off). The catalases of the invention including their signal peptide and propeptide are the primary translation product, which may be further proteolytically processed to the mature protein.

The term "signal peptide" as used herein defines a short amino acid sequence (preferably, the first 19 amino acids as shown in Figure 6) that directs the transport of a protein to a particular cell compartment and preferably to the endoplasmic reticulum.

Many of the catalase enzymes known in the art exhibit low thermal stability at temperatures above 60°C. Moreover, several catalases are rapidly inactivated in the presence of hydrogen peroxide, and most of the catalase enzymes have low activity and stability at elevated temperature and low or high pH, making them unsuitable for many applications. In the literature many examples for thermostable catalases can be found. E.g. catalases of the manganese type have been found in thermophilic and hyperthermophilic organisms, such as *Pyrobaculum calidifontis* (Amo, T., et al. Unique presence of a manganese catalase in a hyperthermophilic Archeon Pyrobaculum calidifontis VA1, J. Bacteriol., June 2002, 3305-3312). WO 2005/044994 A2 describes thermal and pH stable catalases from the thermophilic organism *Thermus brockianus.*

WO 1992/017571 discloses a catalase from *Scytalidium thermophilum* with activity at temperatures of up to 75°C and EP 2256192 A1 describes a catalase isolated from *Humicola grisea* and *Penicillium pinophilum* with activity at temperatures of up to 70°C.

However, to the best knowledge of the inventors the monofunctional heme-containing catalases in the art exhibit little to no activity under conditions of elevated temperature up to 90°C and at low pH such as a pH of 4.0.

Here we disclose a monofunctional, heme-containing catalase. The catalases of the invention, termed B1_1, was purified and characterized from the genus *Penicillium.* As a part of the characterization, the enzyme was tested for H₂O₂-formation under different assay conditions and found to be significantly more thermal/acid stable than these other enzymes.

In more detail, the novel thermal and low pH stable catalase of the invention from the species *Penicillium* was successfully expressed in heterologous hosts such as *Trichoderma reseei, Pichia pastoris* and *Aspergillus niger.* Surprisingly, the catalases of the invention, termed B1_1 (amino acid sequence of SEQ ID NO: 1, nucleotide sequence of SEQ ID NO: 2), showed high efficiency of conversion of hydrogen peroxide of up to 100% at 80°C, which considerably exceeds the temperature which was applied by the inventors during the screening procedure (65°C) leading to the identification of the catalase expressing *Penicillium* strain. Moreover, conversion of hydrogen peroxide of up to 100 % was observed at acidic conditions (pH4) at 65°C and 80°C. Even at the combination of extreme conditions of a temperature of 90°C and pH4 substantial peroxide conversion percentages of 64 and 78, respectively, were measured in two different media (cf. Table 1).

The catalase derived from *Penicillium* strainPEN_2 (catalase B1_1) shows unexpectedly high activity at high temperature- and low pH-, which to the best knowledge of the inventors has been found in this combination for the first time. This is particularly evident from a comparison of the catalase of the invention which is derived from the penicillium strain PEN_2 (catalase B1_1) with a further catalase described herein which is derived from the penicillium strains PEN_1 (catalase A7_1). As it is evident from Table 1 in the examples provided herein below, the catalase B1_1 shows at extreme conditions of 90°C and pH of 4 (and in two different media) a superior peroxide conversion (expressed as lower percentage of residual H₂O₂) as the catalase A7_1.

In this regard, it is noteworthy that the amino acid sequence identity of B1_1 (SEQ ID NO: 1) and A7_1 (SEQ NO: 3) is 98.1% (Figure 6). The nucleotide sequence identity of B1_1 (SEQ ID NO: 2) and A7_1 (SEQ NO: 4) is 96.8%. This indicates that the few amino acid changes between the catalases B1_1 and A7_1 result in a superior peroxide conversion of the catalase B1_1 at 90°C and pH 4. To the best knowledge of the inventors, no prior art catalase shows an as good catalytic activity at 90°C and pH 4 as the catalase B1_1.

In a further embodiment, the invention relates to a polypeptide encoded by the nucleic acid molecule of the invention.

The amino acid sequences of SEQ ID NOs: 1, 5 and 9 are particularly preferred examples of the polypeptide of the invention. Most preferred is the amino acid sequence of SEQ ID NOs: 1.

In another embodiment the invention relates to a vector comprising the nucleic acid molecule of the invention.

A vector according to this invention is generally and preferably capable of directing the replication, and/or the expression of the nucleic acid molecule of the invention and/or the expression of the polypeptide encoded thereby.

Preferably, the vector is a plasmid, cosmid, virus, bacteriophage or another vector used conventionally e.g. in genetic engineering.

Exemplary plasmids and vectors are listed, for example, in Studier and coworkers (Studier, W.F.; Rosenberg A.H.; Dunn J.J.; Dubendroff J.W., 1990, Use of the T7 RNA polymerase to direct expression of cloned genes, Methods Enzymol. 185, 61-89) or the brochures supplied by the companies Novagen, Promega, New England Biolabs, Clontech and Gibco BRL. Other preferred plasmids and vectors can be found in: Glover, D.M., 1985, DNA cloning: a practical approach, Vol. I-III, IRL Press Ltd., Oxford; Rodriguez, R.L. and Denhardt, D.T. (eds), 1988, Vectors: a survey of molecular cloning vectors and their uses, 179-204, Butterworth, Stoneham; Goedeel, D.V., 1990, Systems for heterologous gene expression, Methods Enzymol. 185, 3-7; Sambrook, J.; Russell, D. W., 2001, Molecular cloning: a laboratory manual, 3rd ed., Cold Spring Harbor Laboratory Press, New York.

Non-limiting examples of suitable vectors include prokaryotic plasmid vectors, such as the pUC-series, pBluescript (Stratagene), the pET-series of expression vectors (Novagen) or pCRTOPO (Invitrogen), lambda gt11, pJOE, the pBBR1-MCS series, pJB861, pBSMuL, pBC2, pUCPKS, pTACT1 and vectors compatible with expression in mammalian cells like pREP (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, Okayama-Berg cDNA expression vector pcDV1 (Pharmacia), pRc/CMV, pcDNA1, pcDNA3 (Invitrogene), pSPORT1 (GIBCO BRL), pGEMHE (Promega), pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Invitrogen).

The nucleic acid molecule of the present invention referred to above may also be inserted into vectors such that a translational fusion with another nucleic acid molecule is generated. To this aim, overlap extension PCR can be applied (e.g. Wurch, T., Lestienne, F., and Pauwels, P.J., A modified overlap extension PCR method to create chimeric genes in the absence of restriction enzymes, Biotechn. Techn. 12, 9, Sept. 1998, 653-657). The products arising therefrom are termed fusion proteins and will be described further below. The other nucleic acid molecules may encode a protein which may e.g. increase the solubility and/or facilitate the purifcation of the protein encoded by the nucleic acid molecule of the invention. Non-limiting examples include pET32, pET41, pET43. The vectors may also contain an additional expressible nucleic acid coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21 (DE3)PRARE) or Rosetta®.

Particularly preferred plasmids which can be used to introduce the nucleic acid encoding the polypeptide of the invention having the activity of a catalase into the host cell are: pUC18/19 (Roche Biochemicals), pKK-177-3H (Roche Biochemicals), pBTac2 (Roche Biochemicals), pKK223-3 (Amersham Pharmacia Biotech), pKK-233-3 (Stratagene) and pET (Novagen). Further suitable plasmids are listed in PCT/EP03/07148. Very particular preference is given to an expression system which is based on plasmids belonging to the pET series.

For vector modification techniques, see Sambrook and Russel, 2001. Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, transcriptional termination sequences, promoters, enhancers, and/or insulators), internal ribosomal entry sites (IRES) (Owens *et al.,* 2001) and optionally poly-A signals ensuring termination of transcription and stabilization of the transcript. Additional regulatory elements may include transcriptional as well as translational enhancers, and/or naturally-associated or heterologous promoter regions. The regulatory elements may be native to the catalase of the invention or heterologous regulatory elements. Preferably, the nucleic acid molecule of the invention is operably linked to such expression control sequences allowing expression in prokaryotes or eukaryotic cells. The vector may further comprise nucleotide sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the nucleic acid molecule of the invention. Such leader sequences are well known in the art. Specifically designed vectors allow the shuttling of DNA between different hosts, such as bacteria-fungal cells or bacteria-animal cells.

The nucleic acid molecules of the invention as described herein above may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as vector in eukaryotic expression system for the nucleic acid molecules of the invention. Expression vectors derived from viruses such as retroviruses, vaccinia virus, adeno-associated virus, herpes viruses, or bovine papilloma virus, may be used for delivery of the nucleic acids or vector into targeted cell population. Methods which are well known to those skilled in the art can be used to construct recombinant viral vectors; see, for example, the techniques described in Sambrook, 2001 and Ausubel, 2001.

Promoters which are particularly advantageous for implementing the invention and which are to be used, in particular, in *E. coli* are known to the skilled person (Sambrook, J.; Fritsch, E.F. and Maniatis, T. (1989), Molecular cloning: a laboratory manual, 2nd ed., Cold Spring Harbor Laboratory Press, New York). Further suitable promoters are those selected from T7, lac, tac, trp, ara or rhamnose-inducible promoters. Other promoters are mentioned in (Cantrell, SA (2003) Vectors for the expression of recombinant proteins in E. coli. Methods in Molecular biology 235: 257-275; Sawers, G; Jarsch, M (1996) Alternative principles for the production of recombinant proteins in Escherichia coli. Applied Microbiology and Biotechnology 46(1): 1-9). Very particular preference is given to using the T7 promoter in the vector according to the invention (Studier, W.F.; Rosenberg A.H.; Dunn J.J.; Dubendroff J.W.; (1990), Use of the T7 RNA polymerase to direct expression of cloned genes, Methods Enzymol. 185, 61-89; or brochures supplied by the companies Novagen or Promega).

Examples for regulatory elements permitting expression in eukaryotic host cells are the *AOX1* or *GAL1* promoter in yeast or the CMV- (Cytomegalovirus), SV40-, RSV-promoter (Rous sarcoma virus), chicken beta-actin promoter, CAG-promoter (a combination of chicken beta-actin promoter and cytomegalovirus immediate-early enhancer), the gai10 promoter, human elongation factor 1α-promoter, CMV enhancer, CaM-kinase promoter, the Autographa californica multiple nuclear polyhedrosis virus (AcMNPV) polyhedral promoter or a globin intron in mammalian and other animal cells. Besides elements which are responsible for the initiation of transcription such regulatory elements may also comprise transcription termination signals, such as the SV40-poly-A site or the tk-poly-A site or the SV40, lacZ and AcMNPV polyhedral polyadenylation signals, downstream of the nucleic acid.

The co-transfection with a selectable marker such as kanamycin or ampicillin resistance genes for culturing in *E. coli* and other bacteria allows the identification and isolation of the transfected cells.

Selectable markers for mammalian cell culture are the dhfr, gpt, neomycin, hygromycin resistance genes. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992).

Using such markers, the cells are grown in selective medium and the cells with the highest resistance are selected.

In a further embodiment the invention relates to a host cell carrying the vector of the invention.

The host cell of the invention may "carry" the vector of the invention due to a transformation, transduction or transfection of the host cell with the vector. Accordingly, also describes herein is a host cell transformed, transduced or transfected with the vector of the invention.

Large amounts of the catalase may be produced by said transformed host, wherein the isolated nucleotide sequence encoding the catalase, or a functional fragment thereof, is inserted into an appropriate vector or expression vector before insertion into the host. The vector or expression vector is introduced into an appropriate host cell, which preferably can be grown in large quantities, and the catalase is purified from the host cells or the culture media.

The host cells may also be used to supply the catalase of the invention without requiring purification of the catalase (see Yuan, Y.; Wang, S.; Song, Z.; and Gao, R., Immobilization of an L-aminoacylase-producing strain of Aspergillus oryzae into gelatin pellets and its application in the resolution of D,L-methionine, Biotechnol Appl. Biochem. (2002). 35:107-113). For example, the catalase of the invention may be secreted by host cells, which are contacted with a hydrogen peroxide solution. Those skilled in the field of molecular biology will understand that any of a wide variety of expression systems may be used to provide the catalase protein. The precise host cell used is not critical to the invention, so long as the host cells produce the catalase when grown under suitable growth conditions.

Host cells into which vectors containing the nucleic acid molecule of the invention can be cloned are used for replicating and isolating a sufficient quantity of the recombinant enzyme. The methods used for this purpose are well known to the skilled person (Sambrook, J.; Fritsch, E.F. and Maniatis, T. (1989), Molecular cloning: a laboratory manual, 2nd ed., Cold Spring Harbor Laboratory Press, New York).

Suitable prokaryotic host cells comprise e.g. bacteria of the species Escherichia, such as strains derived from *E. coli* BL21 (e.g. BL21(DE3), BL21(DE3)PlysS, BL21(DE3)RIL, BL21(DE3)PRARE, BL21 codon plus, BL21(DE3) codon plus), Rosetta®, XL1 Blue, NM522, JM101, JM109, JM105, RR1, DH5α, TOP 10, HB101 or MM294. Further suitable bacterial host cells are *Streptomyces, Salmonella* or *Bacillus* such as *Bacillus subtilis. E. coli* strains are preferred prokaryotic host cells in connection with the present invention.

Suitable eukaryotic host cells are e.g. yeasts such as *Saccharomyces cerevisiae, Hansenula polymorpha* or *Pichia sp.* such as *P. pastoris,* insect cells such as Drosophila S2 or Spodoptera Sf9 cells, plant cells, or fungi *cells, preferably of the* family *Trichocomaceae,* more preferably of the genus *Aspergillus, Penicillium* or *Trichoderma reseei.*

Very particular preference is given to an expression system which is present in *E. coli* DH18BΔkat E (Invitrogen) as a procaryotic host and *Pichia pastoris* or *Aspergillus niger* as a eucaryotic host. In general, a eukaryotic host cell is preferred over a procaryotic host cell.

Mammalian host cells that could be used include human Hela, HEK293, H9 and Jurkat cells, mouse NIH3T3 and C127 cells, COS 1, COS 7 and CV1, quail QC1-3 cells, mouse L cells, Bowes melanoma cells and Chinese hamster ovary (CHO) cells.

In another embodiment the invention relates to a method of producing a protein having the activity of a catalase (EC 1.11.1.6) comprising (a) culturing the host cell of the invention, and (b) isolating the produced protein having the activity of a catalase.

Suitable conditions for culturing a prokaryotic or eukaryotic host are well known to the person skilled in the art. Suitable conditions for culturing *E*. *coli* DH18BΔkat E (Invitrogen), *Pichia pastoris* or *Aspergillus niger* are, for example provided in the examples of the invention. In general, suitable conditions for culturing bacteria are growing them under aeration in Luria Bertani (LB) medium. To increase the yield and the solubility of the expression product, the medium can be buffered or supplemented with suitable additives known to enhance or facilitate both. *E. coli* can be cultured from 4 to about 37 °C, the exact temperature or sequence of temperatures depends on the molecule to be overexpressed. In general, *Aspergillus sp.* may be grown on Sabouraud dextrose agar, or potato dextrose agar at about to 10°C to about 40°C, and preferably at about 25°C. Suitable conditions for yeast cultures are known, for example from Guthrie and Fink, "Guide to Yeast Genetics and Molecular Cell Biology" (2002); Academic Pr Inc.. The skilled person is also aware of all these conditions and may further adapt these conditions to the needs of a particular host species and the requirements of the polypeptide expressed. In case an inducible promoter controls the nucleic acid of the invention in the vector present in the host cell, expression of the polypeptide can be induced by addition of an appropriate inducing agent. Suitable expression protocols and strategies are known to the skilled person.

Depending on the cell type and its specific requirements, mammalian cell culture can e.g. be carried out in RPMI or DMEM medium containing 10% (v/v) FCS, 2mM L-glutamine and 100 U/ml penicillin/streptomycin. The cells can be kept at 37 °C in a 5% CO₂, water saturated atmosphere.

Suitable media for insect cell culture is e.g. TNM + 10% FCS or SF900 medium. Insect cells are usually grown at 27 °C as adhesion or suspension culture.

Suitable expression protocols for eukaryotic cells are well known to the skilled person and can be retrieved e.g. from in Sambrook, 2001.

Methods of isolation of the polypeptide produced are well-known in the art and comprise without limitation method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook, 2001.

The step of protein isolation is preferably a step of protein purification. Protein purification in accordance with the invention specifies a process or a series of processes intended to further isolate the polypeptide of the invention from a complex mixture preferably to homogeneity. Purification steps, for example, exploit differences in protein size, physico-chemical properties and binding affinity. For example, proteins may be purified according to their isoelectric points by running them through a pH graded gel or an ion exchange column. Further, proteins may be separated according to their size or molecular weight via size exclusion chromatography or by SDS-PAGE (sodium dodecyl sulfate-polyacrylamide gel electrophoresis) analysis. In the art, proteins are often purified by using 2D-PAGE and are then further analysed by peptide mass fingerprinting to establish the protein identity. This is very useful for scientific purposes and the detection limits for protein are very low and nanogram amounts of protein are sufficient for their analysis. Proteins may also be separated by polarity/hydrophobicity via high performance liquid chromatography or reversed-phase chromatography. Thus, methods for protein purification are well known to the skilled person and are exemplified in the examples of the invention.

In a preferred embodiment, the polypeptide of the invention is a fusion protein.

In addition to the amino acid sequence of the polypeptide of the present invention (which has the activity of a catalase), a fusion protein according to the present invention contains at least one additional, heterologous amino acid sequence. Often, but not necessarily, these additional sequences will be located at the N- or C-terminal end of the polypeptide. It may e.g. be convenient to initially express the polypeptide as a fusion protein from which the additional amino acid residues can be removed, e.g. by a proteinase (e.g. thrombin, factor VIII, factor Xa protease, or PreScission Protease) capable of specifically trimming the polypeptide of the present invention.

For example, the heterologous amino acid sequence may be a tag. Tags are attached to proteins for various purposes. Affinity tags are appended to proteins so that they can be purified from their crude biological source using an affinity technique. These include but are not limited to chitin binding protein (CBP), maltose binding protein (MBP), glutathione-S-transferase (GST), and poly(His) tag. The poly(His) tag is a widely used protein tag; it binds to metal matrices. Solubilization tags are used, especially for recombinant proteins expressed in chaperone-deficient species such as *E. coli,* to assist in the proper folding in proteins and keep them from precipitating. These include but are not limited to thioredoxin (TRX) and poly(NANP). Some affinity tags have a dual role as a solubilization agent, such as MBP, and GST. Chromatography tags are used to alter chromatographic properties of the protein to afford different resolution across a particular separation technique. Chromatography tags comprise but are not limited to of polyanionic amino acids, such as FLAG-tag. Epitope tags are short peptide sequences which are chosen because high-affinity antibodies can be reliably produced in many different species. These are usually derived from viral genes, which explain their high immunoreactivity. Epitope tags include but are not limited to V5-tag, c-myc-tag, and HA-tag. These tags are particularly useful for western blotting and immunoprecipitation experiments, although they also find use in antibody purification. Fluorescence tags are used to give visual readout on a protein. For example, GFP and its variants are the most commonly used fluorescence tags. More advanced applications of GFP include using it as a folding reporter (fluorescent if folded, colorless if not). Moreover, tags find many other usages, such as specific enzymatic modification (such as biotin ligase tags) and chemical modification (FIAsH) tag. Examples of suitable tags to be used in accordance with the invention comprise but are not limited to lacZ, GST, maltose-binding protein, NusA, BCCP, c-myc, CaM, His, FLAG, GFP, YFP, cherry, thioredoxin, poly(NANP), V5, Snap, HA, chitin-binding protein, Softag 1, Softag 3, Strep, or S-protein, and furthermore tags comprising a binding domain capable of binding, directly or indirectly, to the skin.

In this connection, the term a "binding domain capable of binding, directly or indirectly to the skin" specifies a binding domain which binds to an epitope being present directly on the skin or an epitope being present on dermal appendages like hair, glands or nails. In this regard, an epitope has to be understood as the part of a macromolecule (e.g. collagen, keratin, polysaccharides or polysaccharide derivatives, melamine-type polymers, or polyurea) that binds to the binding domain according to the invention. Such binding domains may bind to collagen, keratin, polysaccharides or polysaccharide derivatives, to melamine-type polymers, or to polyurea. Preferably the binding domain is a collagen binding domain, a keratin binding domain, a melamine binding domain or a polyurea binding domain. The collagen binding domain is preferably an epidermal collagen binding domain. The keratin binding domain is preferably a keratin 1, 2, 3, 4, 5, 6, 7, or 8 binding domain. Melamine binding domains are peptide/polypeptide binding domains that bind to melamine (a repeating unit of C₃N₆H₆ [1,3,5 triazine 2,4,6 triamine]) or a melamine-like polymer. Melamine polymers are commonly used as encapsulation materials.

Exemplary fusion proteins of the polypeptide of the invention may assist in expression and/or purification of the protein.

In a further embodiment the invention relates to a composition comprising the nucleic acid molecule of the invention, the polypeptide of the invention, the vector of the invention, the host cell of the invention, and/or the fusion protein of the invention, or combinations thereof.

According to a preferred embodiment of the invention, the composition is a large-scale composition, a pharmaceutical composition, a diagnostic composition or a cosmetic composition.

In accordance with the invention a "large-scale composition" refers to a composition involved in the production of an economic good within an economy with the exception of pharmaceuticals, diagnostics and cosmetic which are further detailed herein below.

Catalases are, for example, widely used in the food industry for removing hydrogen peroxide from beverage and food, for example in milk and/or cheese production (Catalase, Worthington Enzyme Manual. Worthington Biochemical Corporation, http://www.worthingtonbiochem.com/CTL/default.html). The processing of milk leads to low pH conditions (e.g. the pH of whey and yoghurt is in the range of 3.8-5.5). Another use is in food or food wrappers where a catalase prevents food from oxidizing (Hengge A (1999). "Re: how is catalase used in industry?", General Biology. MadSci Network). Catalase is also used in the textile, pulp or paper industry for removing hydrogen peroxide from fabrics to make sure that the material is peroxide-free and/or to stop a bleaching reaction of textile, pulp or paper (e.g. textile industry, Case study 228. International Cleaner Production Information Clearinghouse.).

According to a preferred embodiment of the present invention, the large-scale composition is therefore for processing textile, beverage, food, pulp or paper.

As discussed herein above, catalases are used in the processing of textiles, pulp and paper and for the removal of hydrogen peroxide used for disinfection of food and beverages. In these processes sometimes high temperatures occur. Today mainly chlorine dioxide is used for bleaching textile, pulp, paper, food, as well as skin (and in a number of other industries).

Over 95% of the chlorine dioxide produced in the world today is made from sodium chlorate and is used for pulp bleaching. Chlorine dioxide is sometimes used in combination with chlorine, but it may be also used alone in ECF (elemental chlorine-free) bleaching sequences. It is in general used at a moderately acidic pH of 3.5 to 6. Since the catalase of the invention even displays considerable catalytic activity at a temperature of 90°C and a pH of about 4 (cf. Table 1), this catalase is particularly useful for industrial processing, like processing textile, pulp and paper and food. For example, for the bleaching of linen chlorine dioxide is used in combination with H₂O₂. The catalase of the invention can be used for removing H₂O₂.in the linen bleaching process.

In accordance with the present invention, the term "pharmaceutical composition" relates to a composition for administration to a patient, preferably a human patient. The pharmaceutical composition is preferably used in a disease associated with oxidative stress, a disease associated with overproduction of hydrogen peroxide, or a disease associated with the loss of function or reduced function of a catalase. A non-limiting example of a disease associated with the loss of function or reduced function of a catalase is acatalasia (also known in the art as acatalasemia, or Takahara's disease), which is an autosomal recessive peroxisomal disorder caused by a complete lack of catalase. Acatalasia causes an increased incidence of periodontal infections, and can under rare circumstances lead to gangrene. A non-limiting example of a disease associated with overproduction of hydrogen peroxide and/or oxidative stress is vitiligio (e.g. Liu et al. (2010), Journal of Investigative Dermatology, 130:2647-2653), which is a chronic disorder that causes severe depigmentation of patches of skin. Another non-limiting example of a disease associated with a decreased catalase level is polymorphic light eruption (PLE) (Guarrera et al. (1998), Acta Derm Venerol, 78:335-336), which is an allergic skin reaction caused by sunlight induced oxidative stress. Further non-limiting examples of disorders associated with oxidative stress are acne vulgaris and alopecia areata, for which a topical treatment with a catalase comprising composition can be envisioned. Further non-limiting examples of diseases characterized by the occurrence of oxidative stress are Acne vulgaris (Sarici et al. (2010), J Eur Acad Dermatil Venerol, 24(7):763-7), Alopecia areatea (Abdel Fattah et al. (2010) J Eur Acad Dermatil Venerol, Epub ahead of print), Lichen planus (Aly, DG, Shahin, RS (2010) Acta Dermatoverol. Alp Panonica Adriat. 19,(1):3-11), Psoriasis, and Rheumatoid arthritis (Coaccioli, S. et al. (2009), 160(6): 467-72).

As it is evident from the examples, the catalases of the invention are particularly useful for converting hydrogen peroxide under acidic conditions. Since the skin has a pH of between 4.5 and 6.2 (also known in the art as acid mantle of the skin), the catalases of the invention are particularly useful for applications to the skin.

The pharmaceutical composition of the invention comprises the compounds recited above, alone or in combination. The composition may be in solid or liquid form and may be, *inter alia,* in the form of (a) powder(s), (a) solution(s) or (an) aerosol(s), cream(s), ointment(s) or gel(s). The pharmaceutical composition of the present invention may, optionally and additionally, comprise a pharmaceutically acceptable carrier. By "pharmaceutically acceptable carrier" is meant a non-toxic solid, semisolid or liquid filler, diluent, encapsulating material or formulation auxiliary of any type. Examples of suitable pharmaceutical carriers are well known in the art. Compositions comprising such carriers can be formulated by well known conventional methods. The pharmaceutical composition can be administered topically. The dosage regimen corresponding to a suitable dose for administration will be determined by the attending physician and clinical factors which may, inter alia, depend on the size of the area to be treated, the stage or severity of its condition. The concentration of the compound(s) as recited above in a composition for topical application can be in the range of 0.001 to 1% (w/w), preferably 0.01-0.1 % (w/w). Topical application is preferably repeated in one or more than one daily applications.

The pharmaceutical composition of the invention can be applied in combination with (solid) carriers or matrices such as dressing(s), band aid(s) or tape(s). The compound(s) can be covalently or non-covalently bound to said carrier or matrix. For example, the compound(s) may be incorporated into a dressing to be applied over the area to be treated. Examples of such dressings include staged or layered dressings incorporating slow-release hydrocolloid particles. The concentration of a solution of the pharmaceutical composition to be immobilised in a matrix of a dressing is generally in the range of 0.001 to 1% (w/v) preferably 0.01-0.1% (w/v). Furthermore, the compound(s) as recited above can be incorporated into a suitable material capable of delivering the enzyme to the area to be treated in a slow release or controlled release manner.

A gel formulation of the pharmaceutical composition of the present invention further comprises at least one gel forming agent commonly used in pharmaceutical gel formulations. Examples of gel forming agents are cellulose derivatives such as methyl cellulose, hydroxyethyl cellulose, and carboxymethyl cellulose; vinyl polymers such as polyvinyl alcohols, polyvinyl pyrrolidones; and carboxypoly-methylene derivatives such as carbopol. Further gelling agents that can be used for the present invention are pectins, gums, alginates, agar and gelatine. Furthermore, the gel or emugel formulation may contain auxiliary agents commonly used in this kind of formulations such as preservatives, antioxidants, stabilizers, colorants and perfumes.

In accordance with a more preferred embodiment the pharmaceutical composition of the invention is for use in treating a disease characterized by impaired catalase such as vitiligo, acatalasia, polymorphic light eruption (PLE) or a disease characterized by the occurrence of oxidative stress such as Acne vulgaris, Alopecia areatea, Lichen planus, Psoriasis and Rheumatoid arthritis.

Furthermore, catalases are an useful ingredient in diagnostic compositions. For example, a over- or underproduction of hydrogen peroxide by an individual or a microbial (preferably bacterial) infection with a microbal organism (preferably a bacteria) which metabolizes or produces hydrogen peroxide may be diagnosed using a diagnostic compositions comprising a catalase.

A cosmetic composition according to the invention is for use in non-therapeutic applications.

Cosmetic compositions may also be defined by their intended use, as compositions intended to be rubbed, poured, sprinkled, or sprayed on, or otherwise applied to the human body for cleansing, beautifying, promoting attractiveness, or altering the appearance.

The particular formulation of the cosmetic composition according to the invention is not limited. Envisaged formulations include rinse solutions, emulsions, creams, milks, gels such as hydrogels, ointments, suspensions, dispersions, powders, solid sticks, foams, sprays and shampoos. For this purpose, the cosmetic composition according to the invention may further comprise cosmetically acceptable diluents and/or carriers. Choosing appropriate carriers and diluents in dependency of the desired formulation is within the skills of the skilled person. Suitable cosmetically acceptable diluents and carriers are well known in the art and include agents referred to in Bushell et al. (WO 2006/053613). Preferred formulations for said cosmetic composition are rinse solutions and creams.

The application of the composition of the invention in cosmetics is, for example, aiming at treating the skin and skin appendages (e.g. hair and nails) enzymatically for converting hydrogen peroxide into water and oxygen. A suitable concentration of the compound(s) of the invention for cosmetic use is believed to be in the range of 0,0001 to 1% (w/v), preferably 0,0001 to 0, 1% (w/v), even more preferably 0,001 to 0,1 % (w/v).

Preferred amounts of the cosmetic compositions according to the invention to be applied in a single application are between 0.1 and 10g, more preferred between 0.1 and 1g, most preferred 0.5g. The amount to be applied also depends on the size of the area to be treated and has to be adapted thereto.

Catalases may be used to decompose hydrogen peroxide used to inhibit growth of bacteria and to remove hydrogen peroxide from the surface of the skin or even within the skin tissue. This effect results in skin smoothening and thus qualifies catalases as ingredient in anti-aging cosmetic compositions or as ingredient in, for example, a face mask for improving skin appearance. As it is well-known in the art, skin has an acidic pH of about 5.5. Since the catalase of the invention even displays catalytic activity at a pH of 4 (cf. Table 1), this catalase is particularly useful for the development of cosmetic compositions.

Low levels of catalase are supposed to play a role in the greying process of human hair. Hydrogen peroxide is naturally produced by the body and catalase breaks it down. If there is a dip in catalase levels, hydrogen peroxide cannot be broken down. This causes the hydrogen peroxide to bleach the hair from the inside out. Thus, it is believed in the art catalases may be incorporated into anti-greying treatments, i.e. to prevent aging of hair (e.g., Hitti M (2009-02-25). "Why Hair Goes Gray", Health News. WebMD; and Wood et al. (2009), FASEB J. 23 (7): 2065-75).

Some commercial contact lens-cleaning products disinfect the lens using a hydrogen peroxide solution. A solution containing catalase is then used to decompose the hydrogen peroxide before the lens can be used again (US 5,521,091).

Pigmentary abnormalities of the skin are in accordance with the invention preferably depigmentation abnormalities. Depigmentation may be associated with skin bleaching caused by hydrogen peroxiode. Hydrogen peroxide in turn is enzymatically cleaved by catalases as detailed herein above.

In accordance with a more preferred embodiment of the invention the cosmetic composition is for anti-aging, improving skin appearance, preventing hair greying, cleaning contact lenses and/or correcting pigmentary abnormalities.

In a further embodiment the present invention relates to a method of converting hydrogen peroxide into water and oxygen comprising contacting the nucleic acid molecule of the invention, the polypeptide of the invention, the vector of the invention, the host cell of the invention, and/or the fusion protein of the invention, or combinations thereof with a sample comprising hydrogen peroxide.

In accordance with this method its is preferred that methods for treatment of the human or animal body by surgery or therapy and diagnostic methods practised on the human or animal body are excluded.

In processes where hydrogen peroxide is used as a bleaching or antibacterial agent, the catalase may be used to remove or reduce residual hydrogen peroxide, for example, in contact lens cleaning, in bleaching steps in pulp and paper preparation, dairy production, production of beverages and others.

In another embodiment the invention also relates to the use of the nucleic acid molecule of the invention, the polypeptide of the invention, the vector of the invention, the host cell of the invention, and/or the fusion protein of the invention, or combinations thereof for the conversion of hydrogen peroxide into water and oxygen.

Thus, the invention may be used to treat, e.g. hydrogen peroxide containing solutions in order to convert hydrogen peroxide to water and oxygen. In particular, the invention may be used where the conditions for catalysis are at a high temperature, a low pH, a high concentration of hydrogen peroxide, or a combination thereof. A high temperature includes temperatures preferably between 60°C and 100°C, more preferably between 65°C and about 95°C. An acidic or low pH includes preferably a pH between 2 and 9, more preferably a pH between 3 and 7 and most preferably a pH between 4 and 6.

Moreover, the catalases of the invention may be used as one catalyst involved in a oxidation reaction, e.g., epoxidation and hydroxylation.

Catalases may be used, for example, to remove or reduce the concentration of H₂O₂ for environmental (pollution control/clean-up) purposes. Furthermore the stress induced by the presence of H₂O₂ during the culturing of microorganisms can be significantly reduced by addition of catalases, thus increasing the yield of cultured microorganisms or actually enabling the cultivation of isolated microorganisms with a high sensitivity to peroxide.

As explained herein above, catalases have the enzymatic activity of converting hydrogen peroxide into water and oxygen. Thus, catalases may be used to generate or supply oxygen by treating hydrogen peroxide in the presence of the catalase. Accordingly, the enzymatic conversion by catalase increases the oxygen content which may for example facilitate or enhance bacterial growth of aerobic microorganisms in fermentation processes.

In general the catalases may be combined with further enzymes. For example, a combination of glucose oxidase and catalase may be used for desugaring.

In accordance with a preferred embodiment of the method of the invention or the use of the invention, the conversion serves for controlling or stopping bleaching of a industrial product.

Accordingly, also described herein is a method of controlling or stopping the bleaching of a industrial product comprising contacting the nucleic acid molecule of the invention, the polypeptide of the invention, the vector of the invention, the host cell of the invention, and/or the fusion protein of the invention, or combinations thereof with a bleaching solution used in textile, pulp, or paper industry, wherein the bleaching solution comprises hydrogen peroxide.

Furthermore, described herein is the use of the nucleic acid molecule of the invention, the polypeptide of the invention, the vector of the invention, the host cell of the invention, and/or the fusion protein of the invention, or combinations thereof for controlling or stopping the bleaching of a large-scale product by hydrogen peroxide.

In accordance with an even more preferred embodiment of the method and/or the use of the invention, the industrial product is textile, pulp or paper.

Pulp bleaching and brightening with hydrogen peroxide is commonly used in the pulp and paper industry. Thus, catalases may be used to remove hydrogen peroxide following brightening or bleaching of, for example, textile, pulp or paper.

The Figures show
- FIG. 1:: A. two-dimensional native PAGE (Coomassie stain), 100 times concentrated supernatant from *Pencillium* PEN-2 culture supernatant; B, SDS-PAGE (Coomassie stain) of culture supernatant of strain PEN-2 purified by electroelution showing the apparent molecular weight of catalase of approx. 95 kDa

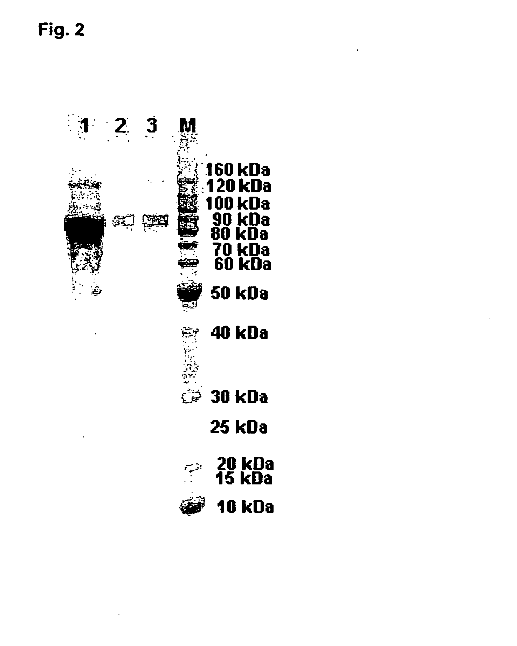

- FIG. 2:: Expression in *Aspergillus niger* and SDS-PAGE analysis of supernatant of PEN-2: lane 1: undiluted, lane 2: 1:20, lane 3: 1:10, lane M: molecular weight marker
- FIG. 3:: Expression in *Pichia pastoris* and purification of culture supernatant by gel filtration and analysis of fractions on SDS-PAGE (lane No. = fraction No.).
- FIG. 4:: Screening for growth of *Pichia* clones after UV-mutagenesis in the presence of H₂O₂ (plate with 96 cavities observed from the bottom). White cavities indicate growth in the presence of H₂O₂. Cavities marked by circles contained mutant of clone B1_1 in two parallel cultures.
- FIG. 5:: Proof of activity of heterologously expressed catalase in *Aspergillus* culture supernatant ("In gel enzyme assay" see example 1).
- FIG.6:: Alignment of amino acid sequences of catalases B1_1 and A7_1. Putative signal sequences are marked by bold letters and putative propeptide sequences are underlined.
- FIG. 7:: Distance matrix with catalases B1_1, A7_1 and the closest NCBI database sequence identity (next neighbour) which is Catalase1 from *Penicillium marneffei* (XP_002153601)

The Examples illustrate the invention.

### Example 1

### Enzyme Activity Assays

Activity assay based on the substrate ABTS (2,2'-azino-bis(3-ethylbenzothiazoline-6-sulfonicacid)diammonium salt in multi-well plates (ABTS-MTP-Assay)

The activity of the catalase was measured by detection of residual amount of hydrogenperoxide (H₂O₂) after incubation of a defined amount of H₂O₂ for 15 min with catalase. For reaction 1 30% hydrogenperoxide (H₂O₂, VWR, 107209.0250)) was diluted in either citrate phosphate buffer, pH4.0 (122.9 mM C₆H₈O₇ x H₂O, 154.2 mM Na₂HPO₄) or pH7.0 (35.3 mM C₆H₈O₇ x H₂O, 329.4 mM Na₂HPO₄) or borate buffer pH9.3 (25°C) (25.0 mM Na₂B₄O₇ x 10 H₂O, 3.6 mM NaOH). 25 µl of prediluted H₂O₂ solution were filled in each well of 96-well PCR-MTP (LS-Labor-Service GmbH, 294981350). Then 45 µl of desired buffer solution was further added. This assay plate was put inside Thermoshaker (BIOER/Mixing Block MB-102) and has been preheated for 5 min. 5 µl of sterile filtered supernatant or catalase containing solution was added and has been shaken for 2 min at 600 rpm. Incubation was continued further 13 min without shaking. Reaction 1 was stopped by acidifying adding 75 µl 1 M H₂SO₄ (98%, Applichem/A4309,1000). The determination of residual H₂O₂ was done in a second reaction step by measuring the horseradish peroxidise catalyzed oxidation of 2,2'-azino-bis(3-ethylbenzothiazoline-6-sulfonicacid)diammonium salt (ABTS, Sigma-Aldrich/A1888). The oxidation reaction yields a coloured compound. The amount of dye that is formed is correlated with the concentration of H₂O₂. The formation of the dye can be monitored spectrophotometrically by measuring the absorption at 405 nm. To detect residual H₂O₂ 95 µl of ABTS/Peroxidase reagent (ABTS/Peroxidase reagent: 8.8 mg ABTS were dissolved in 10 ml citrate phosphate buffer, pH7.6 (12.7 mM C₆H₈O₇ x H₂O, 374.6 mM Na₂HPO₄) and 2160 U of horseradish peroxidase, Type VI-A, Sigma-Aldrich/P6782 dissolved in 5mM sodium phosphate buffer, pH7.0 were added) were filled in each well of 96-well flat bottom MTP (Greiner Bio-one, REF 655101). Reaction 2 was started by addition of 5 µl of each reaction 1. The formation of the dye was immediately measured at 30°C following a 5 sec shaking step (Spectra Max 190 microtiterplate reader, Molecular devices).

### ABTS-agar-MTP-plate assay

10 g agar were added to 500 ml citrate phosphate buffer, pH7.0 (35.3 mM C₆H₈O₇ x H₂O; 329.4 mM Na₂HPO₄) and autoclaved. Shortly before use 0.018% H₂O₂ was added. Then immediately 200 µl were filled in each well of 96-well MTP. After solidifying 5 µl of each sample was added per well and incubated over night at 50°C. To detect residual H₂O₂ 20 µl of ABTS/Peroxidase reagent were added per well. (ABTS/Peroxidase reagent: 8.8 mg ABTS were dissolved in 10 ml citrate phosphate buffer, pH7.6 (12.7 mM C₆H₈O₇ x H₂O, 374.6 mM Na₂HPO₄) and 2160 U of horseradish peroxidase, Type VI-A, Sigma-Aldrich/P6782 dissolved in 5mM sodium phosphate buffer, pH7.0 were added). No colouring gives a hint to catalase activity.

### In-gel enzyme assay

Catalase activity was assayed in polyacrylamide gels following running native gel electrophoresis. (Clare et al., 1984). Following electrophoresis the gel is soaked in 50 mM sodium phosphate buffer, pH 7.0 containing 50 µg / ml horseradish peroxidise (Sigma-Aldrich/P6782) at 20°C for 45 min in the dark. H₂O₂ was then added to a final concentration of 5.0 mM and incubation was continued for 10 min at 20°C. The gel was then rapidly rinsed twice with water and then placed into 0.5 mg/ml of diaminobenzidine (Sigma, D5637) in the phosphate buffer, until staining was completed.

### Example 2

### Screening for producers secreting catalase

In the screening of bacterial and fungal strains catalase producers were determined which secrete catalase in the medium. The aim of this study was to identify such strains secreting catalase highly efficient within a broad profile of both pH and temperature.

More than 400 bacterial and fungal strains were selected for investigation and streaked onto agar plates and incubated until growth on the plates occurred. Some cells were transferred using an inoculating loop for inoculation of 50 ml medium filled in 300 ml Erlenmeyer flasks. Cultures were shaken and grown. Supernatants were harvested by centrifugation and sterile filtered. Until use they were stored at 6 °C. Supernatants were diluted 1:10 and tested for catalase activity applying the ABTS-MTP-assay described in example 1.

In order to identify catalases with activity under extreme pH conditions and at high temperature screening conditions were chosen accordingly. Thus, screening for catalase activity of diluted supernatants was conducted at pH4 or pH9 and at 65°C, and a final concentration of 5.4 mM H₂O₂, respectively. Those supernatants showing reduction of H₂O₂ concentration of more than 29 percent were further investigated measuring activity at pH7. Fungal or bacterial strains showing highest catalase activity that means reduction of more than 29 percent applying conditions as described above were further evaluated by reculturing and retesting. Two fungal isolates were selected whose supernatants showed higher catalase activity in comparison to all other tested supernatants. These were the strains PEN_1 and PEN_2.

### Example 3

### Identification of active catalase and revealing of corresponding cDNA-gene

To isolate wanted active catalase and corresponding cDNA-gene two strategies were followed. On the one hand active protein was identified by protein identification via MALDI-MS/MS analysis and on the other hand a sequence based approach was chosen.

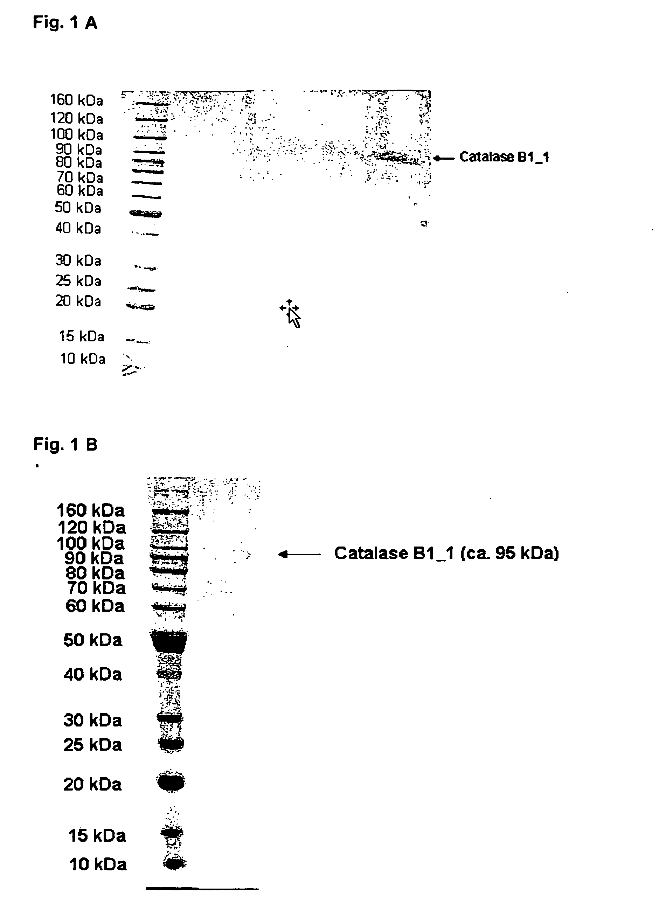

To identify active catalase secreted in medium fungal strain PEN_2 was grown in KP-medium (glucose, 8.0%; polypeptone, 0.25%; KNO₃, 0.05%; yeast extract, 0.5%; KH₂PO₄, 0.1%; CaCO₃, 2.5%). Cells were harvested by centrifugation and supernatant was sterile filtered and stored at 6°C. Protein was concentrated 100times using Vivaspin columns (10 kDa). Native gel electrophoresis was performed using a 7% Tris-Acetate gel and Tris-glycin native buffer (Invitrogen). 7.5 µl of concentrated sample was loaded in each lane. After running gel electrophoresis lanes were separated by cutting. One lane was stained following in gel enzyme assay procedure described above. Further lanes were separated for 2D-SDS_PAGE using invitrogen Two-Dimensional Native/SDS-PAGE system. Colloidal coomassie staining revealed a single band corresponding to white zone of stained native gel stripe representing active catalase (Fig. 1). Fragments of protein corresponding to single band were identified via MALDI-MS/MS analysis. Furthermore for partly purify catalase protein fungal strain PEN_2 was grown in KP-medium again as described above. Protein in supernatant was concentrated about 20times and buffer was exchanged at the same time to sodium phosphate buffer, pH7.0. Then gel filtration chromatography was performed on a Superdex 200 HR 30/10 column (Pharmacia) with sodium phosphate buffer, pH7.0, using a flow rate of 0.5 ml/min. 32 fractions were sampled and analyzed for catalase activity applying ABTS-agar-MTP-plate assay. Fractions showing catalase activity were further investigated using In gel enzyme assay (see above) and Enzyme activity assay (see above). Aliquots of fractions showing catalase activity were applied to SDS-PAGE analysis. Fragments of protein corresponding to a single band around 95 kDa were identified by MALDI-MS/MS-analysis.

Furthermore a sequence based approach was chosen to reveal partial catalase sequences which could be translated in amino acid sequences. Comparison of these amino acid sequences with fragments identified by MALDI-MS/MS-analysis (see above) revealed identical sequence regions. These sequence regions representing partial sequence area of catalase gene corresponding to active catalase of interest could be used to isolate full length catalase gene. Primers for genetic screening for catalase genes were constructed using known catalase sequences of *Aspergillus and Penicillium* strains. To generate templates for PCR reaction cDNA had to be generated using total RNA originating from for example strain PEN_2. To isolate total RNA encoding favoured catalase for example strain PEN_2 was grown in PK-medium, pH7 (glucose, 6.0%; KNO₃ 0.5%; MgSO₄ x 7 H₂O, 0.05%; NaH₂PO₄, 0.3%; K₂HPO₄ x 3 H₂O 0.25%; 2 ml of micronutrients solution; micronutrients solution (Na₂B₄O₇ x 10 H₂O, 0.08%; Na₂MoO₄ x 2 H₂O, 0.08%; FeSO₄ x 7 H₂O, 0.04%; CuSO₄ x 5 H₂O, 0.04%; MnSO₄ x 5 H₂O, 0.08%; ZnSO₄ x 7 H₂O, 0,8%) and shaken in Erlenmeyer flasks. Catalase activity was monitored using ABTS-agar-MTP-plate-assay. Culture showing high catalase activity was harvested by centrifugation and cell pellet was frozen by liquid nitrogen and stored at -80°C. For isolation of total-RNA two cell pellets of 0.5 g were further frozen with liquid nitrogen and mechanically pulverised. RNA was isolated according RNeasy Plant MiniKit from Qiagen (Cat. No. 74903). Either RLT- or RLC-buffer were added. Samples were divided into 5 aliquots. Isolated RNA was dissolved each in 30 µl DEPC-treated water. The generation of cDNA from RNA was accomplished according to the "SMART cDNA Library construction Kit User Manual" (CLONTECH Laboratories, Inc.). Generated cDNA served as template for PCR screening revealing novel partial catalase-sequence areas. Comparison of corresponding partial amino acid sequences with identified MALDI-MS/MS-fragments revealed areas of identity in one case. To identify full length catalase gene sequence representing catalase of interest cDNA fragments were phosphorylated and ligated. Then primers directed against boundaries of known partial catalase-sequence of interest were designed for in side out PCR. Full-length catalase genes were further amplified and subcloned for sequencing.

The catalase isolated from the strain PEN_1 was termed A7_1 and the catalase isolated from PEN_2 was termed B1_1.

An alignment between these two amino acid sequences showing the differences is shown in Fig. 6. The putative signal sequence and the putative propeptide were deduced from information from the literature concerning catalase genes from *Aspergillus fumigatus* and related catalases (Calera, J.A., Paris, S., Monod, M., Hamilton, A.J., Debeaupuis, J.-P-, Diaquin, M., Lopez-Medrano, R., Leal, F. and Latge, J.-P. (1997) Cloning and Disruption of the Antigenic Catalase Gene of Aspergillus fumigatus, Infection and Immunity, 4718-4724). After heterologous expression the mature protein is found in the supernatant. Therefore, it can be concluded that correct processing is accomplished by the host during secretion. BLAST-analysis of the two amino acid sequences were performed in order to identify the next neighbour in the NCBI database, which resulted in *Penicillium marneffei* Catalase Cat1 (NCBI XP_002153601.3). Clustal analysis of this next neighbour in direct alignment with A7_1 and B1_1 and subsequent distance matrix analysis with the full length sequence (including pre- and propeptide) revealed a 90.87% identity to a mycelial catalase (Cat1) of *Penicillium marneffei* for A7_1 and a 91.42% identitiy to the same mycelial catalase (Cat1) of *Penicillium marneffei* for B1_1. The identity between B1_1 and A7_1 amino acid sequences is 97.68%. A distance matrix showing the differences between the two catalases of the invention and their next neighbour is shown in fig. 7A.

When comparing the putative mature amino acid sequence (without prepro-sequence) the values were 91.62% identity to the next neighbour (Cat1 of *P. marneffei*) for B1_1 and 91.18% identity for A7_1 and 98.12% identity to each other (s. Fig. 7B).

The same procedure was applied for the nucleotide sequences resulting in an identity of 85.76 for B1_1 and 85.26% for A7_1 in comparison to *Penicillium marneffei* (NCBI XN_002153565.11) for the full length sequence and 85.67% and 85.23%, respectively, for the sequence coding the putative mature protein (s. Fig. 7C).

### Example 4

### Expression in Escherichia coli

To evaluate feasibility of heterologous expression of catalase gene e.g. B1_1 originating from strain PEN_2 in *Escherichia coli* expression construct set up in a typical expression vector such as pBAD- or pBAD/gIII-System (Invitrogen) and a suitable expression host such as *E. coli* DH10BΔ*katE* (Invitrogen modified) were used. For construction of expression constructs, the gene of interest were PCR amplified to introduce unique restriction enzyme recognition sequences upstream and downstream of the open reading frame (ORF) which allowed to ligate the catalase gene with the expression vector. Gene amplification was done in such a way that for example gene was amplified including or excluding putative propeptide region or substituting putative native signal sequence by the vector encoded leader peptide from the bacteriophage fd gene III protein (gIII) (pBAD/gIII-System, Invitrogen). Restriction enzyme recognition sequences were chosen on the basis of their absence in the coding region of the gene of interest and could be e.g. Ncol, Sall or Kpnl.

For heterologous expression experiments *E*. *coli* host systems transformed with relevant plasmids were grown over night in 5 ml Luria broth culture medium complemented with the appropriate antibiotic. 200 µl of such culture was used to inoculate 20 ml of Luria broth medium containing final 100 µg/ml ampicillin. Cultures were grown at a temperature of 37°C on a gyratory shaker at 180 rpm. Samples were taken when the optical density reached about 0.5. At this point in time for induction of expression sterile filtered L-arabinose was added to final concentrations of 0.2% and 0.002%. Cells were further harvested 4 hours after induction by centrifugation. Pellets were frozen at -80°C for further treatment. Cells were disrupted by ultrasonication. Applying SDS-PAGE analysis showed no heterologous expression of samples with expression constructs including putative native signal sequence. Expression of inclusion bodies was shown investigating expression of constructs with leader peptide gIII. However, no catalase activity could be detected at all using ABTS-agar-MTP-plate assay after incubation of samples at temperature of 80°C for 15 minutes.

### Example 5

### Expression in Pichia pastoris

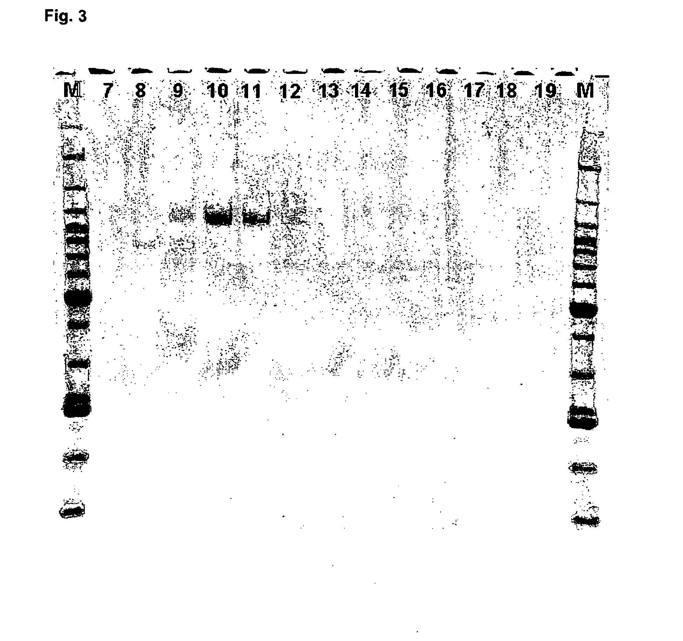

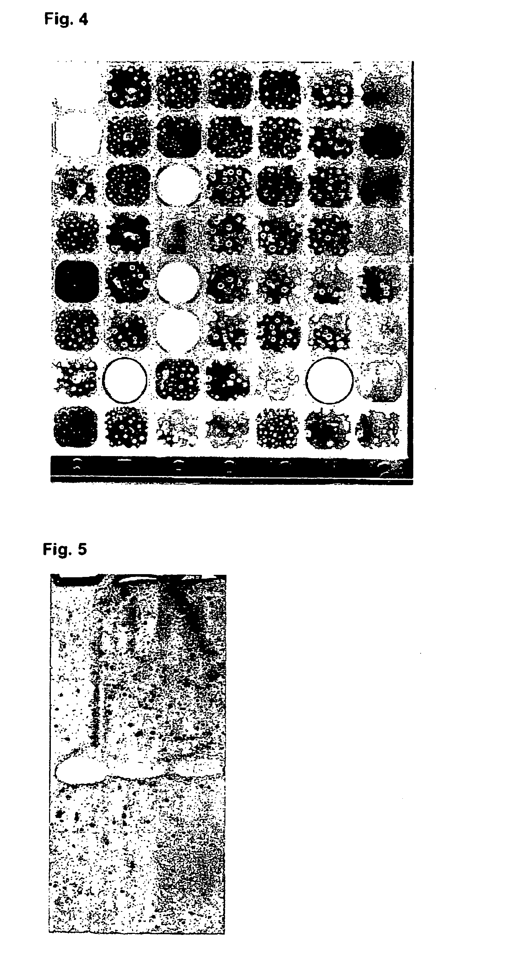

To evaluate feasibility of heterologous expression of catalase genes of interest in the yeast *Pichia pastoris* an integrative vector system was used for expression, providing either a methanol inducible promoter such as pAOX-promoter or a constitutively expressed promoter such as pGAP-, pPGK1 or YPT1-promoter. Native signal sequence of catalase genes of interest were in some cases substituted by e.g. vector encoded α-factor secretion signal sequence from *Saccharomyces cerevisiae* or Phol signal sequence. For construction of expression constructs, the gene of interest were e.g. PCR amplified to introduce unique restriction enzyme recognition sequences upstream and downstream of the open reading frame (ORF) which allowed to ligate the catalase gene with the expression vector. Furthermore expression constructs were assembled for example by overlap extension PCR or using InFusion technology (Clontech Laboratories, Inc.). To evaluate several approaches which could improve yield or secretion of catalase of interest e.g. hac1, sec4 or seb1 were coexpressed or some genes such as pepA or gas1 were deactivated by e.g. knock in strategies. Furthermore to improve yield of expressed catalase several approaches were followed such as increase of copy number, or UV-mutagenesis. In the latter case *Pichia* clones were exposed toward UV radiation. Clones that survived exposure were grown in medium supplemented with H₂O₂ concentration normally killing *Pichia* clones. *Pichia* clones that were able to grow in such medium were further analyzed (e.g. Fig. 4). In order to investigate which *Pichia* clone variant showed highest yield of expressed catalase all variants were grown in 30 ml medium in 300 ml Erlenmeyer flasks at temperature of 28°C and 180 rpm. The initial optical density was adjusted to 0.1. Cultures have been cultured for two days at least. Then catalase activity in sterile filtered supernatant was daily monitored using ABTS-agar-MTP-plate assay either with or no heat pretreatment of sample for 15 min at 80°C. Optical density of active cultures was determined and supernatant sterile filtered. Samples were stored at 4°C until measuring catalase activity using ABTS-MTP-plate-assay as described in example 1 (pH7, 80°C, 5.4 mM H₂O₂). *Pichia* clones which showed highest catalase activity in supernatant were applied to fermentation. To further analyze heterologously expressed *Penicillium* catalase sterile filtered supernatant was taken for partly purify catalase of interest. Following ion-exchange chromatography experiments gel-filtration chromatography was performed using Superdex 200 HR 30/10 column (Pharmacia) (e.g. Fig.3)

### Example 6

### Expression in Aspergillus niger

To further increase yield of heterologous catalase expression, *Aspergillus niger* was evaluated as host system. As a first step a selection was made from a selected number of potential laboratory strains based on catalase background activities and *in vitro* degradation of a Pichia-produced catalase sample. Based on these selection criteria *Aspergillus niger* strain AB1.18 (*pyrG, pep*A18) was chosen. The basic vector used was based on PAN52-4 (Z32699) providing a constitutive promoter gpd and including an AmdS selection marker. In some cases vector pAN7-1 (Z32698.1) carrying a gene expressing a hygromycin resistance marker was cotransformed. As possibly relevant for functional secretion in fungi, the second amino acid of the assumed signal sequence should be arginine (R) and not glycine (G) as in protein B1_1. Therefore the coding region was reconstructed using a small synthetic *A. niger-*codon-optimized 5' gene fragment. To evaluate approaches to further increase the expression yield the copy number of the gene of interest was increased by applying a second transformation cycle using the original construct again. Furthermore, *Aspergillus* clones were exposed to UV radiation. Clones that survived the procedure were further investigated. To identify the best catalase producer strains clones were analyzed in a microplate assay (see fig. 4). Each well of 48-well microtiter plates (VWR International, 736-0349) was filled with 1200 µl PD-medium (BD Becton Dickinson, 254920). Each clone was grown in quadruplicate by inoculating medium with spores. Strains were mostly grown five days at 28°C at 750 rpm using Titramax 1000 (Heidolph). Then aliquot of supernatant was sterile filtered (0.45 µm, PVDF membrane) and catalase activity was measured using the ABTS-MTP-plate assay as described above (pH7, RT, 5.4 mM H₂O₂). *Aspergillus* clones producing the highest catalase levels were cultivated under controlled fermentation conditions to generate catalase samples and to compare yield.

### Example 7

### Proof of activity

### Acitivtv of catalases produced by original Penicillium strains

PEN_1 and PEN_2 were either grown in KP- (see above)- or in PK-medium (see above), cells were harvested by centrifugation and supernatants were sterile filtered. Total protein concentration was determined according to Bradford (Bradford, M. M. A rapid sensitive method for the quantitation of microgram quantities of protein utilizing the principle of protein-dye binding. Anal. Biochemistry (1976) 72, 248 - 254). Samples were stored at 4°C until measuring catalase activity using ABTS-MTP-plate-assay as described in example 1 determining residual H₂O₂ concentration in percent of initial H₂O₂ concentration (see tables below). Surprisingly, at 80°C, which exceeds the temperature applied during the screening considerably, conversion of peroxide of up to 100 % were observed at pH4. Even at the extreme conditions of a temperature of 90°C and pH4 substantial peroxide conversion percentages of 55-78 were measured. Thus, both strains PEN_1 and PEN_2 express catalases with acitivity in a high temperature- and low pH-range, which have not been described in this combination before.

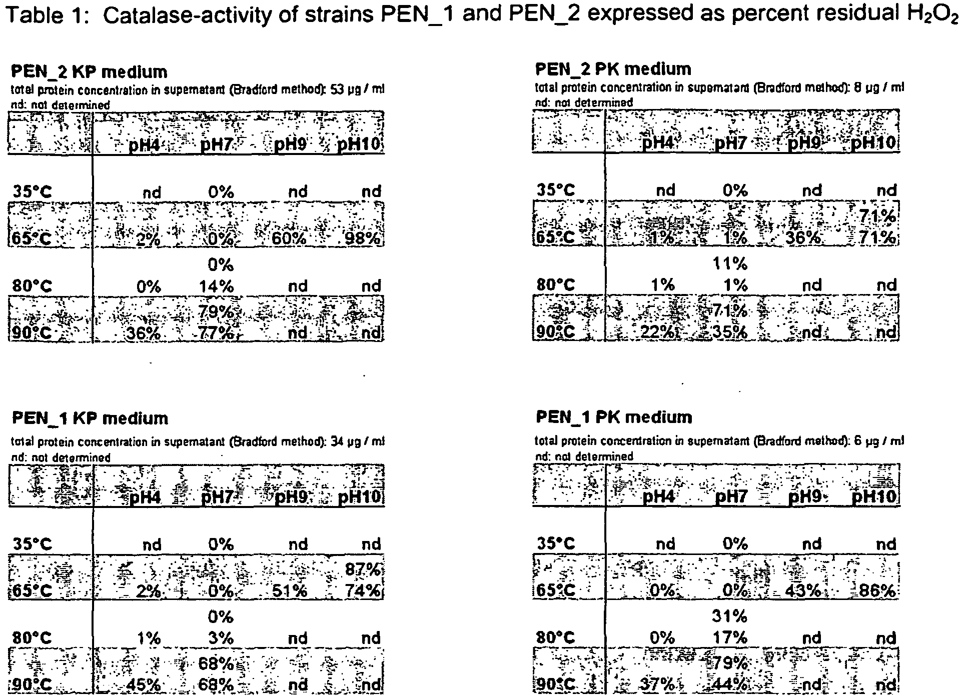

### Activity of catalase clones B1_1 and A7_1 after heterologous expression in Pichia pastoris and Aspergillus niger

To verify catalase activity of heterologously expressed catalase in *Pichia pastoris* sterile filtered supernatant of *Pichia* clones grown and harvested as described above were analyzed for catalase activity applying ABTS-MTP-plate-assay as described in example 1 determining residual H₂O₂ concentration in percent of initial H₂O₂ concentration (see table below). As described above native signal sequence of catalase genes of interest were in some cases substituted by e.g. vector encoded α-factor secretion signal sequence from *Saccharomyces cerevisiae* or Phol signal sequence (PhoA7_1; PhoB1_1; αMFA7_1; αMFB1_1)

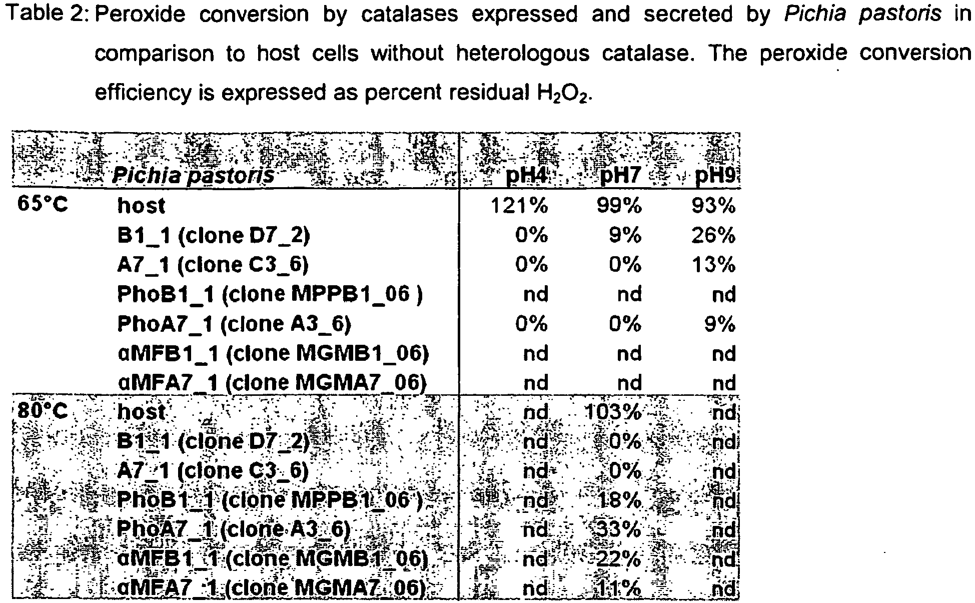

Thus, it was shown that after heterologous expression in *Pichia pastoris* both catalases of the invention show efficient conversion of hydrogen peroxide even at 80°C.

To show catalase activity of heterologously expressed derivates of catalase B1_1 (Cat52_5 (SEQ ID NO: 22; Cat52_5_B (SEQ ID NO: 23), which were optimized with respect to codon usage of the host, in *Aspergillus niger* sterile filtered supernatant of *Aspergillus* clones grown and harvested as described above were analyzed for catalase activity using e.g. In gel enzyme assay (e. g. Fig. 5) and ABTS MTP-plate-assay. Catalase activity in sterile filtered supernatant of *Aspergillus niger* culture heterologously expressing *Penicillium* catalase was detected in both assays. At 80°C a conversion of hydrogen peroxide of over 90% (less than 10% residual H₂O to a mycelial catalase of Penicillium marneffei for A7_1) at pH 7.0 in the ABTS-MTP plate assay was detected with fermentation supernatant in a dilution of 1:150 or even higher dilutions showing the production of active catalase in the heterologous host *Aspergillus.*

## Claims

1. A nucleic acid molecule encoding a polypeptide having the activity of a catalase (EC 1.11.1.6), which nucleic acid molecule is
(a) a nucleic acid molecule encoding a polypeptide comprising or consisting of the amino acid sequence of SEQ ID NO: 1;
(b) a nucleic acid molecule comprising or consisting of the nucleotide sequence of SEQ ID NO: 2;
(c) a nucleic acid molecule comprising or consisting of a nucleic acid molecule encoding a polypeptide having the activity of a catalase the amino acid sequence of which is at least 99% identical to the amino acid sequence of SEQ ID NO: 1,
(d) a nucleic acid molecule encoding a polypeptide having the activity of a catalase and comprising or consisting of a nucleotide sequence which is at least 98% identical to the nucleotide sequence of SEQ ID NO: 2,
(e) a fragment of the polypeptide of any of (a) to (d) having the activity of a catalase and comprising at least 675 amino acids, or
(f) the nucleic acid sequence of any of (a) to (d) wherein T is U.

2. A polypeptide encoded by the nucleic acid molecule of claim 1.

3. A vector comprising the nucleic acid molecule of claim 1.

4. A host cell carrying the vector of claim 3.

5. A method of producing a protein having the activity of a catalase (EC 1.11.1.6) comprising
(a) culturing the host cell of claim 4, and
(b) isolating the produced protein having the activity of a catalase.

6. The polypeptide of claim 2, which is a fusion protein.

7. A composition comprising the nucleic acid molecule of claim 1, the polypeptide of claim 2, the vector of claim 3, the host cell of claim 4, and/or the fusion protein of claim 6, or combinations thereof.

8. The composition of claim 7, which is an large-scale composition, a pharmaceutical composition, a diagnostic composition or a cosmetic composition.

9. The large-scale composition of claim 8, wherein the composition is for processing textile, beverage, food, pulp or paper.

10. The pharmaceutical composition of claim 8 for use in treating a disease **characterized by** impaired catalase, such as vitiligo, acatalasia, polymorphic light eruption (PLE), or a disease **characterized by** the occurrence of oxidative stress such as Acne vulgaris, Alopecia areata, Lichen planus, Psoriasis and Rheumatoid arthritis.

11. The cosmetic composition of claim 8, wherein the composition is for anti-aging, improving skin appearance, preventing hair greying, cleaning contact lenses and/or correcting pigmentary abnormalities.

12. A method of converting hydrogen peroxide into water and oxygen comprising contacting the polypeptide of claim 2, the host cell of claim 4, and/or the fusion protein of claim 6, or combinations thereof with a sample comprising hydrogen peroxide.

13. Use of the nucleic acid molecule of claim 1, the polypeptide of claim 2, the vector of claim 3, the host cell of claim 4, and/or the fusion protein of claim 6, or combinations thereof for the conversion of hydrogen peroxide into water and oxygen.

14. The method according to claim 12 or the use according to claim 13, wherein the conversion serves for controlling or stopping bleaching of an industrial product.

15. The method or use according to claim 14, wherein the industrial product is textile, pulp or paper.

## Patentansprüche

1. Nukleinsäuremolekül, das ein Polypeptid kodiert, das die Aktivität einer Katalase (EC 1.11.1.6) hat, wobei das Nukleinsäuremolekül
(a) ein Nukleinsäuremolekül ist, das ein Polypeptid kodiert, das die Aminosäuresequenz der SEQ ID NO: 1 umfasst oder aus dieser besteht;
(b) ein Nukleinsäuremolekül ist, das die Nukleotidsequenz der SEQ ID NO: 2 umfasst oder aus dieser besteht;
(c) ein Nukleinsäuremolekül ist, das ein Nukleinsäuremolekül umfasst oder aus diesem besteht, das ein Polypeptid kodiert, das die Aktivität einer Katalase hat und dessen Aminosäuresequenz mindestens 99% identisch zu der Aminosäuresequenz der SEQ ID NO: 1 ist;
(d) ein Nukleinsäuremolekül ist, das ein Polypeptid kodiert ist, das die Aktivität einer Katalase hat und das eine Nukleotidsequenz umfasst oder aus dieser besteht, die mindestens 98% identisch zu der Nukleotidsequenz der SEQ ID NO: 2 ist;
(e) ein Fragment des Polypeptids nach einem Punkt (a) bis (d) ist, das die Aktivität einer Katalase hat und mindestens 675 Aminosäuren umfasst, oder
(f) die Nukleinsäuresequenz nach einem Punkt (a) bis (d) ist, wobei T U ist.

2. Polypeptid, kodiert durch das Nukleinsäuremolekül nach Anspruch 1.

3. Vektor, umfassend das Nukleinsäuremolekül nach Anspruch 1.

4. Wirtszelle, die den Vektor nach Anspruch 3 trägt.

5. Verfahren zur Herstellung eines Proteins, das die Aktivität einer Katalase (EC 1.11.1.6) hat, umfassend
(a) Züchtung der Wirtszelle nach Anspruch 4, und
(b) Isolierung des hergestellten Proteins, das die Aktivität einer Katalase hat.

6. Polypeptid nach Anspruch 2, das ein Fusionsprotein ist.

7. Zusammensetzung, umfassend das Nukleinsäuremolekül nach Anspruch 1, das Polypeptid nach Anspruch 2, den Vektor nach Anspruch 3, die Wirtszelle nach Anspruch 4 und/oder das Fusionsprotein nach Anspruch 6 oder Kombinationen davon.

8. Zusammensetzung nach Anspruch 7, die eine Zusammensetzung in großem Maßstab, eine pharmazeutische Zusammensetzung, eine diagnostische Zusammensetzung oder eine kosmetische Zusammensetzung ist.

9. Zusammensetzung in großem Maßstab nach Anspruch 8, wobei die Zusammensetzung zur Bearbeitung von Textil, Getränken, Nahrungsmitteln, Zellstoff oder Papier ist.

10. Pharmazeutische Zusammensetzung nach Anspruch 8 zur Verwendung in der Behandlung einer Krankheit, die durch gestörte Katalaseaktivität gekennzeichnet ist, wie Vitiligo, Akatalasie, polymorphe Lichteruption (PLE), oder einer Krankheit, die durch das Auftreten von oxidativem Stress gekennzeichnet ist, wie Akne vulgaris, Alopecia areata, Knötchenflechte, Psoriasis und rheumatoide Arthritis.

11. Kosmetische Zusammensetzung nach Anspruch 8, wobei die Zusammensetzung gegen die Alterung, zur Verbesserung des Hauterscheinungsbilds, zur Prävention des Ergrauens der Haare, zur Reinigung von Kontaktlinsen und/oder zur Korrektur von Pigmentabnormalitäten ist.

12. Verfahren zur Umwandlung von Wasserstoffperoxid in Wasser und Sauerstoff, umfassend das Inkontaktbringen des Polypeptids nach Anspruch 2, der Wirtszelle nach Anspruch 4, und/oder des Fusionsproteins nach Anspruch 6 oder von Kombinationen davon mit einer Probe, die Wasserstoffperoxid umfasst.

13. Verwendung des Nukleinsäuremoleküls nach Anspruch 1, des Polypeptids nach Anspruch 2, des Vektors nach Anspruch 3, der Wirtszelle nach Anspruch 4, und/oder des Fusionsproteins nach Anspruch 6 oder von Kombinationen davon zur Umwandlung von Wasserstoffperoxid in Wasser und Sauerstoff.

14. Verfahren nach Anspruch 12 oder Verwendung nach Anspruch 13, wobei die Umwandlung zur Kontrolle oder zum Stoppen des Bleichens eines industriellen Produkts dient.

15. Verfahren oder Verwendung nach Anspruch 14, wobei das industrielle Produkt Textil, Zellstoff oder Papier ist.

## Revendications

1. Molécule d'acide nucléique codant pour un polypeptide possédant l'activité d'une catalase (EC 1.11.1.6), laquelle molécule d'acide nucléique est
(a) une molécule d'acide nucléique codant pour un polypeptide comprenant ou constitué de la séquence d'acides aminés de SEQ ID NO : 1 ;
(b) une molécule d'acide nucléique comprenant ou constituée de la séquence nucléotidique de SEQ ID NO : 2 ;
(c) une molécule d'acide nucléique comprenant ou constituée d'une molécule d'acide nucléique codant pour un polypeptide possédant l'activité d'une catalase dont la séquence d'acides aminés est au moins identique à 99 % avec la séquence d'acides aminés de SEQ ID NO : 1,
(d) une molécule d'acide nucléique codant pour un polypeptide possédant l'activité d'une catalase et comprenant ou étant constituée d'une séquence nucléotidique qui est au moins identique à 98 % avec la séquence nucléotidique de SEQ ID NO : 2,
(e) un fragment du polypeptide de l'un quelconque de (a) à (d) possédant l'activité d'une catalase et comprenant au moins 675 acides aminés, ou
(f) la séquence d'acide nucléique de l'un quelconque de (a) à (d) où T est U.

2. Polypeptide codé par la molécule d'acide nucléique selon la revendication 1.

3. Vecteur comprenant la molécule d'acide nucléique selon la revendication 1.

4. Cellule hôte portant le vecteur selon la revendication 3.

5. Méthode de production d'une protéine possédant l'activité d'une catalase (EC 1.11.1.6) comprenant
(a) la culture de la cellule hôte selon la revendication 4, et
(b) l'isolement de la protéine produite possédant l'activité d'une catalase.

6. Polypeptide selon la revendication 2, qui est une protéine de fusion.

7. Composition comprenant la molécule d'acide nucléique selon la revendication 1, le polypeptide selon la revendication 2, le vecteur selon la revendication 3, la cellule hôte selon la revendication 4, et/ou la protéine de fusion selon la revendication 6, ou leurs combinaisons.

8. Composition selon la revendication 7, qui est une composition à grande échelle, une composition pharmaceutique, une composition de diagnostic ou une composition cosmétique.

9. Composition à grande échelle selon la revendication 8, où la composition est destinée au traitement de textiles, de boissons, d'aliments, de pulpe ou de papier.

10. Composition pharmaceutique selon la revendication 8, pour une utilisation dans le traitement d'une maladie **caractérisée par** une catalase altérée, comme le vitiligo, l'acatalasie, l'éruption polymorphe due à la lumière (PLE), ou une maladie **caractérisée par** la survenue d'un stress oxydatif comme l'acné vulgaire, la pelade, le lichen plan, le psoriasis et la polyarthrite rhumatoïde.

11. Composition cosmétique selon la revendication 8, où la composition est destinée au vieillissement, à l'amélioration de l'aspect de la peau, à la prévention des cheveux gris, au nettoyage des lentilles de contact et/ou à la correction des anomalies pigmentaires.

12. Méthode de conversion du peroxyde d'hydrogène en eau et en oxygène comprenant la mise en contact du polypeptide selon la revendication 2, de la cellule hôte selon la revendication 4, et/ou de la protéine de fusion selon la revendication 6, ou de leurs combinaisons avec un échantillon comprenant du peroxyde d'hydrogène.

13. Utilisation de la molécule d'acide nucléique selon la revendication 1, du polypeptide selon la revendication 2, du vecteur selon la revendication 3, de la cellule hôte selon la revendication 4, et/ou de la protéine de fusion selon la revendication 6, ou de leurs combinaisons pour la conversion du peroxyde d'hydrogène en eau et en oxygène.

14. Méthode selon la revendication 12 ou utilisation selon la revendication 13, où la conversion sert à contrôler ou à stopper le blanchiment d'un produit industriel.

15. Méthode ou utilisation selon la revendication 14, où le produit industriel est un textile, de la pulpe ou du papier.


### SEQUENCE LISTING

<110> B.R.A.I.N. Biotechnology Research and Information Network AG
<120> Novel Catalases
<130> S2967 PCT
<150> EP 10 01 5193.5
   <151> 2010-12-01
<160> 23
<170> PatentIn version 3.3
<210> 1
   <211> 692
   <212> PRT
   <213> Penicillium sp.
<400> 1
690
<210> 2
   <211> 2076
   <212> DNA
   <213> Penicillium sp.
<400> 2
   gacgattcaa atgcttcctc cgaaacagaa gcctttctgt ccgagttcta cctcaacgac 60
   aacgatgcct atctcaccac cgatgtaggc ggtccgatcg aggatcaaca cagtttgaag 120
   gccggcattc gtggatcgac cctcttggaa gatttcatct tccgtcagaa aatccagcat 180
   ttcgatcatg agcgtgtccc ggaacgtgcc gtgcatgctc gaggtgcagg tgctcatggt 240
   gtattcactt catatgccga ctggtccaac atcactgctg cttcattctt gggagctgct 300
   ggaaaggaaa cgcccacatt tgtccgcttc tcgactgttg caggcagccg aggaagtgcc 360
   gacaccgctc gtgacgttca cggatttgct actcgcttct atactgacga gggaaactat 420
   gacatcgttg gaaacaacat ccctgtcttc ttcatccaag atgctatcct attcccagat 480
   ctcatccata gcgtcaagcc acagccagcc aatgaaatcc cacaggctgc tactgcacac 540
   gacacggcct atgacttctt tggtcaacag ccaagcactc tgcataccct cttctgggca 600
   atggcaggtc atggtatccc acggtctttc cgtcatgttg acggattcgg cgtccacacc 660
   tatcggttcg tgactgaaga tggctcgtcc aaattggtca aatttcactg gacatcacta 720
   cagggccgag ccagtctggt ctgggaggaa gctcaggcca ctgctggcaa aaatgcggac 780
   tttatgagac aggatctgta tgatagcatt gaggccggcc gttatccaga gtgggagttc 840
   ggcgtgcaaa tcattgatga gtcggatgtc ttaagctacg gatttgactt gttggatcca 900
   accaagattc ttccggtgga aaaagttcca attactccac tcggaaaaat gcaactcaac 960
   cgtaatccat tgaattactt tgccgagaca gagcaagtca tgttccaacc tggccacatt 1020
   gttcgtggta tcgactttac cgaggatcct cttctccaag gtcgtttatt ctcctacctc 1080
   gacactcagc tgaatcgcaa cggtggtccc aactttgaac aaatcccaat caatcgtcct 1140
   cgtgtaccta tccacaacaa taaccgcgat ggattcgccc aaatgtttat tcctttgaac 1200
   caagcagcat attcacccaa caccttgaat aatggctctc ctcgacaagc caacgagact 1260
   gtaggaaatg gcttctttac cacccccagg cgctccgcag atggacacct tgttcgcgct 1320
   actagcccaa catttgccga cgtgtggtct cagcctggct tgttttacaa ctccttgacg 1380
   gctaccgaac aacaatttgt gatcaatgct ttgcgtttcg agttgtccaa tgtaaagagc 1440
   gaggatgtta aaagcaattt catcacacag ataaatcgcg taaacaatac gctagcaaca 1500
   cttgtggctt ctgcaattgg ggtctccgcg cccgaacccg actctacata ctaccacagc 1560
   aataagacgt ctaatgtcgg aacattcggt actccgttga aaaagcttga cggtctcaag 1620
   gtcggagtcc ttgcttcggt gagcgatgaa agtagtattg ccgagggaca agcattggca 1680
   caaagcctag cggactcgaa cgtggacgtc gttatcgtcg ccgagcatct tacttcgaac   1740
   gtgtcagcta catactctgg atcagacgca acgaacttcg atgctgttat tgtcagctca   1800
   ggggctgaag gtctctttgg accccaaacc tttactgccg aatctaacac aacactttat   1860
   ccggcaggcc gtcctagtca gattttggtc gatgccttcc gctttggcaa gccggttgga   1920
   gcagtgggtg gcgccagtgc agctctttcg gcggtggata tcagtactga tcgcagtggt   1980
   gtggttactg gtgattccgt cagtgacgac tttgtcaacc agctaacgga ggaccttgcc   2040
   acattcaaat tcttggaccg attcgctgtg gatgag                             2076
<210> 3
   <211> 692
   <212> PRT
   <213> Penicillium sp.
<400> 3
690
<210> 4
   <211> 2076
   <212> DNA
   <213> Penicillium sp.
<400> 4
   gacgattcaa atgcttcctc cgaaacagaa gcctttctgt ccgagttcta cctcaacgac 60
   aacgatgcct atctcaccac cgatgtaggc ggtccgatcg aggatcaaaa cagtttgaag 120
   gccggcattc gtggatcgac cctcttggaa gacttcatct tccgtcagaa aatccagcat 180
   tttgatcatg agcgtgtccc ggaacgtgcc gtgcatgctc gaggtgcagg tgctcatggt 240
   gtatttactt catatgccga ctggtccaac atcactgctg cttcattttt gggagcttcc 300
   ggaaaggaaa cgcccacatt tgtccgcttc tcgactgttg caggcagccg aggaagtgcc 360
   gacaccgctc gtgacgttca cggatttgct actcgcttct atactgacga gggaaactat 420
   gacattgttg gaaacaacat tcctgtcttc ttcatccaag atgctatctt attcccagat 480
   ctcatccata gcgttaagcc acagccagcc aatgaaatcc cacaggctgc tactgcacac 540
   gacacggcct atgacttctt tggtcaacag ccaagcactc tgcataccct cttctgggca 600
   atggcaggcc atggtatccc acggtctttc cgtcatgttg acggattcgg tgtccacacc 660
   tatcggttcg tgacagatga tggctcgtcc aagttggtca aatttcactg gacatcgctg 720
   caaggtcggg ccagtctggt ctgggaggaa gctcaggcca ctgctggcaa aaatgccgac 780
   tttatgagac aggatctgta tgatagcatt gaggctggcc gttatccaga gtgggagctc 840
   ggcgtgcaaa taattgagga gtcggatgtc ttaagctacg gatttgacct gttggatcca 900
   accaagattc ttccggttga aaaagttcca attactgcgc tcggaaaaat gcaactcaac 960
   cgtaatccat tgaattactt tgccgagaca gagcaagtca tgttccaacc tggccacatt 1020
   gttcgtggta tcgacttcac cgaggatcct cttctccagg gtcgtttatt ctcctacctc 1080
   gatactcagc tgaatcgcaa tggtggtccc aactttgaac aaattccaat caatcgtccg 1140
   cgtgttccta tccacaacaa caaccgcgat ggattcgccc aaatgtttat tcctttgaac 1200
   caggcagcat attcacccaa caccttgaat aatggctctc ctcgacaagc caacgagact 1260
   gtcggaaatg gcttctttac cgcccccggg cgctccgcag atggacacct tgttcgcgct 1320
   acgagcccaa catttgccga cgtgtggtct cagcctggct tgttttacaa ctccttgacg 1380
   gctaccgaac aacagttcgt gatcaatgct ttgcgtttcg aattgtctaa tgtaaagagc 1440
   gaggatgtta aaagcaattt catcacacag ataaatcgcg taaacaacac gttagcaaca 1500
   cttgtggctt ctgcaattgg agtctccgcg cccgaacccg actctacata ctaccacagc 1560
   aataagacgt ctaatgtcgg aacattcggt actccgttga aaaagcttga cggtctcaag 1620
   gtcggagtcc ttgcttcggt gaacggtgaa agtagtattg ccgagggaca agcattggca 1680
   caaagcctag cgggctcgaa cgtggacgtc gttatcgtcg ccgagcatct tacttcgaac 1740
   gtgtcagcta catactctgg atcagacgca acgaactttg atgctgttat tgtcagctca 1800
   ggggctgaag gtctctttgg acctcaaacc tttacagccg aatccaatac aacactttat 1860
   ccggcaggcc gtcctagcca gattttggtc gatgccttcc gctttggcaa gccggttgga 1920
   gcagttggtg gtgccagtgc agctctgtca gcggtggata tcagtactga tcgtagtggt 1980
   gtgattactg gtgattccgt cagtgacgac tttgtcaagc agctaacgga ggaccttgcc 2040
   acattcaaat tcttggaccg attcgctgtg gatgag 2076
<210> 5
   <211> 715
   <212> PRT
   <213> Penicillium sp.
<400> 5
   Ala Cys Pro Met Leu Thr Gly Glu Ile Pro Ala Gly Ser Val Ala Asn
   1               5                   10                  15
   Pro His His His Gly Lys Arg Asp Asp Ser Asn Ala Ser Ser Glu Thr
               20                  25                  30
   Glu Ala Phe Leu Ser Glu Phe Tyr Leu Asn Asp Asn Asp Ala Tyr Leu
           35                  40                  45
   Thr Thr Asp Val Gly Gly Pro Ile Glu Asp Gln His Ser Leu Lys Ala
       50                  55                  60
   Gly Ile Arg Gly Ser Thr Leu Leu Glu Asp Phe Ile Phe Arg Gln Lys
   65                  70                  75                  80
   Ile Gln His Phe Asp His Glu Arg Val Pro Glu Arg Ala Val His Ala
                   85                  90                  95
   Arg Gly Ala Gly Ala His Gly Val Phe Thr Ser Tyr Ala Asp Trp Ser
               100                 105                 110
   Asn Ile Thr Ala Ala Ser Phe Leu Gly Ala Ala Gly Lys Glu Thr Pro
           115                 120                 125
   Thr Phe Val Arg Phe Ser Thr Val Ala Gly Ser Arg Gly Ser Ala Asp
       130                 135                 140
   Thr Ala Arg Asp Val His Gly Phe Ala Thr Arg Phe Tyr Thr Asp Glu
   145                 150                 155                 160
   Gly Asn Tyr Asp Ile Val Gly Asn Asn Ile Pro Val Phe Phe Ile Gln
                   165                 170                 175
   Asp Ala Ile Leu Phe Pro Asp Leu Ile His Ser Val Lys Pro Gln Pro
   180 185 190
   Ala Asn Glu Ile Pro Gln Ala Ala Thr Ala His Asp Thr Ala Tyr Asp
   195 200 205
   Phe Phe Gly Gln Gln Pro Ser Thr Leu His Thr Leu Phe Trp Ala Met
   210 215 220
   Ala Gly His Gly Ile Pro Arg Ser Phe Arg His Val Asp Gly Phe Gly
   225 230 235 240
   Val His Thr Tyr Arg Phe Val Thr Glu Asp Gly Ser Ser Lys Leu Val
   245 250 255
   Lys Phe His Trp Thr Ser Leu Gln Gly Arg Ala Ser Leu Val Trp Glu
   260 265 270
   Glu Ala Gln Ala Thr Ala Gly Lys Asn Ala Asp Phe Met Arg Gln Asp
   275 280 285
   Leu Tyr Asp Ser Ile Glu Ala Gly Arg Tyr Pro Glu Trp Glu Phe Gly
   290 295 300
   Val Gln Ile Ile Asp Glu Ser Asp Val Leu Ser Tyr Gly Phe Asp Leu
   305 310 315 320
   Leu Asp Pro Thr Lys Ile Leu Pro Val Glu Lys Val Pro Ile Thr Pro
   325 330 335
   Leu Gly Lys Met Gln Leu Asn Arg Asn Pro Leu Asn Tyr Phe Ala Glu
   340 345 350
   Thr Glu Gln Val Met Phe Gln Pro Gly His Ile Val Arg Gly Ile Asp
   355 360 365
   Phe Thr Glu Asp Pro Leu Leu Gln Gly Arg Leu Phe Ser Tyr Leu Asp
   370 375 380
   Thr Gln Leu Asn Arg Asn Gly Gly Pro Asn Phe Glu Gln Ile Pro Ile
   385 390 395 400
   Asn Arg Pro Arg Val Pro Ile His Asn Asn Asn Arg Asp Gly Phe Ala
   405 410 415
   Gln Met Phe Ile Pro Leu Asn Gln Ala Ala Tyr Ser Pro Asn Thr Leu
   420 425 430
   Asn Asn Gly Ser Pro Arg Gln Ala Asn Glu Thr Val Gly Asn Gly Phe
           435                 440                 445
   Phe Thr Thr Pro Arg Arg Ser Ala Asp Gly His Leu Val Arg Ala Thr
       450                 455                 460
   Ser Pro Thr Phe Ala Asp Val Trp Ser Gln Pro Gly Leu Phe Tyr Asn
   465                 470                 475                 480
   Ser Leu Thr Ala Thr Glu Gln Gln Phe Val Ile Asn Ala Leu Arg Phe
                   485                 490                 495
   Glu Leu Ser Asn Val Lys Ser Glu Asp Val Lys Ser Asn Phe Ile Thr
               500                 505                 510
   Gln Ile Asn Arg Val Asn Asn Thr Leu Ala Thr Leu Val Ala Ser Ala
           515                 520                 525
   Ile Gly Val Ser Ala Pro Glu Pro Asp Ser Thr Tyr Tyr His Ser Asn
       530                 535                 540
   Lys Thr Ser Asn Val Gly Thr Phe Gly Thr Pro Leu Lys Lys Leu Asp
   545                 550                 555                 560
   Gly Leu Lys Val Gly Val Leu Ala Ser Val Ser Asp Glu Ser Ser Ile
                   565                 570                 575
   Ala Glu Gly Gln Ala Leu Ala Gln Ser Leu Ala Asp Ser Asn Val Asp
               580                 585                 590
   Val Val Ile Val Ala Glu His Leu Thr Ser Asn Val Ser Ala Thr Tyr
           595                 600                 605
   Ser Gly Ser Asp Ala Thr Asn Phe Asp Ala Val Ile Val Ser Ser Gly
       610                 615                 620
   Ala Glu Gly Leu Phe Gly Pro Gln Thr Phe Thr Ala Glu Ser Asn Thr
   625                 630                 635                 640
   Thr Leu Tyr Pro Ala Gly Arg Pro Ser Gln Ile Leu Val Asp Ala Phe
                   645                 650                 655
   Arg Phe Gly Lys Pro Val Gly Ala Val Gly Gly Ala Ser Ala Ala Leu
               660                 665                 670
   Ser Ala Val Asp Ile Ser Thr Asp Arg Ser Gly Val Val Thr Gly Asp
           675                 680                 685
   Ser Val Ser Asp Asp Phe Val Asn Gln Leu Thr Glu Asp Leu Ala Thr
   690 695 700
   Phe Lys Phe Leu Asp Arg Phe Ala Val Asp Glu
   705 710 715
<210> 6
   <211> 2145
   <212> DNA
   <213> Penicillium sp.
<400> 6
   gcatgcccaa tgctgactgg cgaaatccca gctggcagtg ttgcgaatcc tcatcatcac 60
   ggaaagcgtg acgattcaaa tgcttcctcc gaaacagaag cctttctgtc cgagttctac 120
   ctcaacgaca acgatgccta tctcaccacc gatgtaggcg gtccgatcga ggatcaacac 180
   agtttgaagg ccggcattcg tggatcgacc ctcttggaag atttcatctt ccgtcagaaa 240
   atccagcatt tcgatcatga gcgtgtcccg gaacgtgccg tgcatgctcg aggtgcaggt 300
   gctcatggtg tattcacttc atatgccgac tggtccaaca tcactgctgc ttcattcttg 360
   ggagctgctg gaaaggaaac gcccacattt gtccgcttct cgactgttgc aggcagccga 420
   ggaagtgccg acaccgctcg tgacgttcac ggatttgcta ctcgcttcta tactgacgag 480
   ggaaactatg acatcgttgg aaacaacatc cctgtcttct tcatccaaga tgctatccta 540
   ttcccagatc tcatccatag cgtcaagcca cagccagcca atgaaatccc acaggctgct 600
   actgcacacg acacggccta tgacttcttt ggtcaacagc caagcactct gcataccctc 660
   ttctgggcaa tggcaggtca tggtatccca cggtctttcc gtcatgttga cggattcggc 720
   gtccacacct atcggttcgt gactgaagat ggctcgtcca aattggtcaa atttcactgg 780
   acatcactac agggccgagc cagtctggtc tgggaggaag ctcaggccac tgctggcaaa 840
   aatgcggact ttatgagaca ggatctgtat gatagcattg aggccggccg ttatccagag 900
   tgggagttcg gcgtgcaaat cattgatgag tcggatgtct taagctacgg atttgacttg 960
   ttggatccaa ccaagattct tccggtggaa aaagttccaa ttactccact cggaaaaatg 1020
   caactcaacc gtaatccatt gaattacttt gccgagacag agcaagtcat gttccaacct 1080
   ggccacattg ttcgtggtat cgactttacc gaggatcctc ttctccaagg tcgtttattc 1140
   tcctacctcg acactcagct gaatcgcaac ggtggtccca actttgaaca aatcccaatc 1200
   aatcgtcctc gtgtacctat ccacaacaat aaccgcgatg gattcgccca aatgtttatt 1260
   cctttgaacc aagcagcata ttcacccaac accttgaata atggctctcc tcgacaagcc 1320
   aacgagactg taggaaatgg cttctttacc acccccaggc gctccgcaga tggacacctt 1380
   gttcgcgcta ctagcccaac atttgccgac gtgtggtctc agcctggctt gttttacaac 1440
   tccttgacgg ctaccgaaca acaatttgtg atcaatgctt tgcgtttcga gttgtccaat 1500
   gtaaagagcg aggatgttaa aagcaatttc atcacacaga taaatcgcgt aaacaatacg 1560
   ctagcaacac ttgtggcttc tgcaattggg gtctccgcgc ccgaacccga ctctacatac 1620
   taccacagca ataagacgtc taatgtcgga acattcggta ctccgttgaa aaagcttgac 1680
   ggtctcaagg tcggagtcct tgcttcggtg agcgatgaaa gtagtattgc cgagggacaa 1740
   gcattggcac aaagcctagc ggactcgaac gtggacgtcg ttatcgtcgc cgagcatctt 1800
   acttcgaacg tgtcagctac atactctgga tcagacgcaa cgaacttcga tgctgttatt 1860
   gtcagctcag gggctgaagg tctctttgga ccccaaacct ttactgccga atctaacaca 1920
   acactttatc cggcaggccg tcctagtcag attttggtcg atgccttccg ctttggcaag 1980
   ccggttggag cagtgggtgg cgccagtgca gctctttcgg cggtggatat cagtactgat 2040
   cgcagtggtg tggttactgg tgattccgtc agtgacgact ttgtcaacca gctaacggag 2100
   gaccttgcca cattcaaatt cttggaccga ttcgctgtgg atgag 2145
<210> 7
   <211> 715
   <212> PRT
   <213> Penicillium sp.
<400> 7
   Ala Cys Pro Met Leu Thr Gly Glu Ile Pro Ala Gly Ser Val Ala Asn
   1 5 10 15
   Pro His His His Gly Lys Arg Asp Asp Ser Asn Ala Ser Ser Glu Thr
   20 25 30
   Glu Ala Phe Leu Ser Glu Phe Tyr Leu Asn Asp Asn Asp Ala Tyr Leu
   35 40 45
   Thr Thr Asp Val Gly Gly Pro Ile Glu Asp Gln Asn Ser Leu Lys Ala
   50 55 60
   Gly Ile Arg Gly Ser Thr Leu Leu Glu Asp Phe Ile Phe Arg Gln Lys
   65 70 75 80
   Ile Gln His Phe Asp His Glu Arg Val Pro Glu Arg Ala Val His Ala
   85 90 95
   Arg Gly Ala Gly Ala His Gly Val Phe Thr Ser Tyr Ala Asp Trp Ser
   100 105 110
   Asn Ile Thr Ala Ala Ser Phe Leu Gly Ala Ser Gly Lys Glu Thr Pro
   115 120 125
   Thr Phe Val Arg Phe Ser Thr Val Ala Gly Ser Arg Gly Ser Ala Asp
   130 135 140
   Thr Ala Arg Asp Val His Gly Phe Ala Thr Arg Phe Tyr Thr Asp Glu
   145                 150                 155                 160
   Gly Asn Tyr Asp Ile Val Gly Asn Asn Ile Pro Val Phe Phe Ile Gln
                   165                 170                 175
   Asp Ala Ile Leu Phe Pro Asp Leu Ile His Ser Val Lys Pro Gln Pro
               180                 185                 190
   Ala Asn Glu Ile Pro Gln Ala Ala Thr Ala His Asp Thr Ala Tyr Asp
           195                 200                 205
   Phe Phe Gly Gln Gln Pro Ser Thr Leu His Thr Leu Phe Trp Ala Met
       210                 215                 220
   Ala Gly His Gly Ile Pro Arg Ser Phe Arg His Val Asp Gly Phe Gly
   225                 230                 235                 240
   Val His Thr Tyr Arg Phe Val Thr Asp Asp Gly Ser Ser Lys Leu Val
                   245                 250                 255
   Lys Phe His Trp Thr Ser Leu Gln Gly Arg Ala Ser Leu Val Trp Glu
               260                 265                 270
   Glu Ala Gln Ala Thr Ala Gly Lys Asn Ala Asp Phe Met Arg Gln Asp
           275                 280                 285
   Leu Tyr Asp Ser Ile Glu Ala Gly Arg Tyr Pro Glu Trp Glu Leu Gly
       290                 295                 300
   Val Gln Ile Ile Glu Glu Ser Asp Val Leu Ser Tyr Gly Phe Asp Leu
   305                 310                 315                 320
   Leu Asp Pro Thr Lys Ile Leu Pro Val Glu Lys Val Pro Ile Thr Ala
                   325                 330                 335
   Leu Gly Lys Met Gln Leu Asn Arg Asn Pro Leu Asn Tyr Phe Ala Glu
               340                 345                 350
   Thr Glu Gln Val Met Phe Gln Pro Gly His Ile Val Arg Gly Ile Asp
           355                 360                 365
   Phe Thr Glu Asp Pro Leu Leu Gln Gly Arg Leu Phe Ser Tyr Leu Asp
       370                 375                 380
   Thr Gln Leu Asn Arg Asn Gly Gly Pro Asn Phe Glu Gln Ile Pro Ile
   385                 390                 395                 400
   Asn Arg Pro Arg Val Pro Ile His Asn Asn Asn Arg Asp Gly Phe Ala
   405 410 415
   Gln Met Phe Ile Pro Leu Asn Gln Ala Ala Tyr Ser Pro Asn Thr Leu
   420 425 430
   Asn Asn Gly Ser Pro Arg Gln Ala Asn Glu Thr Val Gly Asn Gly Phe
   435 440 445
   Phe Thr Ala Pro Gly Arg Ser Ala Asp Gly His Leu Val Arg Ala Thr
   450 455 460
   Ser Pro Thr Phe Ala Asp Val Trp Ser Gln Pro Gly Leu Phe Tyr Asn
   465 470 475 480
   Ser Leu Thr Ala Thr Glu Gln Gln Phe Val Ile Asn Ala Leu Arg Phe
   485 490 495
   Glu Leu Ser Asn Val Lys Ser Glu Asp Val Lys Ser Asn Phe Ile Thr
   500 505 510
   Gln Ile Asn Arg Val Asn Asn Thr Leu Ala Thr Leu Val Ala Ser Ala
   515 520 525
   Ile Gly Val Ser Ala Pro Glu Pro Asp Ser Thr Tyr Tyr His Ser Asn
   530 535 540
   Lys Thr Ser Asn Val Gly Thr Phe Gly Thr Pro Leu Lys Lys Leu Asp
   545 550 555 560
   Gly Leu Lys Val Gly Val Leu Ala Ser Val Asn Gly Glu Ser Ser Ile
   565 570 575
   Ala Glu Gly Gln Ala Leu Ala Gln Ser Leu Ala Gly Ser Asn Val Asp
   580 585 590
   Val Val Ile Val Ala Glu His Leu Thr Ser Asn Val Ser Ala Thr Tyr
   595 600 605
   Ser Gly Ser Asp Ala Thr Asn Phe Asp Ala Val Ile Val Ser Ser Gly
   610 615 620
   Ala Glu Gly Leu Phe Gly Pro Gln Thr Phe Thr Ala Glu Ser Asn Thr
   625 630 635 640
   Thr Leu Tyr Pro Ala Gly Arg Pro Ser Gln Ile Leu Val Asp Ala Phe
   645 650 655
   Arg Phe Gly Lys Pro Val Gly Ala Val Gly Gly Ala Ser Ala Ala Leu
   660 665 670
   Ser Ala Val Asp Ile Ser Thr Asp Arg Ser Gly Val Ile Thr Gly Asp
   675 680 685
   Ser Val Ser Asp Asp Phe Val Lys Gln Leu Thr Glu Asp Leu Ala Thr
   690 695 700
   Phe Lys Phe Leu Asp Arg Phe Ala Val Asp Glu
   705 710 715
<210> 8
   <211> 2145
   <212> DNA
   <213> Penicillium sp.
<400> 8
   gcatgcccaa tgctgactgg cgaaatccca gctggtagtg ttgccaatcc tcatcatcac      60
   ggaaagcgtg acgattcaaa tgcttcctcc gaaacagaag cctttctgtc cgagttctac     120
   ctcaacgaca acgatgccta tctcaccacc gatgtaggcg gtccgatcga ggatcaaaac     180
   agtttgaagg ccggcattcg tggatcgacc ctcttggaag acttcatctt ccgtcagaaa     240
   atccagcatt ttgatcatga gcgtgtcccg gaacgtgccg tgcatgctcg aggtgcaggt     300
   gctcatggtg tatttacttc atatgccgac tggtccaaca tcactgctgc ttcatttttg     360
   ggagcttccg gaaaggaaac gcccacattt gtccgcttct cgactgttgc aggcagccga     420
   ggaagtgccg acaccgctcg tgacgttcac ggatttgcta ctcgcttcta tactgacgag     480
   ggaaactatg acattgttgg aaacaacatt cctgtcttct tcatccaaga tgctatctta     540
   ttcccagatc tcatccatag cgttaagcca cagccagcca atgaaatccc acaggctgct     600
   actgcacacg acacggccta tgacttcttt ggtcaacagc caagcactct gcataccctc     660
   ttctgggcaa tggcaggcca tggtatccca cggtctttcc gtcatgttga cggattcggt     720
   gtccacacct atcggttcgt gacagatgat ggctcgtcca agttggtcaa atttcactgg     780
   acatcgctgc aaggtcgggc cagtctggtc tgggaggaag ctcaggccac tgctggcaaa     840
   aatgccgact ttatgagaca ggatctgtat gatagcattg aggctggccg ttatccagag     900
   tgggagctcg gcgtgcaaat aattgaggag tcggatgtct taagctacgg atttgacctg     960
   ttggatccaa ccaagattct tccggttgaa aaagttccaa ttactgcgct cggaaaaatg    1020
   caactcaacc gtaatccatt gaattacttt gccgagacag agcaagtcat gttccaacct    1080
   ggccacattg ttcgtggtat cgacttcacc gaggatcctc ttctccaggg tcgtttattc    1140
   tcctacctcg atactcagct gaatcgcaat ggtggtccca actttgaaca aattccaatc    1200
   aatcgtccgc gtgttcctat ccacaacaac aaccgcgatg gattcgccca aatgtttatt    1260
   cctttgaacc aggcagcata ttcacccaac accttgaata atggctctcc tcgacaagcc    1320
   aacgagactg tcggaaatgg cttctttacc gcccccgggc gctccgcaga tggacacctt    1380
   gttcgcgcta cgagcccaac atttgccgac gtgtggtctc agcctggctt gttttacaac 1440
   tccttgacgg ctaccgaaca acagttcgtg atcaatgctt tgcgtttcga attgtctaat 1500
   gtaaagagcg aggatgttaa aagcaatttc atcacacaga taaatcgcgt aaacaacacg 1560
   ttagcaacac ttgtggcttc tgcaattgga gtctccgcgc ccgaacccga ctctacatac 1620
   taccacagca ataagacgtc taatgtcgga acattcggta ctccgttgaa aaagcttgac 1680
   ggtctcaagg tcggagtcct tgcttcggtg aacggtgaaa gtagtattgc cgagggacaa 1740
   gcattggcac aaagcctagc gggctcgaac gtggacgtcg ttatcgtcgc cgagcatctt 1800
   acttcgaacg tgtcagctac atactctgga tcagacgcaa cgaactttga tgctgttatt 1860
   gtcagctcag gggctgaagg tctctttgga cctcaaacct ttacagccga atccaataca 1920
   acactttatc cggcaggccg tcctagccag attttggtcg atgccttccg ctttggcaag 1980
   ccggttggag cagttggtgg tgccagtgca gctctgtcag cggtggatat cagtactgat 2040
   cgtagtggtg tgattactgg tgattccgtc agtgacgact ttgtcaagca gctaacggag 2100
   gaccttgcca cattcaaatt cttggaccga ttcgctgtgg atgag 2145
<210> 9
   <211> 734
   <212> PRT
   <213> Penicillium sp.
<400> 9
   Met Gly Gly Leu Tyr Ser Leu Gly Thr Phe Ala Gly Leu Thr Ala Ala
   1 5 10 15
   Ala Ser Ala Ala Cys Pro Met Leu Thr Gly Glu Ile Pro Ala Gly Ser
   20 25 30
   Val Ala Asn Pro His His His Gly Lys Arg Asp Asp Ser Asn Ala Ser
   35 40 45
   Ser Glu Thr Glu Ala Phe Leu Ser Glu Phe Tyr Leu Asn Asp Asn Asp
   50 55 60
   Ala Tyr Leu Thr Thr Asp Val Gly Gly Pro Ile Glu Asp Gln His Ser
   65 70 75 80
   Leu Lys Ala Gly Ile Arg Gly Ser Thr Leu Leu Glu Asp Phe Ile Phe
   85 90 95
   Arg Gln Lys Ile Gln His Phe Asp His Glu Arg Val Pro Glu Arg Ala
   100 105 110
   Val His Ala Arg Gly Ala Gly Ala His Gly Val Phe Thr Ser Tyr Ala
   115 120 125
   Asp Trp Ser Asn Ile Thr Ala Ala Ser Phe Leu Gly Ala Ala Gly Lys
       130                 135                 140
   Glu Thr Pro Thr Phe Val Arg Phe Ser Thr Val Ala Gly Ser Arg Gly
   145                 150                 155                 160
   Ser Ala Asp Thr Ala Arg Asp Val His Gly Phe Ala Thr Arg Phe Tyr
                   165                 170                 175
   Thr Asp Glu Gly Asn Tyr Asp Ile Val Gly Asn Asn Ile Pro Val Phe
               180                 185                 190
   Phe Ile Gln Asp Ala Ile Leu Phe Pro Asp Leu Ile His Ser Val Lys
           195                 200                 205
   Pro Gln Pro Ala Asn Glu Ile Pro Gln Ala Ala Thr Ala His Asp Thr
       210                 215                 220
   Ala Tyr Asp Phe Phe Gly Gln Gln Pro Ser Thr Leu His Thr Leu Phe
   225                 230                 235                 240
   Trp Ala Met Ala Gly His Gly Ile Pro Arg Ser Phe Arg His Val Asp
                   245                 250                 255
   Gly Phe Gly Val His Thr Tyr Arg Phe Val Thr Glu Asp Gly Ser Ser
               260                 265                 270
   Lys Leu Val Lys Phe His Trp Thr Ser Leu Gln Gly Arg Ala Ser Leu
           275                 280                 285
   Val Trp Glu Glu Ala Gln Ala Thr Ala Gly Lys Asn Ala Asp Phe Met
       290                 295                 300
   Arg Gln Asp Leu Tyr Asp Ser Ile Glu Ala Gly Arg Tyr Pro Glu Trp
   305                 310                 315                 320
   Glu Phe Gly Val Gln Ile Ile Asp Glu Ser Asp Val Leu Ser Tyr Gly
                   325                 330                 335
   Phe Asp Leu Leu Asp Pro Thr Lys Ile Leu Pro Val Glu Lys Val Pro
               340                 345                 350
   Ile Thr Pro Leu Gly Lys Met Gln Leu Asn Arg Asn Pro Leu Asn Tyr
           355                 360                 365
   Phe Ala Glu Thr Glu Gln Val Met Phe Gln Pro Gly His Ile Val Arg
       370                 375                 380
   Gly Ile Asp Phe Thr Glu Asp Pro Leu Leu Gln Gly Arg Leu Phe Ser
   385 390 395 400
   Tyr Leu Asp Thr Gln Leu Asn Arg Asn Gly Gly Pro Asn Phe Glu Gln
   405 410 415
   Ile Pro Ile Asn Arg Pro Arg Val Pro Ile His Asn Asn Asn Arg Asp
   420 425 430
   Gly Phe Ala Gln Met Phe Ile Pro Leu Asn Gln Ala Ala Tyr Ser Pro
   435 440 445
   Asn Thr Leu Asn Asn Gly Ser Pro Arg Gln Ala Asn Glu Thr Val Gly
   450 455 460
   Asn Gly Phe Phe Thr Thr Pro Arg Arg Ser Ala Asp Gly His Leu Val
   465 470 475 480
   Arg Ala Thr Ser Pro Thr Phe Ala Asp Val Trp Ser Gln Pro Gly Leu
   485 490 495
   Phe Tyr Asn Ser Leu Thr Ala Thr Glu Gln Gln Phe Val Ile Asn Ala
   500 505 510
   Leu Arg Phe Glu Leu Ser Asn Val Lys Ser Glu Asp Val Lys Ser Asn
   515 520 525
   Phe Ile Thr Gln Ile Asn Arg Val Asn Asn Thr Leu Ala Thr Leu Val
   530 535 540
   Ala Ser Ala Ile Gly Val Ser Ala Pro Glu Pro Asp Ser Thr Tyr Tyr
   545 550 555 560
   His Ser Asn Lys Thr Ser Asn Val Gly Thr Phe Gly Thr Pro Leu Lys
   565 570 575
   Lys Leu Asp Gly Leu Lys Val Gly Val Leu Ala Ser Val Ser Asp Glu
   580 585 590
   Ser Ser Ile Ala Glu Gly Gln Ala Leu Ala Gln Ser Leu Ala Asp Ser
   595 600 605
   Asn Val Asp Val Val Ile Val Ala Glu His Leu Thr Ser Asn Val Ser
   610 615 620
   Ala Thr Tyr Ser Gly Ser Asp Ala Thr Asn Phe Asp Ala Val Ile Val
   625 630 635 640
   Ser Ser Gly Ala Glu Gly Leu Phe Gly Pro Gln Thr Phe Thr Ala Glu
   645 650 655
   Ser Asn Thr Thr Leu Tyr Pro Ala Gly Arg Pro Ser Gln Ile Leu Val
   660 665 670
   Asp Ala Phe Arg Phe Gly Lys Pro Val Gly Ala Val Gly Gly Ala Ser
   675 680 685
   Ala Ala Leu Ser Ala Val Asp Ile Ser Thr Asp Arg Ser Gly Val Val
   690 695 700
   Thr Gly Asp Ser Val Ser Asp Asp Phe Val Asn Gln Leu Thr Glu Asp
   705 710 715 720
   Leu Ala Thr Phe Lys Phe Leu Asp Arg Phe Ala Val Asp Glu
   725 730
<210> 10
   <211> 2202
   <212> DNA
   <213> Penicillium sp.
<400> 10
   atgggaggat tatactccct cggcaccttc gccggtctca ctgcggcggc ttcggctgca      60
   tgcccaatgc tgactggcga aatcccagct ggcagtgttg cgaatcctca tcatcacgga     120
   aagcgtgacg attcaaatgc ttcctccgaa acagaagcct ttctgtccga gttctacctc     180
   aacgacaacg atgcctatct caccaccgat gtaggcggtc cgatcgagga tcaacacagt     240
   ttgaaggccg gcattcgtgg atcgaccctc ttggaagatt tcatcttccg tcagaaaatc     300
   cagcatttcg atcatgagcg tgtcccggaa cgtgccgtgc atgctcgagg tgcaggtgct     360
   catggtgtat tcacttcata tgccgactgg tccaacatca ctgctgcttc attcttggga     420
   gctgctggaa aggaaacgcc cacatttgtc cgcttctcga ctgttgcagg cagccgagga     480
   agtgccgaca ccgctcgtga cgttcacgga tttgctactc gcttctatac tgacgaggga     540
   aactatgaca tcgttggaaa caacatccct gtcttcttca tccaagatgc tatcctattc     600
   ccagatctca tccatagcgt caagccacag ccagccaatg aaatcccaca ggctgctact     660
   gcacacgaca cggcctatga cttctttggt caacagccaa gcactctgca taccctcttc     720
   tgggcaatgg caggtcatgg tatcccacgg tctttccgtc atgttgacgg attcggcgtc     780
   cacacctatc ggttcgtgac tgaagatggc tcgtccaaat tggtcaaatt tcactggaca     840
   tcactacagg gccgagccag tctggtctgg gaggaagctc aggccactgc tggcaaaaat     900
   gcggacttta tgagacagga tctgtatgat agcattgagg ccggccgtta tccagagtgg     960
   gagttcggcg tgcaaatcat tgatgagtcg gatgtcttaa gctacggatt tgacttgttg    1020
   gatccaacca agattcttcc ggtggaaaaa gttccaatta ctccactcgg aaaaatgcaa    1080
   ctcaaccgta atccattgaa ttactttgcc gagacagagc aagtcatgtt ccaacctggc 1140
   cacattgttc gtggtatcga ctttaccgag gatcctcttc tccaaggtcg tttattctcc 1200
   tacctcgaca ctcagctgaa tcgcaacggt ggtcccaact ttgaacaaat cccaatcaat 1260
   cgtcctcgtg tacctatcca caacaataac cgcgatggat tcgcccaaat gtttattcct 1320
   ttgaaccaag cagcatattc acccaacacc ttgaataatg gctctcctcg acaagccaac 1380
   gagactgtag gaaatggctt ctttaccacc cccaggcgct ccgcagatgg acaccttgtt 1440
   cgcgctacta gcccaacatt tgccgacgtg tggtctcagc ctggcttgtt ttacaactcc 1500
   ttgacggcta ccgaacaaca atttgtgatc aatgctttgc gtttcgagtt gtccaatgta 1560
   aagagcgagg atgttaaaag caatttcatc acacagataa atcgcgtaaa caatacgcta 1620
   gcaacacttg tggcttctgc aattggggtc tccgcgcccg aacccgactc tacatactac 1680
   cacagcaata agacgtctaa tgtcggaaca ttcggtactc cgttgaaaaa gcttgacggt 1740
   ctcaaggtcg gagtccttgc ttcggtgagc gatgaaagta gtattgccga gggacaagca 1800
   ttggcacaaa gcctagcgga ctcgaacgtg gacgtcgtta tcgtcgccga gcatcttact 1860
   tcgaacgtgt cagctacata ctctggatca gacgcaacga acttcgatgc tgttattgtc 1920
   agctcagggg ctgaaggtct ctttggaccc caaaccttta ctgccgaatc taacacaaca 1980
   ctttatccgg caggccgtcc tagtcagatt ttggtcgatg ccttccgctt tggcaagccg 2040
   gttggagcag tgggtggcgc cagtgcagct ctttcggcgg tggatatcag tactgatcgc 2100
   agtggtgtgg ttactggtga ttccgtcagt gacgactttg tcaaccagct aacggaggac 2160
   cttgccacat tcaaattctt ggaccgattc gctgtggatg ag 2202
<210> 11
   <211> 734
   <212> PRT
   <213> Penicillium sp.
<400> 11
   Met Arg Gly Leu Tyr Ser Leu Gly Ala Phe Ala Ser Leu Ile Ala Ala
   1               5                   10                  15
   Ala Ser Ala Ala Cys Pro Met Leu Thr Gly Glu Ile Pro Ala Gly Ser
               20                  25                  30
   Val Ala Asn Pro His His His Gly Lys Arg Asp Asp Ser Asn Ala Ser
           35                  40                  45
   Ser Glu Thr Glu Ala Phe Leu Ser Glu Phe Tyr Leu Asn Asp Asn Asp
       50                  55                  60
   Ala Tyr Leu Thr Thr Asp Val Gly Gly Pro Ile Glu Asp Gln Asn Ser
   65                  70                  75                  80
   Leu Lys Ala Gly Ile Arg Gly Ser Thr Leu Leu Glu Asp Phe Ile Phe
   85 90 95
   Arg Gln Lys Ile Gln His Phe Asp His Glu Arg Val Pro Glu Arg Ala
   100 105 110
   Val His Ala Arg Gly Ala Gly Ala His Gly Val Phe Thr Ser Tyr Ala
   115 120 125
   Asp Trp Ser Asn Ile Thr Ala Ala Ser Phe Leu Gly Ala Ser Gly Lys
   130 135 140
   Glu Thr Pro Thr Phe Val Arg Phe Ser Thr Val Ala Gly Ser Arg Gly
   145 150 155 160
   Ser Ala Asp Thr Ala Arg Asp Val His Gly Phe Ala Thr Arg Phe Tyr
   165 170 175
   Thr Asp Glu Gly Asn Tyr Asp Ile Val Gly Asn Asn Ile Pro Val Phe
   180 185 190
   Phe Ile Gln Asp Ala Ile Leu Phe Pro Asp Leu Ile His Ser Val Lys
   195 200 205
   Pro Gln Pro Ala Asn Glu Ile Pro Gln Ala Ala Thr Ala His Asp Thr
   210 215 220
   Ala Tyr Asp Phe Phe Gly Gln Gln Pro Ser Thr Leu His Thr Leu Phe
   225 230 235 240
   Trp Ala Met Ala Gly His Gly Ile Pro Arg Ser Phe Arg His Val Asp
   245 250 255
   Gly Phe Gly Val His Thr Tyr Arg Phe Val Thr Asp Asp Gly Ser Ser
   260 265 270
   Lys Leu Val Lys Phe His Trp Thr Ser Leu Gln Gly Arg Ala Ser Leu
   275 280 285
   Val Trp Glu Glu Ala Gln Ala Thr Ala Gly Lys Asn Ala Asp Phe Met
   290 295 300
   Arg Gln Asp Leu Tyr Asp Ser Ile Glu Ala Gly Arg Tyr Pro Glu Trp
   305 310 315 320
   Glu Leu Gly Val Gln Ile Ile Glu Glu Ser Asp Val Leu Ser Tyr Gly
   325 330 335
   Phe Asp Leu Leu Asp Pro Thr Lys Ile Leu Pro Val Glu Lys Val Pro
   340 345 350
   Ile Thr Ala Leu Gly Lys Met Gln Leu Asn Arg Asn Pro Leu Asn Tyr
   355 360 365
   Phe Ala Glu Thr Glu Gln Val Met Phe Gln Pro Gly His Ile Val Arg
   370 375 380
   Gly Ile Asp Phe Thr Glu Asp Pro Leu Leu Gln Gly Arg Leu Phe Ser
   385 390 395 400
   Tyr Leu Asp Thr Gln Leu Asn Arg Asn Gly Gly Pro Asn Phe Glu Gln
   405 410 415
   Ile Pro Ile Asn Arg Pro Arg Val Pro Ile His Asn Asn Asn Arg Asp
   420 425 430
   Gly Phe Ala Gln Met Phe Ile Pro Leu Asn Gln Ala Ala Tyr Ser Pro
   435 440 445
   Asn Thr Leu Asn Asn Gly Ser Pro Arg Gln Ala Asn Glu Thr Val Gly
   450 455 460
   Asn Gly Phe Phe Thr Ala Pro Gly Arg Ser Ala Asp Gly His Leu Val
   465 470 475 480
   Arg Ala Thr Ser Pro Thr Phe Ala Asp Val Trp Ser Gln Pro Gly Leu
   485 490 495
   Phe Tyr Asn Ser Leu Thr Ala Thr Glu Gln Gln Phe Val Ile Asn Ala
   500 505 510
   Leu Arg Phe Glu Leu Ser Asn Val Lys Ser Glu Asp Val Lys Ser Asn
   515 520 525
   Phe Ile Thr Gln Ile Asn Arg Val Asn Asn Thr Leu Ala Thr Leu Val
   530 535 540
   Ala Ser Ala Ile Gly Val Ser Ala Pro Glu Pro Asp Ser Thr Tyr Tyr
   545 550 555 560
   His Ser Asn Lys Thr Ser Asn Val Gly Thr Phe Gly Thr Pro Leu Lys
   565 570 575
   Lys Leu Asp Gly Leu Lys Val Gly Val Leu Ala Ser Val Asn Gly Glu
   580 585 590
   Ser Ser Ile Ala Glu Gly Gln Ala Leu Ala Gln Ser Leu Ala Gly Ser
   595 600 605
   Asn Val Asp Val Val Ile Val Ala Glu His Leu Thr Ser Asn Val Ser
   610 615 620
   Ala Thr Tyr Ser Gly Ser Asp Ala Thr Asn Phe Asp Ala Val Ile Val
   625 630 635 640
   Ser Ser Gly Ala Glu Gly Leu Phe Gly Pro Gln Thr Phe Thr Ala Glu
   645 650 655
   Ser Asn Thr Thr Leu Tyr Pro Ala Gly Arg Pro Ser Gln Ile Leu Val
   660 665 670
   Asp Ala Phe Arg Phe Gly Lys Pro Val Gly Ala Val Gly Gly Ala Ser
   675 680 685
   Ala Ala Leu Ser Ala Val Asp Ile Ser Thr Asp Arg Ser Gly Val Ile
   690 695 700
   Thr Gly Asp Ser Val Ser Asp Asp Phe Val Lys Gln Leu Thr Glu Asp
   705 710 715 720
   Leu Ala Thr Phe Lys Phe Leu Asp Arg Phe Ala Val Asp Glu
   725 730
<210> 12
   <211> 2202
   <212> DNA
   <213> Penicillium sp.
<400> 12
   atgcgaggat tatactccct cggcgccttc gccagtctca ttgcggcggc ttcggctgca      60
   tgcccaatgc tgactggcga aatcccagct ggtagtgttg ccaatcctca tcatcacgga     120
   aagcgtgacg attcaaatgc ttcctccgaa acagaagcct ttctgtccga gttctacctc     180
   aacgacaacg atgcctatct caccaccgat gtaggcggtc cgatcgagga tcaaaacagt     240
   ttgaaggccg gcattcgtgg atcgaccctc ttggaagact tcatcttccg tcagaaaatc     300
   cagcattttg atcatgagcg tgtcccggaa cgtgccgtgc atgctcgagg tgcaggtgct     360
   catggtgtat ttacttcata tgccgactgg tccaacatca ctgctgcttc atttttggga     420
   gcttccggaa aggaaacgcc cacatttgtc cgcttctcga ctgttgcagg cagccgagga     480
   agtgccgaca ccgctcgtga cgttcacgga tttgctactc gcttctatac tgacgaggga     540
   aactatgaca ttgttggaaa caacattcct gtcttcttca tccaagatgc tatcttattc     600
   ccagatctca tccatagcgt taagccacag ccagccaatg aaatcccaca ggctgctact     660
   gcacacgaca cggcctatga cttctttggt caacagccaa gcactctgca taccctcttc     720
   tgggcaatgg caggccatgg tatcccacgg tctttccgtc atgttgacgg attcggtgtc 780
   cacacctatc ggttcgtgac agatgatggc tcgtccaagt tggtcaaatt tcactggaca 840
   tcgctgcaag gtcgggccag tctggtctgg gaggaagctc aggccactgc tggcaaaaat 900
   gccgacttta tgagacagga tctgtatgat agcattgagg ctggccgtta tccagagtgg 960
   gagctcggcg tgcaaataat tgaggagtcg gatgtcttaa gctacggatt tgacctgttg 1020
   gatccaacca agattcttcc ggttgaaaaa gttccaatta ctgcgctcgg aaaaatgcaa 1080
   ctcaaccgta atccattgaa ttactttgcc gagacagagc aagtcatgtt ccaacctggc 1140
   cacattgttc gtggtatcga cttcaccgag gatcctcttc tccagggtcg tttattctcc 1200
   tacctcgata ctcagctgaa tcgcaatggt ggtcccaact ttgaacaaat tccaatcaat 1260
   cgtccgcgtg ttcctatcca caacaacaac cgcgatggat tcgcccaaat gtttattcct 1320
   ttgaaccagg cagcatattc acccaacacc ttgaataatg gctctcctcg acaagccaac 1380
   gagactgtcg gaaatggctt ctttaccgcc cccgggcgct ccgcagatgg acaccttgtt 1440
   cgcgctacga gcccaacatt tgccgacgtg tggtctcagc ctggcttgtt ttacaactcc 1500
   ttgacggcta ccgaacaaca gttcgtgatc aatgctttgc gtttcgaatt gtctaatgta 1560
   aagagcgagg atgttaaaag caatttcatc acacagataa atcgcgtaaa caacacgtta 1620
   gcaacacttg tggcttctgc aattggagtc tccgcgcccg aacccgactc tacatactac 1680
   cacagcaata agacgtctaa tgtcggaaca ttcggtactc cgttgaaaaa gcttgacggt 1740
   ctcaaggtcg gagtccttgc ttcggtgaac ggtgaaagta gtattgccga gggacaagca 1800
   ttggcacaaa gcctagcggg ctcgaacgtg gacgtcgtta tcgtcgccga gcatcttact 1860
   tcgaacgtgt cagctacata ctctggatca gacgcaacga actttgatgc tgttattgtc 1920
   agctcagggg ctgaaggtct ctttggacct caaaccttta cagccgaatc caatacaaca 1980
   ctttatccgg caggccgtcc tagccagatt ttggtcgatg ccttccgctt tggcaagccg 2040
   gttggagcag ttggtggtgc cagtgcagct ctgtcagcgg tggatatcag tactgatcgt 2100
   agtggtgtga ttactggtga ttccgtcagt gacgactttg tcaagcagct aacggaggac 2160
   cttgccacat tcaaattctt ggaccgattc gctgtggatg ag 2202
<210> 13
   <211> 2214
   <212> DNA
   <213> Pichia pastoris
<400> 13 caaaacagtt tgaaggccgg cattcgtgga tcgaccctct tggaagactt catcttccgt 300
cagaaaatcc agcattttga tcatgagcgt gtcccggaac gtgccgtgca tgctcgaggt 360
gcaggtgctc atggtgtatt tacttcatat gccgactggt ccaacatcac tgctgcttca 420
tttttgggag cttccggaaa ggaaacgccc acatttgtcc gcttctcgac tgttgcaggc 480
agccgaggaa gtgccgacac cgctcgtgac gttcacggat ttgctactcg cttctatact 540
gacgagggaa actatgacat tgttggaaac aacattcctg tcttcttcat ccaagatgct 600
atcttattcc cagatctcat ccatagcgtt aagccacagc cagccaatga aatcccacag 660
gctgctactg cacacgacac ggcctatgac ttctttggtc aacagccaag cactctgcat 720
accctcttct gggcaatggc aggccatggt atcccacggt ctttccgtca tgttgacgga 780
ttcggtgtcc acacctatcg gttcgtgaca gatgatggct cgtccaagtt ggtcaaattt 840
cactggacat cgctgcaagg tcgggccagt ctggtctggg aggaagctca ggccactgct 900
ggcaaaaatg ccgactttat gagacaggat ctgtatgata gcattgaggc tggccgttat 960
ccagagtggg agctcggcgt gcaaataatt gaggagtcgg atgtcttaag ctacggattt 1020
gacctgttgg atccaaccaa gattcttccg gttgaaaaag ttccaattac tgcgctcgga 1080
aaaatgcaac tcaaccgtaa tccattgaat tactttgccg agacagagca agtcatgttc 1140
caacctggcc acattgttcg tggtatcgac ttcaccgagg atcctcttct ccagggtcgt 1200
ttattctcct acctcgatac tcagctgaat cgcaatggtg gtcccaactt tgaacaaatt 1260
ccaatcaatc gtccgcgtgt tcctatccac aacaacaacc gcgatggatt cgcccaaatg 1320
tttattcctt tgaaccaggc agcatattca cccaacacct tgaataatgg ctctcctcga 1380
caagccaacg agactgtcgg aaatggcttc tttaccgccc ccgggcgctc cgcagatgga 1440
caccttgttc gcgctacgag cccaacattt gccgacgtgt ggtctcagcc tggcttgttt 1500
tacaactcct tgacggctac cgaacaacag ttcgtgatca atgctttgcg tttcgaattg 1560
tctaatgtaa agagcgagga tgttaaaagc aatttcatca cacagataaa tcgcgtaaac 1620
aacacgttag caacacttgt ggcttctgca attggagtct ccgcgcccga acccgactct 1680
acatactacc acagcaataa gacgtctaat gtcggaacat tcggtactcc gttgaaaaag 1740
cttgacggtc tcaaggtcgg agtccttgct tcggtgaacg gtgaaagtag tattgccgag 1800
ggacaagcat tggcacaaag cctagcgggc tcgaacgtgg acgtcgttat cgtcgccgag 1860
catcttactt cgaacgtgtc agctacatac tctggatcag acgcaacgaa ctttgatgct 1920
gttattgtca gctcaggggc tgaaggtctc tttggacctc aaacctttac agccgaatcc 1980
aatacaacac tttatccggc aggccgtcct agccagattt tggtcgatgc cttccgcttt 2040
ggcaagccgg ttggagcagt tggtggtgcc agtgcagctc tgtcagcggt ggatatcagt 2100
actgatcgta gtggtgtgat tactggtgat tccgtcagtg acgactttgt caagcagcta 2160
<210> 14
   <211> 2214
   <212> DNA
   <213> Pichia pastoris
<400> 14
   atgttctctc caattttgtc cttggaaatt attttagctt tggctacttt gcaatctgtc      60
   ttcgctgcat gcccaatgct gactggcgaa atcccagctg gcagtgttgc gaatcctcat     120
   catcacggaa agcgtgacga ttcaaatgct tcctccgaaa cagaagcctt tctgtccgag     180
   ttctacctca acgacaacga tgcctatctc accaccgatg taggcggtcc gatcgaggat     240
   caacacagtt tgaaggccgg cattcgtgga tcgaccctct tggaagattt catcttccgt     300
   cagaaaatcc agcatttcga tcatgagcgt gtcccggaac gtgccgtgca tgctcgaggt     360
   gcaggtgctc atggtgtatt cacttcatat gccgactggt ccaacatcac tgctgcttca     420
   ttcttgggag ctgctggaaa ggaaacgccc acatttgtcc gcttctcgac tgttgcaggc     480
   agccgaggaa gtgccgacac cgctcgtgac gttcacggat ttgctactcg cttctatact     540
   gacgagggaa actatgacat cgttggaaac aacatccctg tcttcttcat ccaagatgct     600
   atcctattcc cagatctcat ccatagcgtc aagccacagc cagccaatga aatcccacag     660
   gctgctactg cacacgacac ggcctatgac ttctttggtc aacagccaag cactctgcat     720
   accctcttct gggcaatggc aggtcatggt atcccacggt ctttccgtca tgttgacgga     780
   ttcggcgtcc acacctatcg gttcgtgact gaagatggct cgtccaaatt ggtcaaattt     840
   cactggacat cactacaggg ccgagccagt ctggtctggg aggaagctca ggccactgct     900
   ggcaaaaatg cggactttat gagacaggat ctgtatgata gcattgaggc cggccgttat     960
   ccagagtggg agttcggcgt gcaaatcatt gatgagtcgg atgtcttaag ctacggattt    1020
   gacttgttgg atccaaccaa gattcttccg gtggaaaaag ttccaattac tccactcgga    1080
   aaaatgcaac tcaaccgtaa tccattgaat tactttgccg agacagagca agtcatgttc    1140
   caacctggcc acattgttcg tggtatcgac tttaccgagg atcctcttct ccaaggtcgt    1200
   ttattctcct acctcgacac tcagctgaat cgcaacggtg gtcccaactt tgaacaaatc    1260
   ccaatcaatc gtcctcgtgt acctatccac aacaataacc gcgatggatt cgcccaaatg    1320
   tttattcctt tgaaccaagc agcatattca cccaacacct tgaataatgg ctctcctcga    1380
   caagccaacg agactgtagg aaatggcttc tttaccaccc ccaggcgctc cgcagatgga    1440
   caccttgttc gcgctactag cccaacattt gccgacgtgt ggtctcagcc tggcttgttt    1500
   tacaactcct tgacggctac cgaacaacaa tttgtgatca atgctttgcg tttcgagttg    1560
   tccaatgtaa agagcgagga tgttaaaagc aatttcatca cacagataaa tcgcgtaaac    1620
   aatacgctag caacacttgt ggcttctgca attggggtct ccgcgcccga acccgactct    1680
   acatactacc acagcaataa gacgtctaat gtcggaacat tcggtactcc gttgaaaaag 1740
   cttgacggtc tcaaggtcgg agtccttgct tcggtgagcg atgaaagtag tattgccgag 1800
   ggacaagcat tggcacaaag cctagcggac tcgaacgtgg acgtcgttat cgtcgccgag 1860
   catcttactt cgaacgtgtc agctacatac tctggatcag acgcaacgaa cttcgatgct 1920
   gttattgtca gctcaggggc tgaaggtctc tttggacccc aaacctttac tgccgaatct 1980
   aacacaacac tttatccggc aggccgtcct agtcagattt tggtcgatgc cttccgcttt 2040
   ggcaagccgg ttggagcagt gggtggcgcc agtgcagctc tttcggcggt ggatatcagt 2100
   actgatcgca gtggtgtggt tactggtgat tccgtcagtg acgactttgt caaccagcta 2160
   acggaggacc ttgccacatt caaattcttg gaccgattcg ctgtggatga gtag 2214
<210> 15
   <211> 2415
   <212> DNA
   <213> Pichia pastoris
<400> 15
   atgagatttc cttcaatttt tactgctgtt ttattcgcag catcctccgc attagctgct 60
   ccagtcaaca ctacaacaga agatgaaacg gcacaaattc cggctgaagc tgtcatcggt 120
   tactcagatt tagaagggga tttcgatgtt gctgttttgc cattttccaa cagcacaaat 180
   aacgggttat tgtttataaa tactactatt gccagcattg ctgctaaaga agaaggggta 240
   tctctcgaga aaagagaggc tgaagctgca tgcccaatgc tgactggcga aatcccagct 300
   ggtagtgttg ccaatcctca tcatcacgga aagcgtgacg attcaaatgc ttcctccgaa 360
   acagaagcct ttctgtccga gttctacctc aacgacaacg atgcctatct caccaccgat 420
   gtaggcggtc cgatcgagga tcaaaacagt ttgaaggccg gcattcgtgg atcgaccctc 480
   ttggaagact tcatcttccg tcagaaaatc cagcattttg atcatgagcg tgtcccggaa 540
   cgtgccgtgc atgctcgagg tgcaggtgct catggtgtat ttacttcata tgccgactgg 600
   tccaacatca ctgctgcttc atttttggga gcttccggaa aggaaacgcc cacatttgtc 660
   cgcttctcga ctgttgcagg cagccgagga agtgccgaca ccgctcgtga cgttcacgga 720
   tttgctactc gcttctatac tgacgaggga aactatgaca ttgttggaaa caacattcct 780
   gtcttcttca tccaagatgc tatcttattc ccagatctca tccatagcgt taagccacag 840
   ccagccaatg aaatcccaca ggctgctact gcacacgaca cggcctatga cttctttggt 900
   caacagccaa gcactctgca taccctcttc tgggcaatgg caggccatgg tatcccacgg 960
   tctttccgtc atgttgacgg attcggtgtc cacacctatc ggttcgtgac agatgatggc 1020
   tcgtccaagt tggtcaaatt tcactggaca tcgctgcaag gtcgggccag tctggtctgg 1080
   gaggaagctc aggccactgc tggcaaaaat gccgacttta tgagacagga tctgtatgat 1140
   agcattgagg ctggccgtta tccagagtgg gagctcggcg tgcaaataat tgaggagtcg 1200
   gatgtcttaa gctacggatt tgacctgttg gatccaacca agattcttcc ggttgaaaaa 1260
   gttccaatta ctgcgctcgg aaaaatgcaa ctcaaccgta atccattgaa ttactttgcc 1320
   gagacagagc aagtcatgtt ccaacctggc cacattgttc gtggtatcga cttcaccgag 1380
   gatcctcttc tccagggtcg tttattctcc tacctcgata ctcagctgaa tcgcaatggt 1440
   ggtcccaact ttgaacaaat tccaatcaat cgtccgcgtg ttcctatcca caacaacaac 1500
   cgcgatggat tcgcccaaat gtttattcct ttgaaccagg cagcatattc acccaacacc 1560
   ttgaataatg gctctcctcg acaagccaac gagactgtcg gaaatggctt ctttaccgcc 1620
   cccgggcgct ccgcagatgg acaccttgtt cgcgctacga gcccaacatt tgccgacgtg 1680
   tggtctcagc ctggcttgtt ttacaactcc ttgacggcta ccgaacaaca gttcgtgatc 1740
   aatgctttgc gtttcgaatt gtctaatgta aagagcgagg atgttaaaag caatttcatc 1800
   acacagataa atcgcgtaaa caacacgtta gcaacacttg tggcttctgc aattggagtc 1860
   tccgcgcccg aacccgactc tacatactac cacagcaata agacgtctaa tgtcggaaca 1920
   ttcggtactc cgttgaaaaa gcttgacggt ctcaaggtcg gagtccttgc ttcggtgaac 1980
   ggtgaaagta gtattgccga gggacaagca ttggcacaaa gcctagcggg ctcgaacgtg 2040
   gacgtcgtta tcgtcgccga gcatcttact tcgaacgtgt cagctacata ctctggatca 2100
   gacgcaacga actttgatgc tgttattgtc agctcagggg ctgaaggtct ctttggacct 2160
   caaaccttta cagccgaatc caatacaaca ctttatccgg caggccgtcc tagccagatt 2220
   ttggtcgatg ccttccgctt tggcaagccg gttggagcag ttggtggtgc cagtgcagct 2280
   ctgtcagcgg tggatatcag tactgatcgt agtggtgtga ttactggtga ttccgtcagt 2340
   gacgactttg tcaagcagct aacggaggac cttgccacat tcaaattctt ggaccgattc 2400
   gctgtggatg agtga 2415
<210> 16
   <211> 2415
   <212> DNA
   <213> Pichia pastoris
<400> 16
   atgagatttc cttcaatttt tactgctgtt ttattcgcag catcctccgc attagctgct     60
   ccagtcaaca ctacaacaga agatgaaacg gcacaaattc cggctgaagc tgtcatcggt    120
   tactcagatt tagaagggga tttcgatgtt gctgttttgc cattttccaa cagcacaaat    180
   aacgggttat tgtttataaa tactactatt gccagcattg ctgctaaaga agaaggggta    240
   tctctcgaga aaagagaggc tgaagctgca tgcccaatgc tgactggcga aatcccagct    300
   ggcagtgttg cgaatcctca tcatcacgga aagcgtgacg attcaaatgc ttcctccgaa    360
   acagaagcct ttctgtccga gttctacctc aacgacaacg atgcctatct caccaccgat    420
   gtaggcggtc cgatcgagga tcaacacagt ttgaaggccg gcattcgtgg atcgaccctc    480
   ttggaagatt tcatcttccg tcagaaaatc cagcatttcg atcatgagcg tgtcccggaa 540
   cgtgccgtgc atgctcgagg tgcaggtgct catggtgtat tcacttcata tgccgactgg 600
   tccaacatca ctgctgcttc attcttggga gctgctggaa aggaaacgcc cacatttgtc 660
   cgcttctcga ctgttgcagg cagccgagga agtgccgaca ccgctcgtga cgttcacgga 720
   tttgctactc gcttctatac tgacgaggga aactatgaca tcgttggaaa caacatccct 780
   gtcttcttca tccaagatgc tatcctattc ccagatctca tccatagcgt caagccacag 840
   ccagccaatg aaatcccaca ggctgctact gcacacgaca cggcctatga cttctttggt 900
   caacagccaa gcactctgca taccctcttc tgggcaatgg caggtcatgg tatcccacgg 960
   tctttccgtc atgttgacgg attcggcgtc cacacctatc ggttcgtgac tgaagatggc 1020
   tcgtccaaat tggtcaaatt tcactggaca tcactacagg gccgagccag tctggtctgg 1080
   gaggaagctc aggccactgc tggcaaaaat gcggacttta tgagacagga tctgtatgat 1140
   agcattgagg ccggccgtta tccagagtgg gagttcggcg tgcaaatcat tgatgagtcg 1200
   gatgtcttaa gctacggatt tgacttgttg gatccaacca agattcttcc ggtggaaaaa 1260
   gttccaatta ctccactcgg aaaaatgcaa ctcaaccgta atccattgaa ttactttgcc 1320
   gagacagagc aagtcatgtt ccaacctggc cacattgttc gtggtatcga ctttaccgag 1380
   gatcctcttc tccaaggtcg tttattctcc tacctcgaca ctcagctgaa tcgcaacggt 1440
   ggtcccaact ttgaacaaat cccaatcaat cgtcctcgtg tacctatcca caacaataac 1500
   cgcgatggat tcgcccaaat gtttattcct ttgaaccaag cagcatattc acccaacacc 1560
   ttgaataatg gctctcctcg acaagccaac gagactgtag gaaatggctt ctttaccacc 1620
   cccaggcgct ccgcagatgg acaccttgtt cgcgctacta gcccaacatt tgccgacgtg 1680
   tggtctcagc ctggcttgtt ttacaactcc ttgacggcta ccgaacaaca atttgtgatc 1740
   aatgctttgc gtttcgagtt gtccaatgta aagagcgagg atgttaaaag caatttcatc 1800
   acacagataa atcgcgtaaa caatacgcta gcaacacttg tggcttctgc aattggggtc 1860
   tccgcgcccg aacccgactc tacatactac cacagcaata agacgtctaa tgtcggaaca 1920
   ttcggtactc cgttgaaaaa gcttgacggt ctcaaggtcg gagtccttgc ttcggtgagc 1980
   gatgaaagta gtattgccga gggacaagca ttggcacaaa gcctagcgga ctcgaacgtg 2040
   gacgtcgtta tcgtcgccga gcatcttact tcgaacgtgt cagctacata ctctggatca 2100
   gacgcaacga acttcgatgc tgttattgtc agctcagggg ctgaaggtct ctttggaccc 2160
   caaaccttta ctgccgaatc taacacaaca ctttatccgg caggccgtcc tagtcagatt 2220
   ttggtcgatg ccttccgctt tggcaagccg gttggagcag tgggtggcgc cagtgcagct 2280
   ctttcggcgg tggatatcag tactgatcgc agtggtgtgg ttactggtga ttccgtcagt 2340
   gacgactttg tcaaccagct aacggaggac cttgccacat tcaaattctt ggaccgattc 2400
   gctgtggatg agtag 2415
<210> 17
   <211> 2205
   <212> DNA
   <213> Aspergillus niger
<400> 17
   atgagaggcc tctactccct cggcaccttc gccggcctca ccgccgctgc ctccgccgcc 60
   tgccccatgc tcaccggcga gatccctgcc ggctccgtcg ccaaccccca ccaccacggc 120
   aagcgcgacg attccaacgc ctcctccgaa accgaggcct tcctctccga gttctacctc 180
   aacgataacg atgcctacct caccaccgat gtcggcggtc cgatcgagga tcaacacagt 240
   ttgaaggccg gcattcgtgg atcgaccctc ttggaagatt tcatcttccg tcagaaaatc 300
   cagcatttcg atcatgagcg tgtcccggaa cgtgccgtgc atgctcgagg tgcaggtgct 360
   catggtgtat tcacttcata tgccgactgg tccaacatca ctgctgcttc attcttggga 420
   gctgctggaa aggaaacgcc cacatttgtc cgcttctcga ctgttgcagg cagccgagga 480
   agtgccgaca ccgctcgtga cgttcacgga tttgctactc gcttctatac tgacgaggga 540
   aactatgaca tcgttggaaa caacatccct gtcttcttca tccaagatgc tatcctattc 600
   ccagatctca tccatagcgt caagccacag ccagccaatg aaatcccaca ggctgctact 660
   gcacacgaca cggcctatga cttctttggt caacagccaa gcactctgca taccctcttc 720
   tgggcaatgg caggtcatgg tatcccacgg tctttccgtc atgttgacgg attcggcgtc 780
   cacacctatc ggttcgtgac tgaagatggc tcgtccaaat tggtcaaatt tcactggaca 840
   tcactacagg gccgagccag tctggtctgg gaggaagctc aggccactgc tggcaaaaat 900
   gcggacttta tgagacagga tctgtatgat agcattgagg ccggccgtta tccagagtgg 960
   gagttcggcg tgcaaatcat tgatgagtcg gatgtcttaa gctacggatt tgacttgttg 1020
   gatccaacca agattcttcc ggtggaaaaa gttccaatta ctccactcgg aaaaatgcaa 1080
   ctcaaccgta atccattgaa ttactttgcc gagacagagc aagtcatgtt ccaacctggc 1140
   cacattgttc gtggtatcga ctttaccgag gatcctcttc tccaaggtcg tttattctcc 1200
   tacctcgaca ctcagctgaa tcgcaacggt ggtcccaact ttgaacaaat cccaatcaat 1260
   cgtcctcgtg tacctatcca caacaataac cgcgatggat tcgcccaaat gtttattcct 1320
   ttgaaccaag cagcatattc acccaacacc ttgaataatg gctctcctcg acaagccaac 1380
   gagactgtag gaaatggctt ctttaccacc cccaggcgct ccgcagatgg acaccttgtt 1440
   cgcgctacta gcccaacatt tgccgacgtg tggtctcagc ctggcttgtt ttacaactcc 1500
   ttgacggcta ccgaacaaca atttgtgatc aatgctttgc gtttcgagtt gtccaatgta 1560
   aagagcgagg atgttaaaag caatttcatc acacagataa atcgcgtaaa caatacgcta 1620
   gcaacacttg tggcttctgc aattggggtc tccgcgcccg aacccgactc tacatactac 1680
   cacagcaata agacgtctaa tgtcggaaca ttcggtactc cgttgaaaaa gcttgacggt 1740
   ctcaaggtcg gagtccttgc ttcggtgagc gatgaaagta gtattgccga gggacaagca   1800
   ttggcacaaa gcctagcgga ctcgaacgtg gacgtcgtta tcgtcgccga gcatcttact   1860
   tcgaacgtgt cagctacata ctctggatca gacgcaacga acttcgatgc tgttattgtc   1920
   agctcagggg ctgaaggtct ctttggaccc caaaccttta ctgccgaatc taacacaaca   1980
   ctttatccgg caggccgtcc tagtcagatt ttggtcgatg ccttccgctt tggcaagccg   2040
   gttggagcag tgggtggcgc cagtgcagct ctttcggcgg tggatatcag tactgatcgc   2100
   agtggtgtgg ttactggtga ttccgtcagt gacgactttg tcaaccagct aacggaggac   2160
   cttgccacat tcaaattctt ggaccgattc gctgtggatg agtag                    2205
<210> 18
   <211> 737
   <212> PRT
   <213> Pichia pastoris
<400> 18
   Met Phe Ser Pro Ile Leu Ser Leu Glu Ile Ile Leu Ala Leu Ala Thr
   1               5                   10                  15
   Leu Gln Ser Val Phe Ala Ala Cys Pro Met Leu Thr Gly Glu Ile Pro
               20                  25                  30
   Ala Gly Ser Val Ala Asn Pro His His His Gly Lys Arg Asp Asp Ser
           35                  40                  45
   Asn Ala Ser Ser Glu Thr Glu Ala Phe Leu Ser Glu Phe Tyr Leu Asn
       50                  55                  60
   Asp Asn Asp Ala Tyr Leu Thr Thr Asp Val Gly Gly Pro Ile Glu Asp
   65                  70                  75                  80
   Gln Asn Ser Leu Lys Ala Gly Ile Arg Gly Ser Thr Leu Leu Glu Asp
                   85                  90                  95
   Phe Ile Phe Arg Gln Lys Ile Gln His Phe Asp His Glu Arg Val Pro
               100                 105                 110
   Glu Arg Ala Val His Ala Arg Gly Ala Gly Ala His Gly Val Phe Thr
           115                 120                 125
   Ser Tyr Ala Asp Trp Ser Asn Ile Thr Ala Ala Ser Phe Leu Gly Ala
       130                 135                 140
   Ser Gly Lys Glu Thr Pro Thr Phe Val Arg Phe Ser Thr Val Ala Gly
   145                 150                 155                 160
   Ser Arg Gly Ser Ala Asp Thr Ala Arg Asp Val His Gly Phe Ala Thr
   165 170 175
   Arg Phe Tyr Thr Asp Glu Gly Asn Tyr Asp Ile Val Gly Asn Asn Ile
   180 185 190
   Pro Val Phe Phe Ile Gln Asp Ala Ile Leu Phe Pro Asp Leu Ile His
   195 200 205
   Ser Val Lys Pro Gln Pro Ala Asn Glu Ile Pro Gln Ala Ala Thr Ala
   210 215 220
   His Asp Thr Ala Tyr Asp Phe Phe Gly Gln Gln Pro Ser Thr Leu His
   225 230 235 240
   Thr Leu Phe Trp Ala Met Ala Gly His Gly Ile Pro Arg Ser Phe Arg
   245 250 255
   His Val Asp Gly Phe Gly Val His Thr Tyr Arg Phe Val Thr Asp Asp
   260 265 270
   Gly Ser Ser Lys Leu Val Lys Phe His Trp Thr Ser Leu Gln Gly Arg
   275 280 285
   Ala Ser Leu Val Trp Glu Glu Ala Gln Ala Thr Ala Gly Lys Asn Ala
   290 295 300
   Asp Phe Met Arg Gln Asp Leu Tyr Asp Ser Ile Glu Ala Gly Arg Tyr
   305 310 315 320
   Pro Glu Trp Glu Leu Gly Val Gln Ile Ile Glu Glu Ser Asp Val Leu
   325 330 335
   Ser Tyr Gly Phe Asp Leu Leu Asp Pro Thr Lys Ile Leu Pro Val Glu
   340 345 350
   Lys Val Pro Ile Thr Ala Leu Gly Lys Met Gln Leu Asn Arg Asn Pro
   355 360 365
   Leu Asn Tyr Phe Ala Glu Thr Glu Gln Val Met Phe Gln Pro Gly His
   370 375 380
   Ile Val Arg Gly Ile Asp Phe Thr Glu Asp Pro Leu Leu Gln Gly Arg
   385 390 395 400
   Leu Phe Ser Tyr Leu Asp Thr Gln Leu Asn Arg Asn Gly Gly Pro Asn
   405 410 415
   Phe Glu Gln Ile Pro Ile Asn Arg Pro Arg Val Pro Ile His Asn Asn
   420 425 430
   Asn Arg Asp Gly Phe Ala Gln Met Phe Ile Pro Leu Asn Gln Ala Ala
   435 440 445
   Tyr Ser Pro Asn Thr Leu Asn Asn Gly Ser Pro Arg Gln Ala Asn Glu
   450 455 460
   Thr Val Gly Asn Gly Phe Phe Thr Ala Pro Gly Arg Ser Ala Asp Gly
   465 470 475 480
   His Leu Val Arg Ala Thr Ser Pro Thr Phe Ala Asp Val Trp Ser Gln
   485 490 495
   Pro Gly Leu Phe Tyr Asn Ser Leu Thr Ala Thr Glu Gln Gln Phe Val
   500 505 510
   Ile Asn Ala Leu Arg Phe Glu Leu Ser Asn Val Lys Ser Glu Asp Val
   515 520 525
   Lys Ser Asn Phe Ile Thr Gln Ile Asn Arg Val Asn Asn Thr Leu Ala
   530 535 540
   Thr Leu Val Ala Ser Ala Ile Gly Val Ser Ala Pro Glu Pro Asp Ser
   545 550 555 560
   Thr Tyr Tyr His Ser Asn Lys Thr Ser Asn Val Gly Thr Phe Gly Thr
   565 570 575
   Pro Leu Lys Lys Leu Asp Gly Leu Lys Val Gly Val Leu Ala Ser Val
   580 585 590
   Asn Gly Glu Ser Ser Ile Ala Glu Gly Gln Ala Leu Ala Gln Ser Leu
   595 600 605
   Ala Gly Ser Asn Val Asp Val Val Ile Val Ala Glu His Leu Thr Ser
   610 615 620
   Asn Val Ser Ala Thr Tyr Ser Gly Ser Asp Ala Thr Asn Phe Asp Ala
   625 630 635 640
   Val Ile Val Ser Ser Gly Ala Glu Gly Leu Phe Gly Pro Gln Thr Phe
   645 650 655
   Thr Ala Glu Ser Asn Thr Thr Leu Tyr Pro Ala Gly Arg Pro Ser Gln
   660 665 670
   Ile Leu Val Asp Ala Phe Arg Phe Gly Lys Pro Val Gly Ala Val Gly
   675 680 685
   Gly Ala Ser Ala Ala Leu Ser Ala Val Asp Ile Ser Thr Asp Arg Ser
   690 695 700
   Gly Val Ile Thr Gly Asp Ser Val Ser Asp Asp Phe Val Lys Gln Leu
   705 710 715 720
   Thr Glu Asp Leu Ala Thr Phe Lys Phe Leu Asp Arg Phe Ala Val Asp
   725 730 735
   Glu
<210> 19
   <211> 737
   <212> PRT
   <213> Pichia pastoris
<400> 19
   Met Phe Ser Pro Ile Leu Ser Leu Glu Ile Ile Leu Ala Leu Ala Thr
   1 5 10 15
   Leu Gln Ser Val Phe Ala Ala Cys Pro Met Leu Thr Gly Glu Ile Pro
   20 25 30
   Ala Gly Ser Val Ala Asn Pro His His His Gly Lys Arg Asp Asp Ser
   35 40 45
   Asn Ala Ser Ser Glu Thr Glu Ala Phe Leu Ser Glu Phe Tyr Leu Asn
   50 55 60
   Asp Asn Asp Ala Tyr Leu Thr Thr Asp Val Gly Gly Pro Ile Glu Asp
   65 70 75 80
   Gln His Ser Leu Lys Ala Gly Ile Arg Gly Ser Thr Leu Leu Glu Asp
   85 90 95
   Phe Ile Phe Arg Gln Lys Ile Gln His Phe Asp His Glu Arg Val Pro
   100 105 110
   Glu Arg Ala Val His Ala Arg Gly Ala Gly Ala His Gly Val Phe Thr
   115 120 125
   Ser Tyr Ala Asp Trp Ser Asn Ile Thr Ala Ala Ser Phe Leu Gly Ala
   130 135 140
   Ala Gly Lys Glu Thr Pro Thr Phe Val Arg Phe Ser Thr Val Ala Gly
   145 150 155 160
   Ser Arg Gly Ser Ala Asp Thr Ala Arg Asp Val His Gly Phe Ala Thr
   165 170 175
   Arg Phe Tyr Thr Asp Glu Gly Asn Tyr Asp Ile Val Gly Asn Asn Ile
   180 185 190
   Pro Val Phe Phe Ile Gln Asp Ala Ile Leu Phe Pro Asp Leu Ile His
   195 200 205
   Ser Val Lys Pro Gln Pro Ala Asn Glu Ile Pro Gln Ala Ala Thr Ala
   210 215 220
   His Asp Thr Ala Tyr Asp Phe Phe Gly Gln Gln Pro Ser Thr Leu His
   225 230 235 240
   Thr Leu Phe Trp Ala Met Ala Gly His Gly Ile Pro Arg Ser Phe Arg
   245 250 255
   His Val Asp Gly Phe Gly Val His Thr Tyr Arg Phe Val Thr Glu Asp
   260 265 270
   Gly Ser Ser Lys Leu Val Lys Phe His Trp Thr Ser Leu Gln Gly Arg
   275 280 285
   Ala Ser Leu Val Trp Glu Glu Ala Gln Ala Thr Ala Gly Lys Asn Ala
   290 295 300
   Asp Phe Met Arg Gln Asp Leu Tyr Asp Ser Ile Glu Ala Gly Arg Tyr
   305 310 315 320
   Pro Glu Trp Glu Phe Gly Val Gln Ile Ile Asp Glu Ser Asp Val Leu
   325 330 335
   Ser Tyr Gly Phe Asp Leu Leu Asp Pro Thr Lys Ile Leu Pro Val Glu
   340 345 350
   Lys Val Pro Ile Thr Pro Leu Gly Lys Met Gln Leu Asn Arg Asn Pro
   355 360 365
   Leu Asn Tyr Phe Ala Glu Thr Glu Gln Val Met Phe Gln Pro Gly His
   370 375 380
   Ile Val Arg Gly Ile Asp Phe Thr Glu Asp Pro Leu Leu Gln Gly Arg
   385 390 395 400
   Leu Phe Ser Tyr Leu Asp Thr Gln Leu Asn Arg Asn Gly Gly Pro Asn
   405 410 415
   Phe Glu Gln Ile Pro Ile Asn Arg Pro Arg Val Pro Ile His Asn Asn
   420 425 430
   Asn Arg Asp Gly Phe Ala Gln Met Phe Ile Pro Leu Asn Gln Ala Ala
   435 440 445
   Tyr Ser Pro Asn Thr Leu Asn Asn Gly Ser Pro Arg Gln Ala Asn Glu
   450 455 460
   Thr Val Gly Asn Gly Phe Phe Thr Thr Pro Arg Arg Ser Ala Asp Gly
   465 470 475 480
   His Leu Val Arg Ala Thr Ser Pro Thr Phe Ala Asp Val Trp Ser Gln
   485 490 495
   Pro Gly Leu Phe Tyr Asn Ser Leu Thr Ala Thr Glu Gln Gln Phe Val
   500 505 510
   Ile Asn Ala Leu Arg Phe Glu Leu Ser Asn Val Lys Ser Glu Asp Val
   515 520 525
   Lys Ser Asn Phe Ile Thr Gln Ile Asn Arg Val Asn Asn Thr Leu Ala
   530 535 540
   Thr Leu Val Ala Ser Ala Ile Gly Val Ser Ala Pro Glu Pro Asp Ser
   545 550 555 560
   Thr Tyr Tyr His Ser Asn Lys Thr Ser Asn Val Gly Thr Phe Gly Thr
   565 570 575
   Pro Leu Lys Lys Leu Asp Gly Leu Lys Val Gly Val Leu Ala Ser Val
   580 585 590
   Ser Asp Glu Ser Ser Ile Ala Glu Gly Gln Ala Leu Ala Gln Ser Leu
   595 600 605
   Ala Asp Ser Asn Val Asp Val Val Ile Val Ala Glu His Leu Thr Ser
   610 615 620
   Asn Val Ser Ala Thr Tyr Ser Gly Ser Asp Ala Thr Asn Phe Asp Ala
   625 630 635 640
   Val Ile Val Ser Ser Gly Ala Glu Gly Leu Phe Gly Pro Gln Thr Phe
   645 650 655
   Thr Ala Glu Ser Asn Thr Thr Leu Tyr Pro Ala Gly Arg Pro Ser Gln
   660 665 670
   Ile Leu Val Asp Ala Phe Arg Phe Gly Lys Pro Val Gly Ala Val Gly
   675 680 685
   Gly Ala Ser Ala Ala Leu Ser Ala Val Asp Ile Ser Thr Asp Arg Ser
   690 695 700
   Gly Val Val Thr Gly Asp Ser Val Ser Asp Asp Phe Val Asn Gln Leu
   705 710 715 720
   Thr Glu Asp Leu Ala Thr Phe Lys Phe Leu Asp Arg Phe Ala Val Asp
   725 730 735
   Glu
<210> 20
   <211> 804
   <212> PRT
   <213> Pichia pastoris
<400> 20
   Met Arg Phe Pro Ser Ile Phe Thr Ala Val Leu Phe Ala Ala Ser Ser
   1 5 10 15
   Ala Leu Ala Ala Pro Val Asn Thr Thr Thr Glu Asp Glu Thr Ala Gln
   20 25 30
   Ile Pro Ala Glu Ala Val Ile Gly Tyr Ser Asp Leu Glu Gly Asp Phe
   35 40 45
   Asp Val Ala Val Leu Pro Phe Ser Asn Ser Thr Asn Asn Gly Leu Leu
   50 55 60
   Phe Ile Asn Thr Thr Ile Ala Ser Ile Ala Ala Lys Glu Glu Gly Val
   65 70 75 80
   Ser Leu Glu Lys Arg Glu Ala Glu Ala Ala Cys Pro Met Leu Thr Gly
   85 90 95
   Glu Ile Pro Ala Gly Ser Val Ala Asn Pro His His His Gly Lys Arg
   100 105 110
   Asp Asp Ser Asn Ala Ser Ser Glu Thr Glu Ala Phe Leu Ser Glu Phe
   115 120 125
   Tyr Leu Asn Asp Asn Asp Ala Tyr Leu Thr Thr Asp Val Gly Gly Pro
   130 135 140
   Ile Glu Asp Gln Asn Ser Leu Lys Ala Gly Ile Arg Gly Ser Thr Leu
   145 150 155 160
   Leu Glu Asp Phe Ile Phe Arg Gln Lys Ile Gln His Phe Asp His Glu
   165 170 175
   Arg Val Pro Glu Arg Ala Val His Ala Arg Gly Ala Gly Ala His Gly
   180 185 190
   Val Phe Thr Ser Tyr Ala Asp Trp Ser Asn Ile Thr Ala Ala Ser Phe
   195 200 205
   Leu Gly Ala Ser Gly Lys Glu Thr Pro Thr Phe Val Arg Phe Ser Thr
   210 215 220
   Val Ala Gly Ser Arg Gly Ser Ala Asp Thr Ala Arg Asp Val His Gly
   225 230 235 240
   Phe Ala Thr Arg Phe Tyr Thr Asp Glu Gly Asn Tyr Asp Ile Val Gly
   245 250 255
   Asn Asn Ile Pro Val Phe Phe Ile Gln Asp Ala Ile Leu Phe Pro Asp
   260 265 270
   Leu Ile His Ser Val Lys Pro Gln Pro Ala Asn Glu Ile Pro Gln Ala
   275 280 285
   Ala Thr Ala His Asp Thr Ala Tyr Asp Phe Phe Gly Gln Gln Pro Ser
   290 295 300
   Thr Leu His Thr Leu Phe Trp Ala Met Ala Gly His Gly Ile Pro Arg
   305 310 315 320
   Ser Phe Arg His Val Asp Gly Phe Gly Val His Thr Tyr Arg Phe Val
   325 330 335
   Thr Asp Asp Gly Ser Ser Lys Leu Val Lys Phe His Trp Thr Ser Leu
   340 345 350
   Gln Gly Arg Ala Ser Leu Val Trp Glu Glu Ala Gln Ala Thr Ala Gly
   355 360 365
   Lys Asn Ala Asp Phe Met Arg Gln Asp Leu Tyr Asp Ser Ile Glu Ala
   370 375 380
   Gly Arg Tyr Pro Glu Trp Glu Leu Gly Val Gln Ile Ile Glu Glu Ser
   385 390 395 400
   Asp Val Leu Ser Tyr Gly Phe Asp Leu Leu Asp Pro Thr Lys Ile Leu
   405 410 415
   Pro Val Glu Lys Val Pro Ile Thr Ala Leu Gly Lys Met Gln Leu Asn
   420 425 430
   Arg Asn Pro Leu Asn Tyr Phe Ala Glu Thr Glu Gln Val Met Phe Gln
   435 440 445
   Pro Gly His Ile Val Arg Gly Ile Asp Phe Thr Glu Asp Pro Leu Leu
   450 455 460
   Gln Gly Arg Leu Phe Ser Tyr Leu Asp Thr Gln Leu Asn Arg Asn Gly
   465 470 475 480
   Gly Pro Asn Phe Glu Gln Ile Pro Ile Asn Arg Pro Arg Val Pro Ile
   485 490 495
   His Asn Asn Asn Arg Asp Gly Phe Ala Gln Met Phe Ile Pro Leu Asn
   500 505 510
   Gln Ala Ala Tyr Ser Pro Asn Thr Leu Asn Asn Gly Ser Pro Arg Gln
   515 520 525
   Ala Asn Glu Thr Val Gly Asn Gly Phe Phe Thr Ala Pro Gly Arg Ser
   530 535 540
   Ala Asp Gly His Leu Val Arg Ala Thr Ser Pro Thr Phe Ala Asp Val
   545 550 555 560
   Trp Ser Gln Pro Gly Leu Phe Tyr Asn Ser Leu Thr Ala Thr Glu Gln
   565 570 575
   Gln Phe Val Ile Asn Ala Leu Arg Phe Glu Leu Ser Asn Val Lys Ser
   580 585 590
   Glu Asp Val Lys Ser Asn Phe Ile Thr Gln Ile Asn Arg Val Asn Asn
   595 600 605
   Thr Leu Ala Thr Leu Val Ala Ser Ala Ile Gly Val Ser Ala Pro Glu
   610 615 620
   Pro Asp Ser Thr Tyr Tyr His Ser Asn Lys Thr Ser Asn Val Gly Thr
   625 630 635 640
   Phe Gly Thr Pro Leu Lys Lys Leu Asp Gly Leu Lys Val Gly Val Leu
   645 650 655
   Ala Ser Val Asn Gly Glu Ser Ser Ile Ala Glu Gly Gln Ala Leu Ala
   660 665 670
   Gln Ser Leu Ala Gly Ser Asn Val Asp Val Val Ile Val Ala Glu His
   675 680 685
   Leu Thr Ser Asn Val Ser Ala Thr Tyr Ser Gly Ser Asp Ala Thr Asn
   690 695 700
   Phe Asp Ala Val Ile Val Ser Ser Gly Ala Glu Gly Leu Phe Gly Pro
   705 710 715 720
   Gln Thr Phe Thr Ala Glu Ser Asn Thr Thr Leu Tyr Pro Ala Gly Arg
   725 730 735
   Pro Ser Gln Ile Leu Val Asp Ala Phe Arg Phe Gly Lys Pro Val Gly
   740 745 750
   Ala Val Gly Gly Ala Ser Ala Ala Leu Ser Ala Val Asp Ile Ser Thr
   755 760 765
   Asp Arg Ser Gly Val Ile Thr Gly Asp Ser Val Ser Asp Asp Phe Val
   770 775 780
   Lys Gln Leu Thr Glu Asp Leu Ala Thr Phe Lys Phe Leu Asp Arg Phe
   785 790 795 800
   Ala Val Asp Glu
<210> 21
   <211> 804
   <212> PRT
   <213> Pichia pastoris
<400> 21
   Met Arg Phe Pro Ser Ile Phe Thr Ala Val Leu Phe Ala Ala Ser Ser
   1 5 10 15
   Ala Leu Ala Ala Pro Val Asn Thr Thr Thr Glu Asp Glu Thr Ala Gln
   20 25 30
   Ile Pro Ala Glu Ala Val Ile Gly Tyr Ser Asp Leu Glu Gly Asp Phe
   35 40 45
   Asp Val Ala Val Leu Pro Phe Ser Asn Ser Thr Asn Asn Gly Leu Leu
   50 55 60
   Phe Ile Asn Thr Thr Ile Ala Ser Ile Ala Ala Lys Glu Glu Gly Val
   65 70 75 80
   Ser Leu Glu Lys Arg Glu Ala Glu Ala Ala Cys Pro Met Leu Thr Gly
   85 90 95
   Glu Ile Pro Ala Gly Ser Val Ala Asn Pro His His His Gly Lys Arg
                   100                 105                 110
   Asp Asp Ser Asn Ala Ser Ser Glu Thr Glu Ala Phe Leu Ser Glu Phe
               115                 120                 125
   Tyr Leu Asn Asp Asn Asp Ala Tyr Leu Thr Thr Asp Val Gly Gly Pro
       130                 135                 140
   Ile Glu Asp Gln His Ser Leu Lys Ala Gly Ile Arg Gly Ser Thr Leu
   145                 150                 155                 160
   Leu Glu Asp Phe Ile Phe Arg Gln Lys Ile Gln His Phe Asp His Glu
                   165                 170                 175
   Arg Val Pro Glu Arg Ala Val His Ala Arg Gly Ala Gly Ala His Gly
               180                 185                 190
   Val Phe Thr Ser Tyr Ala Asp Trp Ser Asn Ile Thr Ala Ala Ser Phe
           195                 200                 205
   Leu Gly Ala Ala Gly Lys Glu Thr Pro Thr Phe Val Arg Phe Ser Thr
       210                 215                 220
   Val Ala Gly Ser Arg Gly Ser Ala Asp Thr Ala Arg Asp Val His Gly
   225                 230                 235                 240
   Phe Ala Thr Arg Phe Tyr Thr Asp Glu Gly Asn Tyr Asp Ile Val Gly
                   245                 250                 255
   Asn Asn Ile Pro Val Phe Phe Ile Gln Asp Ala Ile Leu Phe Pro Asp
               260                 265                 270
   Leu Ile His Ser Val Lys Pro Gln Pro Ala Asn Glu Ile Pro Gln Ala
           275                 280                 285
   Ala Thr Ala His Asp Thr Ala Tyr Asp Phe Phe Gly Gln Gln Pro Ser
       290                 295                 300
   Thr Leu His Thr Leu Phe Trp Ala Met Ala Gly His Gly Ile Pro Arg
   305                 310                 315                 320
   Ser Phe Arg His Val Asp Gly Phe Gly Val His Thr Tyr Arg Phe Val
                   325                 330                 335
   Thr Glu Asp Gly Ser Ser Lys Leu Val Lys Phe His Trp Thr Ser Leu
               340                 345                 350
   Gln Gly Arg Ala Ser Leu Val Trp Glu Glu Ala Gln Ala Thr Ala Gly
   355 360 365
   Lys Asn Ala Asp Phe Met Arg Gln Asp Leu Tyr Asp Ser Ile Glu Ala
   370 375 380
   Gly Arg Tyr Pro Glu Trp Glu Phe Gly Val Gln Ile Ile Asp Glu Ser
   385 390 395 400
   Asp Val Leu Ser Tyr Gly Phe Asp Leu Leu Asp Pro Thr Lys Ile Leu
   405 410 415
   Pro Val Glu Lys Val Pro Ile Thr Pro Leu Gly Lys Met Gln Leu Asn
   420 425 430
   Arg Asn Pro Leu Asn Tyr Phe Ala Glu Thr Glu Gln Val Met Phe Gln
   435 440 445
   Pro Gly His Ile Val Arg Gly Ile Asp Phe Thr Glu Asp Pro Leu Leu
   450 455 460
   Gln Gly Arg Leu Phe Ser Tyr Leu Asp Thr Gln Leu Asn Arg Asn Gly
   465 470 475 480
   Gly Pro Asn Phe Glu Gln Ile Pro Ile Asn Arg Pro Arg Val Pro Ile
   485 490 495
   His Asn Asn Asn Arg Asp Gly Phe Ala Gln Met Phe Ile Pro Leu Asn
   500 505 510
   Gln Ala Ala Tyr Ser Pro Asn Thr Leu Asn Asn Gly Ser Pro Arg Gln
   515 520 525
   Ala Asn Glu Thr Val Gly Asn Gly Phe Phe Thr Thr Pro Arg Arg Ser
   530 535 540
   Ala Asp Gly His Leu Val Arg Ala Thr Ser Pro Thr Phe Ala Asp Val
   545 550 555 560
   Trp Ser Gln Pro Gly Leu Phe Tyr Asn Ser Leu Thr Ala Thr Glu Gln
   565 570 575
   Gln Phe Val Ile Asn Ala Leu Arg Phe Glu Leu Ser Asn Val Lys Ser
   580 585 590
   Glu Asp Val Lys Ser Asn Phe Ile Thr Gln Ile Asn Arg Val Asn Asn
   595 600 605
   Thr Leu Ala Thr Leu Val Ala Ser Ala Ile Gly Val Ser Ala Pro Glu
   610 615 620
   Pro Asp Ser Thr Tyr Tyr His Ser Asn Lys Thr Ser Asn Val Gly Thr
   625 630 635 640
   Phe Gly Thr Pro Leu Lys Lys Leu Asp Gly Leu Lys Val Gly Val Leu
   645 650 655
   Ala Ser Val Ser Asp Glu Ser Ser Ile Ala Glu Gly Gln Ala Leu Ala
   660 665 670
   Gln Ser Leu Ala Asp Ser Asn Val Asp Val Val Ile Val Ala Glu His
   675 680 685
   Leu Thr Ser Asn Val Ser Ala Thr Tyr Ser Gly Ser Asp Ala Thr Asn
   690 695 700
   Phe Asp Ala Val Ile Val Ser Ser Gly Ala Glu Gly Leu Phe Gly Pro
   705 710 715 720
   Gln Thr Phe Thr Ala Glu Ser Asn Thr Thr Leu Tyr Pro Ala Gly Arg
   725 730 735
   Pro Ser Gln Ile Leu Val Asp Ala Phe Arg Phe Gly Lys Pro Val Gly
   740 745 750
   Ala Val Gly Gly Ala Ser Ala Ala Leu Ser Ala Val Asp Ile Ser Thr
   755 760 765
   Asp Arg Ser Gly Val Val Thr Gly Asp Ser Val Ser Asp Asp Phe Val
   770 775 780
   Asn Gln Leu Thr Glu Asp Leu Ala Thr Phe Lys Phe Leu Asp Arg Phe
   785 790 795 800
   Ala Val Asp Glu
<210> 22
   <211> 734
   <212> PRT
   <213> Aspergillus niger
<400> 22
   Met Arg Gly Leu Tyr Ser Leu Gly Thr Phe Ala Gly Leu Thr Ala Ala
   1 5 10 15
   Ala Ser Ala Ala Cys Pro Met Leu Thr Gly Glu Ile Pro Ala Gly Ser
   20 25 30
   Val Ala Asn Pro His His His Gly Lys Arg Asp Asp Ser Asn Ala Ser
           35                  40                  45
   Ser Glu Thr Glu Ala Phe Leu Ser Glu Phe Tyr Leu Asn Asp Asn Asp
       50                  55                  60
   Ala Tyr Leu Thr Thr Asp Val Gly Gly Pro Ile Glu Asp Gln His Ser
   65                  70                  75                  80
   Leu Lys Ala Gly Ile Arg Gly Ser Thr Leu Leu Glu Asp Phe Ile Phe
                   85                  90                  95
   Arg Gln Lys Ile Gln His Phe Asp His Glu Arg Val Pro Glu Arg Ala
               100                 105                 110
   Val His Ala Arg Gly Ala Gly Ala His Gly Val Phe Thr Ser Tyr Ala
           115                 120                 125
   Asp Trp Ser Asn Ile Thr Ala Ala Ser Phe Leu Gly Ala Ala Gly Lys
       130                 135                 140
   Glu Thr Pro Thr Phe Val Arg Phe Ser Thr Val Ala Gly Ser Arg Gly
   145                 150                 155                 160
   Ser Ala Asp Thr Ala Arg Asp Val His Gly Phe Ala Thr Arg Phe Tyr
                   165                 170                 175
   Thr Asp Glu Gly Asn Tyr Asp Ile Val Gly Asn Asn Ile Pro Val Phe
               180                 185                 190
   Phe Ile Gln Asp Ala Ile Leu Phe Pro Asp Leu Ile His Ser Val Lys
           195                 200                 205
   Pro Gln Pro Ala Asn Glu Ile Pro Gln Ala Ala Thr Ala His Asp Thr
       210                 215                 220
   Ala Tyr Asp Phe Phe Gly Gln Gln Pro Ser Thr Leu His Thr Leu Phe
   225                 230                 235                 240
   Trp Ala Met Ala Gly His Gly Ile Pro Arg Ser Phe Arg His Val Asp
                   245                 250                 255
   Gly Phe Gly Val His Thr Tyr Arg Phe Val Thr Glu Asp Gly Ser Ser
               260                 265                 270
   Lys Leu Val Lys Phe His Trp Thr Ser Leu Gln Gly Arg Ala Ser Leu
           275                 280                 285
   Val Trp Glu Glu Ala Gln Ala Thr Ala Gly Lys Asn Ala Asp Phe Met
   290 295 300
   Arg Gln Asp Leu Tyr Asp Ser Ile Glu Ala Gly Arg Tyr Pro Glu Trp
   305 310 315 320
   Glu Phe Gly Val Gln Ile Ile Asp Glu Ser Asp Val Leu Ser Tyr Gly
   325 330 335
   Phe Asp Leu Leu Asp Pro Thr Lys Ile Leu Pro Val Glu Lys Val Pro
   340 345 350
   Ile Thr Pro Leu Gly Lys Met Gln Leu Asn Arg Asn Pro Leu Asn Tyr
   355 360 365
   Phe Ala Glu Thr Glu Gln Val Met Phe Gln Pro Gly His Ile Val Arg
   370 375 380
   Gly Ile Asp Phe Thr Glu Asp Pro Leu Leu Gln Gly Arg Leu Phe Ser
   385 390 395 400
   Tyr Leu Asp Thr Gln Leu Asn Arg Asn Gly Gly Pro Asn Phe Glu Gln
   405 410 415
   Ile Pro Ile Asn Arg Pro Arg Val Pro Ile His Asn Asn Asn Arg Asp
   420 425 430
   Gly Phe Ala Gln Met Phe Ile Pro Leu Asn Gln Ala Ala Tyr Ser Pro
   435 440 445
   Asn Thr Leu Asn Asn Gly Ser Pro Arg Gln Ala Asn Glu Thr Val Gly
   450 455 460
   Asn Gly Phe Phe Thr Thr Pro Arg Arg Ser Ala Asp Gly His Leu Val
   465 470 475 480
   Arg Ala Thr Ser Pro Thr Phe Ala Asp Val Trp Ser Gln Pro Gly Leu
   485 490 495
   Phe Tyr Asn Ser Leu Thr Ala Thr Glu Gln Gln Phe Val Ile Asn Ala
   500 505 510
   Leu Arg Phe Glu Leu Ser Asn Val Lys Ser Glu Asp Val Lys Ser Asn
   515 520 525
   Phe Ile Thr Gln Ile Asn Arg Val Asn Asn Thr Leu Ala Thr Leu Val
   530 535 540
   Ala Ser Ala Ile Gly Val Ser Ala Pro Glu Pro Asp Ser Thr Tyr Tyr
   545 550 555 560
   His Ser Asn Lys Thr Ser Asn Val Gly Thr Phe Gly Thr Pro Leu Lys
   565 570 575
   Lys Leu Asp Gly Leu Lys Val Gly Val Leu Ala Ser Val Ser Asp Glu
   580 585 590
   Ser Ser Ile Ala Glu Gly Gln Ala Leu Ala Gln Ser Leu Ala Asp Ser
   595 600 605
   Asn Val Asp Val Val Ile Val Ala Glu His Leu Thr Ser Asn Val Ser
   610 615 620
   Ala Thr Tyr Ser Gly Ser Asp Ala Thr Asn Phe Asp Ala Val Ile Val
   625 630 635 640
   Ser Ser Gly Ala Glu Gly Leu Phe Gly Pro Gln Thr Phe Thr Ala Glu
   645 650 655
   Ser Asn Thr Thr Leu Tyr Pro Ala Gly Arg Pro Ser Gln Ile Leu Val
   660 665 670
   Asp Ala Phe Arg Phe Gly Lys Pro Val Gly Ala Val Gly Gly Ala Ser
   675 680 685
   Ala Ala Leu Ser Ala Val Asp Ile Ser Thr Asp Arg Ser Gly Val Val
   690 695 700
   Thr Gly Asp Ser Val Ser Asp Asp Phe Val Asn Gln Leu Thr Glu Asp
   705 710 715 720
   Leu Ala Thr Phe Lys Phe Leu Asp Arg Phe Ala Val Asp Glu
   725 730
<210> 23
   <211> 734
   <212> PRT
   <213> Aspergillus niger
<400> 23
   Met Arg Gly Leu Tyr Ser Leu Gly Thr Phe Ala Gly Leu Thr Ala Ala
   1 5 10 15
   Ala Ser Ala Ala Cys Pro Met Leu Thr Gly Glu Ile Pro Ala Gly Ser
   20 25 30
   Val Ala Asn Pro His His His Gly Lys Arg Asp Asp Ser Asn Ala Ser
   35 40 45
   Ser Glu Thr Glu Ala Phe Leu Ser Glu Phe Tyr Leu Asn Asp Asn Asp
   50 55 60
   Ala Tyr Leu Thr Thr Asp Val Gly Gly Pro Ile Glu Asp Gln His Ser
   65 70 75 80
   Leu Lys Ala Gly Ile Arg Gly Ser Thr Leu Leu Glu Asp Phe Ile Phe
   85 90 95
   Arg Gln Lys Ile Gln His Phe Asp His Glu Arg Val Pro Glu Arg Ala
   100 105 110
   Val His Ala Arg Gly Ala Gly Ala His Gly Val Phe Thr Ser Tyr Ala
   115 120 125
   Asp Trp Ser Asn Ile Thr Ala Ala Ser Phe Leu Gly Ala Ala Gly Lys
   130 135 140
   Glu Thr Pro Thr Phe Val Arg Phe Ser Thr Val Ala Gly Ser Arg Gly
   145 150 155 160
   Ser Ala Asp Thr Ala Arg Asp Val His Gly Phe Ala Thr Arg Phe Tyr
   165 170 175
   Thr Asp Glu Gly Asn Tyr Asp Ile Val Gly Asn Asn Ile Pro Val Phe
   180 185 190
   Phe Ile Gln Asp Ala Ile Leu Phe Pro Asp Leu Ile His Ser Val Lys
   195 200 205
   Pro Gln Pro Ala Asn Glu Ile Pro Gln Ala Ala Thr Ala His Asp Thr
   210 215 220
   Ala Tyr Asp Phe Phe Gly Gln Gln Pro Ser Thr Leu His Thr Leu Phe
   225 230 235 240
   Trp Ala Met Ala Gly His Gly Ile Pro Arg Ser Phe Arg His Val Asp
   245 250 255
   Gly Phe Gly Val His Thr Tyr Arg Phe Val Thr Glu Asp Gly Ser Ser
   260 265 270
   Lys Leu Val Lys Phe His Trp Thr Ser Leu Gln Gly Arg Ala Ser Leu
   275 280 285
   Val Trp Glu Glu Ala Gln Ala Thr Ala Gly Lys Asn Ala Asp Phe Met
   290 295 300
   Arg Gln Asp Leu Tyr Asp Ser Ile Glu Ala Gly Arg Tyr Pro Glu Trp
   305 310 315 320
   Glu Phe Gly Val Gln Ile Ile Asp Glu Ser Asp Val Leu Ser Tyr Gly
   325 330 335
   Phe Asp Leu Leu Asp Pro Thr Lys Ile Leu Pro Val Glu Lys Val Pro
   340 345 350
   Ile Thr Pro Leu Gly Lys Met Gln Leu Asn Arg Asn Pro Leu Asn Tyr
   355 360 365
   Phe Ala Glu Thr Glu Gln Val Met Phe Gln Pro Gly His Ile Val Arg
   370 375 380
   Gly Ile Asp Phe Thr Glu Asp Pro Leu Leu Gln Gly Arg Leu Phe Ser
   385 390 395 400
   Tyr Leu Asp Thr Gln Leu Asn Arg Asn Gly Gly Pro Asn Phe Glu Gln
   405 410 415
   Ile Pro Ile Asn Arg Pro Arg Val Pro Ile His Asn Asn Asn Arg Asp
   420 425 430
   Gly Phe Ala Gln Met Phe Ile Pro Leu Asn Gln Ala Ala Tyr Ser Pro
   435 440 445
   Asn Thr Leu Asn Asn Gly Ser Pro Arg Gln Ala Asn Glu Thr Val Gly
   450 455 460
   Asn Gly Phe Phe Thr Thr Pro Arg Arg Ser Ala Asp Gly His Leu Val
   465 470 475 480
   Arg Ala Thr Ser Pro Thr Phe Ala Asp Val Trp Ser Gln Pro Gly Leu
   485 490 495
   Phe Tyr Asn Ser Leu Thr Ala Thr Glu Gln Gln Phe Val Ile Asn Ala
   500 505 510
   Leu Arg Phe Glu Leu Ser Asn Val Lys Ser Glu Asp Val Lys Ser Asn
   515 520 525
   Phe Ile Thr Gln Ile Asn Arg Val Asn Asn Thr Leu Ala Thr Leu Val
   530 535 540
   Ala Ser Ala Ile Gly Val Ser Ala Pro Glu Pro Asp Ser Thr Tyr Tyr
   545 550 555 560
   His Ser Asn Lys Thr Ser Asn Val Gly Thr Phe Gly Thr Pro Leu Lys
   565 570 575
   Lys Leu Asp Gly Leu Lys Val Gly Val Leu Ala Ser Val Ser Asp Glu
   580 585 590
   Ser Ser Ile Ala Glu Gly Gln Ala Leu Ala Gln Ser Leu Ala Asp Ser
   595 600 605
   Asn Val Asp Val Val Ile Val Ala Glu His Leu Thr Ser Asn Val Ser
   610 615 620
   Ala Thr Tyr Ser Gly Ser Asp Ala Thr Asn Phe Asp Ala Val Ile Val
   625 630 635 640
   Ser Ser Gly Ala Glu Gly Leu Phe Gly Pro Gln Thr Phe Thr Ala Glu
   645 650 655
   Ser Asn Thr Thr Leu Tyr Pro Ala Gly Arg Pro Ser Gln Ile Leu Val
   660 665 670
   Asp Ala Phe Arg Phe Gly Lys Pro Val Gly Ala Val Gly Ser Ala Arg
   675 680 685
   Ala Ala Leu Ser Ala Val Asp Ile Ser Thr Asp Arg Ser Gly Val Val
   690 695 700
   Thr Gly Asp Ser Val Ser Asp Asp Phe Val Asn Gln Leu Thr Glu Asp
   705 710 715 720
   Leu Ala Thr Phe Lys Phe Leu Asp Arg Phe Ala Val Asp Glu
   725 730